# EUROPEAN PATENT SPECIFICATION

(11) **EP 1 794 304 B1**
(45) Date of publication and mention of the grant of the patent: **19.06.2013**
(21) Application number: 05794660.0
(22) Date of filing: 23.09.2005
(51) Int. Cl.: C12N 15/82, A01H 5/00, C07K 14/415, C07K 16/16

(54) **PLANT CELLS AND PLANTS WITH INCREASED TOLERANCE TO ENVIRONMENTAL STRESS**
PFLANZENZELLEN UND PFLANZEN MIT ERHÖHTER UMWELTSTRESSTOLERANZ
CELLULES VEGETALES ET PLANTES PRESENTANT UNE TOLERANCE ACCRUE AU STRESS ENVIRONNEMENTAL

(30) Priority: 24.09.2004 US 613154 P; 14.10.2004 US 618737 P
(43) Date of publication of application: 13.06.2007
(73) Proprietor: BASF Plant Science GmbH, 67056 Ludwigshafen (DE)
(72) Inventor: MCKERSIE, Bryan, Raleigh, NC 27617 (US); PLESCH, Gunnar, 14482 Potsdam (DE); PUZIO, Piotr, 13505 Berlin (DE); WILD, Harry, 14059 Berlin (DE); CHARDONNENS, Agnes, NL-1602 NH Enkhuizen (NL)
(86) International application number: PCT/EP2005/055023
(87) International publication number: WO 2006/032707

(56) References cited:
- WO-A-02/16655
- WO-A-02/22675
- WO-A-02/097097
- WO-A-03/020015
- WO-A-2004/092349
- US-A1- 2004 031 072
- SEKI M ET AL: "Monitoring the expression pattern of 1300 Arabidopsis genes under drought and cold stresses by using a full-length cDNA microarray" PLANT CELL, AMERICAN SOCIETY OF PLANT PHYSIOLOGISTS, ROCKVILLE, MD, US, vol. 13, no. 1, January 2001 (2001-01), pages 61-72, XP002216349 ISSN: 1040-4651
- SCHENK P M ET AL: "Coordinated plant defense responses in Arabidopsis revealed by microarray analysis" PROCEEDINGS OF THE NATIONAL ACADEMY OF SCIENCES OF USA, NATIONAL ACADEMY OF SCIENCE, WASHINGTON, DC, US, vol. 97, no. 21, 10 October 2000 (2000-10-10), pages 11655-11660, XP002153163 ISSN: 0027-8424
- XIONG L ET AL: "Disease Resistance and Abiotic Stress Tolerance in Rice Are Inversely Modulated by an Abscisic Acid-Inducible Mitogen-Actived Protein Kinase" PLANT CELL, AMERICAN SOCIETY OF PLANT PHYSIOLOGISTS, ROCKVILLE, MD, US, vol. 15, March 2003 (2003-03), pages 745-759, XP002264237 ISSN: 1040-4651
- WI SOO JIN ET AL: "Antisense expression of carnation cDNA encoding ACC synthase or ACC oxidase enhances polyamine content and abiotic stress tolerance in transgenic tobacco plants" MOLECULES AND CELLS, SEOUL, KR, vol. 13, no. 2, 30 April 2002 (2002-04-30), pages 209-220, XP002338203 ISSN: 1016-8478
- BAJAJ SHAVINDRA ET AL: "Transgenic approaches to increase dehydration-stress tolerance in plants" MOLECULAR BREEDING, vol. 5, no. 6, 1999, pages 493-503, XP002376591 ISSN: 1380-3743

## Description

The instant application is based on and claims the benefit of prior filed U.S. Patent Application No. 60/613,154, filed September 24, 2004 and prior filed U.S. Patent Application No. 60/618,737, filed October 14, 2004.

This invention relates generally to transformed plant cells and plants comprising an inactivated or down-regulated gene resulting in increased tolerance and/or resistance to environmental stress as compared to non-transformed wild type cells and methods of producing such plant cells or plants. The scope of protection conferred by the present patent is defined solely in the claims.

This invention further relates generally to transformed plant cells with increased tolerance and/or resistance to environmental stress as compared to a corresponding non-transformed wild type plant cell, wherein the increased tolerance and/or resistance to environmental stress as compared to a corresponding non-transformed wild type plant cell is altered by an inactivated or down-regulated gene, methods of producing, screening for and breeding such plant cells or plants and method of detecting stress in plants cells or plants.

In particular, this invention relates to transformed plant cells and plants comprising an inactivated or down-regulated gene resulting in increased tolerance and/or resistance to environmental stress, preferably by altering the metabolic activity, as compared to non-transformed wild type cells and methods of producing such plant cells or plants.

Abiotic environmental stress, such as drought stress, salinity stress, heat stress, and cold stress, is a major limiting factor of plant growth and productivity (Boyer. 1982. Science 218, 443-448). Crop losses and crop yield losses of major crops such as rice, maize (corn) and wheat caused by these stresses represent a significant economic and political factor and contribute to food shortages in many underdeveloped and third-world countries.

Plants are typically exposed during their life cycle to conditions of reduced environmental water content. Most plants have evolved strategies to protect themselves against these conditions of low water or desiccation (drought) for short period of time. However, if the severity and duration of the drought conditions are too great, the effects on plant development, growth and yield of most crop plants are profound. Continuous exposure to drought causes major alterations in the plant metabolism. These great changes in metabolism ultimately lead to cell death and consequently yield losses.

Developing stress-tolerant and/or resistant plants is a strategy that has the potential to solve or mediate at least some of these problems (McKersie and Leshem, 1994. Stress and Stress Coping in Cultivated Plants, Kluwer Academic Publishers; or see US2004/0031072 or Bajaj et al., 1999, Molecular Breeding 5(6):493-503, or Xiong et al., 2003 (The Plant Cell 15: 745-759), Wi Soo Jin et al., 2002 (Molecules And Cells 13 (2): 209-220), WO 02/09797, WO 03/020015, WO 2004/092349). However, traditional plant breeding strategies to develop new lines of plants that exhibit resistance (tolerance) to these types of stress are relatively slow and require specific resistant lines for crossing with the desired line. Limited germplasm resources for stress tolerance and incompatibility in crosses between distantly related plant species represent significant problems encountered in conventional breeding. Additionally, the cellular processes leading to drought, cold and salt tolerance and/or resistance are complex in nature and involve multiple mechanisms of cellular adaptation and numerous metabolic pathways (McKersie and Leshem, 1994. Stress and Stress Coping in Cultivated Plants, Kluwer Academic Publishers). This multi-component nature of stress tolerance and/or resistance has not only made breeding for tolerance and/or resistance largely unsuccessful, but has also limited the ability to genetically engineer stress tolerance plants using biotechnological methods.

Drought, heat, cold and salt stress have a common theme important for plant growth and that is water availability. Plants are exposed during their entire life cycle to conditions of reduced environmental water content. Most plants have evolved strategies to protect themselves against lack of water. However, if the severity and duration of the drought conditions are too great, the effects on plant development, growth and yield of most crop plants are profound. Since high salt content in some soils result in less available water for cell intake, its effect is similar to those observed under drought conditions. Likewise, under freezing temperatures, plant cells loose water as a result of ice formation that starts in the apoplast and withdraws water from the symplast (McKersie and Leshem, 1994. Stress and Stress Coping in Cultivated Plants, Kluwer Academic Publishers). Commonly, a plant's molecular response mechanisms to each of these stress conditions are the same.

The results of current research indicate that drought tolerance and/or resistance is a complex quantitative trait and that no real diagnostic marker is available yet. High salt concentrations or dehydration may cause damage at the cellular level during drought stress but the precise injury is not entirely clear (Bray, 1997. Trends Plant Sci. 2, 48-54). This lack of a mechanistic understanding makes it difficult to design a transgenic approach to improve drought tolerance and/or resistance. However, an important consequence of damage may be the production of reactive oxygen radicals that cause cellular injury, such as lipid peroxidation or protein and nucleic acid modification. Details of oxygen free radical chemistry and their reaction with cellular components such as cell membranes have been described (McKersie and Leshem, 1994. Stress and Stress Coping in Cultivated Plants, Kluwer Academic Publishers).

There are numerous sites of oxygen activation in the plant cell, which are highly controlled and tightly coupled to prevent release of intermediate products (McKersie and Leshem, 1994. Stress and Stress Coping in Cultivated Plants, Kluwer Academic Publishers). Under abiotic stress situations, it is likely that this control or coupling breaks down and the process "dysfunctions" leaking activated oxygen. These uncoupling events are not detrimental provided that they are short in duration and that the oxygen scavenging systems are able to detoxify the various forms of activated oxygen. If the production of activated oxygen exceeds the plant's capacity to detoxify it, deleterious degenerative reactions occur. At the subcellular level, disintegration of membranes and aggregation of proteins are typical symptoms. Therefore it is the balance between the production and the scavenging of activated oxygen that is critical to the maintenance of active growth and metabolism of the plant and overall environmental (abiotic) stress tolerance and/or resistance.

Preventing or diminishing the accumulation of oxygen free radicals in response to drought is a potential way to engineer tolerance (Allen, 1995. Plant Physiol. 107, 1049-1054). Overexpression of antioxidant enzymes or ROS-scavenging enzymes is one possibility for the induction of functional detoxification systems. For example, transgenic alfalfa plants expressing Mn-superoxide dismutase tend to have reduced injury after water-deficit stress (McKersie et al., 1996. Plant Physiol. 111, 1177-1181). These same transgenic plants have increased biomass production in field trials (McKersie et al., 1999. Plant Physiology, 119: 839-847; McKersie et al., 1996. Plant Physiol. 111, 1177-1181). Transgenic plants that overproduce osmolytes such as mannitol, fructans, proline or glycine-betaine also show increased resistance to some forms of abiotic stress and it is proposed that the synthesized osmolytes act as ROS scavengers (Tarczynski. et al. 1993 Science 259, 508-510; Sheveleva,. et al. 1997. Plant Physiol.115, 1211-1219).

It is the object of this invention to identify new, unique genes capable of conferring stress tolerance to plants upon inactivation or down-regulation of genes.

It is further object of this invention to identify, produce new, unique stress tolerant and/or resistant plant cells or plants and methods of inducing and detecting stress tolerance and/or resistance in plants or plant cells. It is a further object to identify new methods to detect stress tolerance and/or resistance in plants or plant cells.

The present invention provides a transformed plant cell, preferably with altered metabolic activity compared to a corresponding non transformed wild type plant cell, wherein the increased tolerance and/or resistance to an environmental stress as compared to a corresponding non-transformed wild type plant cell is altered by an inactivated or down-regulated gene.

The present invention provides a transformed plant cell with increased tolerance and/or resistance to an environmental stress as compared to a corresponding non-transformed wild type plant cell, wherein the increased tolerance and/or resistance to an environmental stress is altered by an inactivated or down-regulated gene.

As used herein, the term "inactivated or down-regulated gene" means the transgenic reduction or deletion of the expression of nucleic acid of SEQ ID NO:53 leading to an increased tolerance and/or resistance to an environmental stress as compared to a corresponding non-transformed wild type plant cell.

In the transgenic plant cell of the invention, the reduction or deletion of the expression of said nucleic acid results in increased tolerance to an environmental stress as compared to a corresponding non-transformed wild type plant cell. Herein, the environmental stress is selected from the group consisting of salinity, drought, temperature, metal, chemical, pathogenic and oxidative stresses, or combinations thereof, preferably drought and/or temperature.

The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic,local or temporal, for example limited to certain cell types, tissuesorgans or time periods.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, the terms "gene(s)", "polynucleotide", "nucleic acid sequence", "nucleotide sequence", or "nucleic acid molecule(s)" as used herein include double- and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

For the purposes of the invention, as a rule the plural is intended to encompass the singular and vice versa.

The terms "reduction", "decrease" or "deletion" relate to a corresponding change of a property in an organism, a part of an organism such as a tissue, seed, root, leave, flower etc. or in a cell. Under "change of a property" it is understood that the activity, expression level or amount of a gene product or the metabolite content is changed in a specific volume or in a specific amount of protein relative to a corresponding volume or amount of protein of a control, reference or wild type. Preferably, the overall activity in the volume is reduced, decreased or deleted in cases if the reduction, decrease or deletion is related to the reduction, decrease or deletion of an activity of a gene product, independent whether the amount of gene product or the specific activity of the gene product or both is reduced, decreased or deleted or whether the amount, stability or translation efficacy of the nucleic acid sequence or gene encoding for the gene product is reduced, decreased or deleted.

The terms "reduction", "decrease" or "deletion" include the change of said property in only parts of the subject of the present invention, for example, the modification can be found in compartment of a cell, like an organelle, or in a part of a plant, like tissue, seed, root, leave, flower etc. but is not detectable if the overall subject, i.e. complete cell or plant, is tested. Preferably, the "reduction", "decrease" or "deletion" is found cellular, thus the term "reduction, decrease or deletion of an acitivity" or "reduction, decrease or deletion of a metabolite content" relates to the cellular reduction, decrease or deletion compared to the wild typ cell. In addition the terms "reduction", "decrease" or "deletion" include the change of said property only during different growth phases of the organism used in the inventive process, for example the reduction, decrease or deletion takes place only during the seed growth or during blooming. Furtheremore the terms include a transitional reduction, decrease or deletion for example because the used RNAi is not stable integrated in the genom of the organism and has therefore only a transient effect.

Accordingly, the term "reduction", "decrease" or "deletion" means that the specific activity of an enzyme or other protein or regulatory RNA as well as the amount of a compound or metabolite, e.g. of a polypeptide, a nucleic acid molelcule or the fine chemical of the invention or an encoding mRNA or DNA, can be reduced, decreased or deleted in a volume.

The terms "wild type", "control" or "reference" are exchangeable and can be a cell or a part of organisms such as an organelle or tissue, or an organism, in particular a microorganism or a plant, which was not modified or treated according to the herein described process according to the invention. Accordingly, the cell or a part of organisms such as an organelle or a tissue, or an organism, in particular a microorganism or a plant used as wild type, control or reference corresponds to the cell, organism or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant or a microorganism, which was not modified or treated according to the herein described process of the invention and is in any other property as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or -organism, in particular plant or microorganism, relates to an organelle, cell, tissue or organism, in particular plant or micororganism, which is nearly genetically identical to the organelle, cell, tissue or organism, in particular microorganism or plant, of the present invention or a part thereof preferably 95%, more peferred are 98%, even more preferred are 99,00%, in particular 99,10%, 99,30%, 99,50%, 99,70%, 99,90%, 99,99%, 99, 999% or more. Most preferable the "reference", "control", or "wild type" is preferably a subject, e.g. an organelle, a cell, a tissue, an organism, which is genetically identical to the organism, cell organelle used according to the process of the invention except that nucleic acid molecules or the gene product encoded by them are changed according to the inventive process.

Preferably, the reference, control or wild type differs form the subject of the present invention only in the cellular activity of the polypeptide or RNA of the invention, e.g. as result of a reduction, decrease or deletion in the level of the nucleic acid molecule of the present invention or a reduction, decrease or deletion of the specific activity of the polypeptide or RNA of the invention, e.g. by or in the expression level or activity of protein or RNA that means its biological activity and/or its biochemical or genetical causes.

The term "expression" means the transcription of a gene into structural RNA (rRNA, tRNA, miRNA) or messenger RNA (mRNA) with the subsequent translation of the latter into a protein. Experimentally, expression can be detected by e.g. Northern, qRT PCR, transcriptional run-on assays or Western blotting and other immuno assays. As consequence of the reduction, decrease or deletion of the expression that means as consequence of the reduced, decreased or deleted transcription of a gene a related phenotypic trait appears such as the enhanced or increased stress tolerance.

Accordingly, preferred reference subject is the starting subject of the present process of the invention. Preferably, the reference and the subject matter of the invention are compared after standardization and normalization, e.g. to the amount of total RNA, DNA, or Protein or activity or expression of reference genes, like housekeeping genes, such as ubiquitin.

A series of mechanisms exists via which a modification in the polypeptide of the invention can directly or indirectly affect stress tolerance. For example, the molecule number or the specific activity of the polypeptide of the invention or the number of expression of the nucleic acid molecule of the invention may be reduced, decreased or deleted. However, it is also possible to reduce, decrease or delete the expression of the gene which is naturally present in the organisms, for example by modifying the regulation of the gene, or by reducing or decreasing the stability of the mRNA or of the gene product encoded by the nucleic acid molecule of the invention.

This also applies analogously to the combined reduction, decrease or deletion of the expression of the nucleic acid molecule of the present invention or its gene product together with the manipulation of further acitivities such as enzymes wich confer stress tolerance.

The reduction, decrease, deletion or modulation according to this invention can be constitutive, e.g. due to a stable permanent transgenic expression or to a stable mutation in the corresponding endogenous gene encoding the nucleic acid molecule of the invention or to a modulation of the expression or of the behaviour of a gene conferring the expression of the polypeptide of the invention, or transient, e.g. due to an transient transformation, a transiently active promotor or temporary addition of a modulator such as an antagonist or inductor, e.g. after transformation with a inducible construct carrying a double-stranded RNA nucleic acid molecule, an antisense nucleic acid molecule, a ribozyme of the invention etc. under control of a inducible promoter and adding the inducer, e.g. tetracycline or as described herein below.

The reduction, decrease or deletion in activity amounts preferably by at least 10%, preferably by at least 30% or at least 60%, especially preferably by at least 70%, 80%, 85%, 90% or more, very especially preferably are at least 95%, more preferably are at least 99% or more in comparison to the control, reference or wild type. Most preferably the reduction, decrease or deletion in activity amounts to 100%.

In this context, inactivation means that the enzymatic or biological activity of the polypeptides encoded is no longer detectable in the organism or in the cell such as, for example, within the plant or plant cell. For the purposes of the invention, downregulation (= reduction) means that the enzymatic or biological activity of the polypeptides encoded is partly or essentially completely reduced in comparison with the activity of the untreated organism. This can be achieved by different cell-biological mechanisms. In this context, the activity can be downregulated in the entire organism or, in the case of multi-celled organisms, in individual parts of the organism, in the case of plants for example in tissues such as the seed, the leaf, the root or other parts. In this context, the enzymatic activity or biological activity is reduced by at least 10%, advantageously at least 20%, preferably at least 30%, especially preferably at least 40%, 50% or 60%, very especially preferably at least 70%, 80%, 85% or 90% or more, very especially preferably are at least 95%, more preferably are at least 99% or more in comparison to the control, reference or wild type. Most preferably the reduction, decrease or deletion in activity amounts to 100%.

Various strategies for reducing the quantity (≈ expression), the activity or the function of proteins encoded by the nucleic acids or the nucleic acid sequences itself according to the invention are encompassed in accordance with the invention. The skilled worker will recognize that a series of different methods are available for influencing the quantity of a protein, the activity or the function in the desired manner.

The term "biological activity" means the biological function of the protein of the invention. In contrast to the term "biological activity" the term "activity" means the increase in the production of the compound produced by the inventive process. The term "biological activity" preferably refers to the enzymatic function, transporter carrier function, DNA-packaging function, heat shock protein function, recombination protein function, beta-galactosidase function, Serine/threonine-protein kinase CTR1 function, lipase function, enoyl-CoA hydratase function, UDP-glucose glucosyltransferase function, cell division protein function, flavonol synthase function, tracylglycerol lipase, MADS-box protein function, pectinesterase function, pectin metylesterase function, calcium transporting ATPase function, protein kinase function, lysophospholipase function, Chlorophyll A-B binding proteins function, Ca2+-transporting ATPase-like protein function, peroxidase function, disease resistance RPP5 like protein function, or regulatory function of a peptide or protein in an organism, a tissue, a cell or a cell compartment. Suitable substrates are low-molecular-weight compounds and also the protein interaction partners of a protein. The term "reduction" of the biological function refers, for example, to the quantitative reduction in binding capacity or binding strength of a protein for at least one substrate in an organism, a tissue, a cell or a cell compartment - for example by one of the methods described herein below - in comparison with the wild type of the same genus and species to which this method has not been applied, under otherwise identical conditions (such as, for example, culture conditions, age of the plants and the like). Reduction is also understood as meaning the modification of the substrate specificity as can be expressed for example, by the kcat/Km value. In this context, a reduction of the function of at least 10%, advantageously of at least 20%, preferably at least 30%, especially preferably of at least 40%, 50% or 60%, very especially preferably of at least 70%, 80%, 90% or 95%, in comparison with the untreated organism is advantageous. A particularly advantageous embodiment is the inactivation of the function. Binding partners for the protein can be identified in the manner with which the skilled worker is familiar, for example by the yeast 2-hybrid system.

A modification, i.e. a decrease, can be caused by endogenous or exogenous factors. For example, a decrease in activity in an organism or a part thereof can be caused by adding a chemical compound such as an antagonist to the media, nutrition, soil of the plants or to the plants themselves.

The transformed plant cells are compared to the corresponding non-transformed wild type of the same genus and species under otherwise identical conditions (such as, for example, culture conditions, age of the plants and the like). In this context, a change of at least 10%, advantageously of at least 20%, preferably at least 30%, especially preferably of at least 40%, 50% or 60%, very especially preferably of at least 70%, 80%, 90%, 95% or even 100% or more, in comparison with the non-transformed organism is advantageous.

In the invention inactivation or down-regulation of a gene in the plant cell results in altered metabolic activity as compared to a corresponding non-transformed wild type plant cell. One preferred wild type plant cell is a non-transformed Arabidopsis plant cell. An example here is the Arabidopsis wild type C24 (Nottingham Arabidopsis Stock Centre, UK; NASC Stock N906).

Other preferred wild type plant cells are a non-transformed from plants selected from the group consisting of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass and forage crops.

More preferred wild type plant cells are a non-transformed Linum plant cell, preferably Linum usitatissimum, more preferably the variety Brigitta, Golda, Gold Merchant, Helle, Juliel, Olpina, Livia, Marlin, Maedgold, Sporpion, Serenade, Linus, Taunus, Lifax or Liviola, a non-transformed Heliantus plant cell, preferably Heliantus annuus, more preferably the variety Aurasol, Capella, Flavia, Flores, Jazzy, Palulo, Pegasol, PIR64A54, Rigasol, Sariuca, Sideral, Sunny, Alenka, Candisol or Floyd, or a non-transformed Brassica plant cell, preferably Brassica napus, more preferably the variety Dorothy, Evita, Heros, Hyola, Kimbar, Lambada, Licolly, Liconira, Licosmos, Lisonne, Mistral, Passat, Serator, Siapula, Sponsor, Star, Caviar, Hybridol, Baical, Olga, Lara, Doublol, Karola, Falcon, Spirit, Olymp, Zeus, Libero, Kyola, Licord, Lion, Lirajet, Lisbeth, Magnum, Maja, Mendel, Mica, Mohican, Olpop, Ontarion, Panthar, Prinoe, Pronio, Susanna, Talani, Titan, Transfer, Wiking, Woltan, Zeniah, Artus, Contact or Smart.

Inactivation or down-regulation of a gene is advantageous since no new gene must be introduced to achieve the altered metabolic activity resulting in increased tolerance and/or resistance to environmental stress. Only an endogenous gene is hindered in its expression.

The inactivated or down-regulated gene or genes directly or indirectly influence the stress tolerance of plants, preferably the metabolic activity of the transformed plant cells.

Stress tolelance may be confered by one or more inactivated or down-regulated genes encoded by one or more nucleic acid sequences selected from the group consisting of
a) nucleic acid molecule encoding on of the polypeptide shown in SEQ ID NO:54;
b) nucleic acid molecule comprising a nucleic acid sequence, which, as a result of the degeneracy of the genetic code, can be derived from a polypeptide sequence depicted in SEQ ID NO:54;
c) nucleic acid molecule encoding a polypeptide having at least 80% identity with the amino acid sequence of the polypeptide encoded by the nucleic acid molecule of (a) to (c) and having the biological activity represented by protein according to SEQ ID NO:54;
d)
or which comprises a sequence which is complementary thereto.

With the present invention it is possible to identify the genes encoded by a nucleic acid sequence according to SEQ ID NO:53 and/or homologs thereof in target plants, especially crop plants, and then inactivate or down-regulate the corresponding gene to achieve an increased tolerance and/or resistance to environmental stress (prefarably by the altered metabolic activity). Consequently the invention is not limited to a specific plant.

It is further possible to detect environmental stress in plant cells or plants by screening the plant cells for altered metabolic activity as compared to non-stress conditions. This allows for monitoring of stress levels in plants, even when no symptoms are visuable. Therefore counter action can be taken ealier and e.g. crop losses minimized by timely watering.

It is also within the scope of the invention to screen plant cells or plants for increased tolerance and/or resistance to environmental stress by screening the plant cells under stress conditions for increased tolerance and/or resistance to environmental stress as compared to non-stress conditions. This allows selection of plants with increased tolerance and/or resistance to environmental stress without the identification of genes or visual symptoms.

Screening is well known to those skilled in the art and generally refers to the search for a particular attribute or trait. In the invention this trait in a plant or plant cell is preferably the concentration of a metabolite or estimate the general appearance. The methods and devices for screening are familiar to those skilled in the art and include GC (gas chromatography), LC (liquid chromatography), HPLC (high performance (pressure) liquid chromatography), MS (mass spectrometry), NMR (nuclear magnetic resonance) spectroscopy, IR (infra red) spectroscopy, photometric methods etc and combinations of these methods.

Environmental stress includes but is not limited to salinity, drought, temperature, metal, chemical, pathogenic and oxidative stress, or combinations thereof, preferably drought and/or temperature.

As used herein, the term "environmental stress" refers to any sub-optimal growing condition and includes, but is not limited to, sub-optimal conditions associated with salinity, drought, temperature, metal, chemical, pathogenic and oxidative stresses, or combinations thereof. In preferred embodiments, environmental stress may be salinity, drought, heat, or low temperature, or combinations thereof, and in particular, may be low water content or low temperature. Wherein drought stress means any environmental stress which leads to a lack of water in plants or reduction of water supply to plants, wherein low temperature stress means freezing of plants below + 4 °C as well as chilling of plants below 15 °C and wherein high temperature stress means for example a temperature above 35 °C. The range of stress and stress response depends on the different plants which are used for the invention, i.e. it differs for example between a plant such as wheat and a plant such as Arabidopsis. It is also to be understood that as used in the specification and in the claims, "a" or "an" can mean one or more, depending upon the context in which it is used. Thus, for example, reference to "a cell" means that at least one cell may be utilized.

The invention also provides a transformed plant cell with a nucleic acid sequences according to SEQ ID NO:53, wherein the plant is selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.
The present invention further provides a transgenic plant cell with an inactivated or down-regulated gene comprising SEQ ID NO: 53 and/or homologs thereof, preferably from Brassica napus, Glycine max, Zea mays or Oryza sativa..

Furthermore it is possible to identify the genes encoded by a nucleic acid sequence consisting of SEQ ID NO:53 and/or homologs thereof in target plants, especially crop plants, and then inactivate or down-regulate the corresponding gene to achieve increased tolerance and/or resistance to environmental stress. Consequently the invention is not limited to a specific plant.

The invention also provides a transformed plant cell with a nucleic acid sequence according to SEQ ID NO:53, wherein the plant is selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

According to the invention the transformed plant cell may be derived from a monocotyledonous or a dicotyledonous plant.

The monocotyledonous or a dicotyledonous plant may be selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

The transformed plant cell may be derived from a gymnosperm plant and can preferably be selected from the group of spruce, pine and fir.

The invention also provides a transformed plant generated from said plant cell and which is a monocot or dicot plant.

The transformed plant may be selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

Preferably the transformed plant generated from said plant cell is a gymnosperm plant, more preferred a plant selected from the group consisting of spruce, pine and fir.

The invention not only deals with plants but also with an agricultural product produced by any of the described transformed plants, plant parts such as leafs, petal, anther, roots, tubers, stems, buds, flowers or especially seeds produced by said transformed plant, which are at least genetically heterozygous, preferably homozygous for a gene or its homolog, that when inactivated or down-regulated confers an increased tolerance and/or resistance to environmental stress as compared to a wild type plant.

Homologs of the aforementioned sequences can be isolated advantageously from yeast, fungi, viruses, algae bacteria, such as Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosteroni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilla sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis, preferably Salmonella sp. or Escherichia coli or plants, preferably from yeasts such as from the genera Saccharomyces, Pichia, Candida, Hansenula, Torulopsis or Schizosaccharomyces, or even more preferred from plants such as Arabidopsis thaliana, maize, wheat, rye, oat, triticale, rice, barley, soybean, peanut, cotton, borage, safflower, linseed, primrose, rapeseed, canola and turnip rape, manihot, pepper, sunflower, tagetes, solanaceous plant such as potato, tobacco, eggplant and tomato, Vicia species, pea, alfalfa, bushy plants such as coffee, cacao, tea, Salix species, trees such as oil palm, coconut, perennial grass, such as ryegrass and fescue, and forage crops, such as alfalfa and clover and from spruce, pine or fir for example, more preferably from Saccharomyces cerevisiae or plants, preferably Brassica napus, Glycine max, Zea mays or Oryza sativa.

"Homologs" are defined herein as two nucleic acids or proteins that have similar, or "homologous", nucleotide or amino acid sequences, respectively. Homologs include allelic variants, orthologs, paralogs, agonists and antagonists of SRP as defined hereafter. The term "homolog" further encompasses nucleic acid molecules that differ from one of the nucleotide sequences shown in sequences of SEQ ID NO:53 (and portions thereof) due to degeneracy of the genetic code and thus encode the same SRP as that encoded by the amino acid sequences shown in SEQ ID NO:54. As used herein a "naturally occurring" SRP refers to a SRP amino acid sequence that occurs in nature.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

Functional equivalents derived from one of the polypeptides as shown in SEQ ID NO:54 according to the invention by substitution, insertion or deletion have at least 80%, especially preferably at least 85% or 90%, 91%, 92%, 93% or 94%, very especially preferably at least 95%, 97%, 98% or 99% homology with one of the polypeptides as shown in SEQ ID NO:54 according to the invention and are distinguished by essentially the same properties as the polypeptide as shown in SEQ ID NO:54.

Functional equivalents derived from the nucleic acid sequence according to SEQ ID NO:53 according to the invention by substitution, insertion or deletion have at least 80%, especially preferably at least 85% or 90%, 91%, 92%, 93% or 94%, very especially preferably at least 95%, 97%, 98% or 99% homology with one of the polypeptides as shown in SEQ ID NO:54 according to the invention and encode polypeptides having essentially the same properties as the polypeptide as shown in SEQ ID NO:54.

"Essentially the same properties" of a functional equivalent is above all understood as meaning that the functional equivalent has above mentioned acitivty, e.g conferring an increase in the fine chemical amount while increasing the amount of protein, activity or function of said functional equivalent in an organism, e.g. a microorgansim, a plant or plant or animal tissue, plant or animal cells or a part of the same.

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

According to the invention, DNA as well as RNA molecules of the nucleic acid of the invention can be used as probes. Further, as template for the identification of functional homologues Northern blot assays as well as Southern blot assays can be performed. The Northern blot assay advantageously provides further informations about the expressed gene product: e.g. expression pattern, occurance of processing steps, like splicing and capping, etc. The Southern blot assay provides additional information about the chromosomal localization and organization of the gene encoding the nucleic acid molecule of the invention.

A preferred, nonlimiting example of stringent hydridization conditions are hybridizations in 6 x sodium chloride/sodium citrate (= SSC) at approximately 45°C, followed by one or more wash steps in 0.2 x SSC, 0.1% SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.

Thus, in a preferred embodiment, Northern blots are prehybridized with Rothi-Hybri-Quick buffer (Roth, Karlsruhe) at 68°C for 2h. Hybridzation with radioactive labelled probe is done overnight at 68°C. Subsequent washing steps are performed at 68°C with 1xSSC.

For Southern blot assays the membrane is prehybridized with Rothi-Hybri-Quick buffer (Roth, Karlsruhe) at 68°C for 2h. The hybridzation with radioactive labelled probe is conducted over night at 68°C. Subsequently the hybridization buffer is discarded and the filter shortly washed using 2xSSC; 0,1% SDS. After discarding the washing buffer new 2xSSC; 0,1% SDS buffer is added and incubated at 68°C for 15 minutes. This washing step is performed twice followed by an additional washing step using 1xSSC; 0,1% SDS at 68°C for 10 min.
Some further examples of conditions for DNA hybridization (Southern blot assays) and wash step are shown hereinbelow:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1 % bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCl/0.0015 M sodium citrate/0.1 % SDS at 50°C.
   b) 0.1XSSC at 65°C.
   c) 0.1X SSC, 0.5 % SDS at 68°C.
   d) 0.1X SSC, 0.5% SDS, 50% formamide at 42°C.
   e) 0.2X SSC, 0.1% SDS at 42°C.
   f) 2X SSC at 65°C (low-stringency condition).

With regard to the invention described here, "transformed" means all those plants or parts thereof which have been brought about and/or modified by manipulation methods and in which either
a) one or more genes, encoded by one or more nucleic acid sequence as depicted in SEQ ID NO:53 or a homolog thereof, or
b) a genetic regulatory element or elements, for example promoters, which are functionally linked e.g. to a nucleic acid sequence of SEQ ID NO:53 or a homolog thereof, or
c) (a) and (b)
is/are not present in its/their natural genetic environment and/or has/have been modified by means of manipulation methods.

It is possible for the modification to be, by way of example, a substitution, addition, deletion, inversion or insertion of one or more nucleotides.
Manipulation in the present invention is also meant to encompass all changes in the plant cell, including induced or non-induced (spontaneous) mutagenesis, directed or non-directed genetic manipulation by conventional breeding or by modern genetic manipulation methods, e. g. reduction of gene expression by double-stranded RNA interference (dsRNAi), introduction of an antisense nucleic acid, a ribozyme, an antisense nucleic acid combined with a ribozyme, a nucleic acid encoding a co-suppressor, a nucleic acid encoding a dominant negative protein, DNA- or RNA- or protein-binding factors targeting said gene or -RNA or -proteins, RNA degradation inducing viral nucleic acids and expression systems, systems for inducing a homolog recombination of said genes, mutations in said genes or a combination of the above. Additional modifications and manipulation methods will become apparent from the further description.

"Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

A plant or plant cell is considered "true breeding" for a particular attribute if it is genetically homozygous for that attribute to the extent that, when the true-breeding plant is self-pollinated, a significant amount of independent segregation of the attribute among the progeny is not observed.

As also used herein, the terms "nucleic acid" and "nucleic acid molecule" are intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. The nucleic acid molecule can be single-stranded or double-stranded, but preferably is double-stranded DNA.

An "isolated" nucleic acid molecule is one that is substantially separated from other nucleic acid molecules, which are present in the natural source of the nucleic acid. That means other nucleic acid molecules are present in an amount less than 5% based on weight of the amount of the desired nucleic acid, preferably less than 2% by weight, more preferably less than 1 % by weight, most preferably less than 0.5% by weight. Preferably, an "isolated" nucleic acid is free of some of the sequences that naturally flank the nucleic acid (i.e., sequences located at the 5' and 3' ends of the nucleic acid) in the genomic DNA of the organism from which the nucleic acid is derived. For example, in various embodiments, the isolated gene encoding nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

A nucleic acid molecule of the present invention, i. e., a nucleic acid molecule encoding a gene or a portion thereof according to SEQ ID NO:54 which confers tolerance and/or resistance to environmental stress in plants, when inactivated or down-regulated, can be isolated using standard molecular biology techniques and the sequence information provided herein. For example, an Arabidopsis thaliana gene encoding cDNA can be isolated from an A. thaliana library using all or portion of the nucleic acid according to SEQ ID NO:53. Moreover, a nucleic acid molecule encompassing all or a portion of one of the sequence of SEQ ID NO:53 can be isolated by the polymerase chain reaction using oligonucleotide primers designed based upon this sequence. For example, mRNA can be isolated from plant cells (e.g., by the guanidinium-thiocyanate extraction procedure of Chirgwin et al., 1979 Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences according to SEQ ID NO:53. A nucleic acid molecule of the invention can be amplified using cDNA or, alternatively, genomic DNA, as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to a gene encoding nucleotide sequence can be prepared by standard synthetic techniques, e.g., using an automated DNA synthesizer.

The nucleic acid molecule of the invention comprises one of the nucleotide sequences shown in sequences of SEQ ID NO:53 or homologs thereof encoding a gene (i.e., the "coding region"), as well as 5' untranslated sequences and 3' untranslated sequences.

Moreover, the nucleic acid molecule of the invention can comprise only a portion of the coding region of SEQ ID NO:53 or homologs thereof, for example, a fragment encoding a biologically active portion of a gene.
Portions of genes or proteins encoded by said gene encoding nucleic acid molecules of the invention are preferably biologically active portions of genes or proteins described herein. As used herein, the term "biologically active portion of" a gene or protein encoded by said gene is intended to include a portion, e.g., a domain/motif, of the gene or protein that participates in stress tolerance and/or resistance response in a plant, which is preferably achieved by altering metabolic activity, and finally resulting in increased tolerance and/or resistance to environmental stress. To determine whether inactivation or down-regulation of a gene or protein encoded by said gene, or a biologically active portion thereof, results in increased stress tolerance in a plant, which is preferably achieved by altering metabolic activity, a stress analysis of a plant comprising the protein may be performed for example by the above screening method or by estimating the general appearance and health. More specifically, nucleic acid fragments encoding biologically active portions of a gene or protein encoded by said gene can be prepared by isolating a portion of the nucleic acid according to SEQ ID NO:53 or homologs thereof expressing the encoded portion of the gene, protein or peptide (e.g., by recombinant expression in vitro) and assessing the activity of the encoded portion of the gene, protein or peptide.

Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin.

Typically portions of a protein encompassed by the present invention include peptides comprising amino acid sequences derived from the amino acid sequence of the protein encoded by SEQ ID NO:54, which include fewer amino acids than the full length protein or a full length protein which is homologous to the protein, and exhibits at least some activity of the protein. Prefered portions according to the present invention (e.g., peptides or proteins which are, for example, 5, 10, 15, 20, 30, 35, 36, 37, 38, 39, 40, 50, 100 or more amino acids in length) comprise a domain or motif with at least some activity of the protein. Moreover, other biologically active portions in which other regions of the protein are deleted can be prepared by recombinant techniques and evaluated for one or more of the activities described herein. Preferably, the biologically active portions of the protein include one or more selected domains/motifs or portions thereof having biological activity.

"Homologs" are defined herein as two nucleic acids or proteins that have similar, or "homologous", nucleotide or amino acid sequences, respectively. Homologs include allelic variants, orthologs, paralogs, agonists and antagonists of the protein as defined hereafter. The term "homolog" further encompasses nucleic acid molecules that differ from one of the nucleotide sequences shown in SEQ ID NO:53 (and portions thereof) due to degeneracy of the genetic code and thus encode the same protein as that encoded by the amino acid sequences. As used herein a "naturally occurring" refers to an amino acid sequence that occurs in nature.

In addition to fragments and fusion polypeptides of the invention described herein, the present invention includes homologs and analogs of naturally occurring proteins and protein encoding nucleic acids of the invenion in a plant. "Homologs" are defined herein as two nucleic acids or polypeptides that have similar, or substantially identical, nucleotide or amino acid sequences, respectively. Homologs include allelic variants, orthologs, paralogs, agonists and antagonists of SRPs as defined hereafter. The term "homolog" further encompasses nucleic acid molecules that differ from one of the nucleotide sequences shown in SEQ ID NO:53 (and portions thereof) due to degeneracy of the genetic code and thus encode the same SRP as that encoded by the nucleotide sequences shown in SEQ ID NO:53 having at least 80% identity. As used herein a "naturally occurring" protein refers to amino acid sequence that occurs in nature. Preferably, a naturally occurring protein whose reduction or deletion results in increased tolerance and/or resistance to an environmental stress comprises amino acid sequence SEQ ID NO:54.

An agonist of the protein whose reduction or deletion results in increased tolerance and/or resistance to an environmental stress can retain substantially the same, or a subset, of the biological activities of the said protein. An antagonist of the said protein can inhibit one or more of the activities of the naturally occurring form of the protein whose reduction or deletion results in increased tolerance and/or resistance to an environmental stress. For example, an antagonist can competitively bind to a downstream or upstream member of the cell membrane component metabolic cascade that includes said protein, or bind to the protein of the invention that mediates transport of compounds across such membranes, thereby preventing translocation from taking place.

Nucleic acid molecules corresponding to natural allelic variants and analogs, orthologs and paralogs of a protein of the invention cDNA can be isolated based on their identity to the Arabidopsis thaliana, Saccharomyces cerevisiae, E.coli, , preferably Brassica napus, Glycine max, Zea mays or Oryza sativa protein nucleic acids described herein using said proteins cDNAs, or a portion thereof, as a hybridization probe according to standard hybridization techniques under stringent hybridization conditions. In an alternative embodiment, homologs of the protein whose reduction or deletion results in increased tolerance and/or resistance to an environmental stress can be identified by screening combinatorial libraries of mutants, e.g., truncation mutants, of said protein for their agonist or antagonist activity. In one embodiment, a variegated library of protein whose reduction or deletion results in increased tolerance and/or resistance to an environmental stress variants is generated by combinatorial mutagenesis at the nucleic acid level and is encoded by a variegated gene library. A variegated library of SRP variants can be produced by, for example, enzymatically ligating a mixture of synthetic oligonucleotides into gene sequences such that a degenerate set of potential SRP sequences is expressible as individual polypeptides, or alternatively, as a set of larger fusion polypeptides (e.g., for phage display) containing the set of protein whose reduction or deletion results in increased tolerance and/or resistance to an environmental stress sequences therein. There are a variety of methods that can be used to produce libraries of potential protein homologs from a degenerate oligonucleotide sequence. Chemical synthesis of a degenerate gene sequence can be performed in an automatic DNA synthesizer, and the synthetic gene is then ligated into an appropriate expression vector. Use of a degenerate set of genes allows for the provision, in one mixture, of all of the sequences encoding the desired set of potential protein of the invention sequences. Methods for synthesizing degenerate oligonucleotides are known in the art. See, e.g., Narang, S.A., 1983, Tetrahedron 39:3; Itakura et al., 1984, Annu. Rev. Biochem. 53:323; Itakura et al., 1984, Science 198:1056; Ike et al., 1983, Nucleic Acid Res. 11:477.

In addition, libraries of fragments of the protein of the invention coding regions can be used to generate a variegated population of protein fragments for screening and subsequent selection of homologs of a said proteins. In one embodiment, a library of coding sequence fragments can be generated by treating a double stranded PCR fragment of a protein of the invention coding sequence with a nuclease under conditions wherein nicking occurs only about once per molecule, denaturing the double stranded DNA, renaturing the DNA to form double stranded DNA, which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal, and internal fragments of various sizes of the protein whose reduction or deletion results in increased tolerance and/or resistance to an environmental stress.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of SRP homologs. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify SRP homologs (Arkin and Yourvan, 1992, PNAS 89:7811-7815; Delgrave et al., 1993, Polypeptide Engineering 6(3):327-331). Cell based assays can be exploited to analyze a variegated protein (of the invention) library, using methods well known in the art. The present invention further provides a method of identifying a novel protein whose reduction or deletion results in increased tolerance and/or resistance to an environmental stress, comprising (a) raising a specific antibody response to said protein, or a fragment thereof, as described herein; (b) screening putative SRP material with the antibody, wherein specific binding of the antibody to the material indicates the presence of a potentially novel protein whose reduction or deletion results in increased tolerance and/or resistance to an environmental stress; and (c) analyzing the bound material in comparison to known proteins, to determine its novelty.

As stated above, the present invention includes protein whose reduction or deletion results in increased tolerance and/or resistance to an environmental stress and homologs thereof. To determine the percent sequence identity of two amino acid sequences (e.g., SEQ ID NO:54, and a mutant form thereof), the sequences are aligned for optimal comparison purposes (e.g., gaps can be introduced in the sequence of one polypeptide for optimal alignment with the other polypeptide or nucleic acid). The amino acid residues at corresponding amino acid positions are then compared. When a position in one sequence (e.g., one of the sequences of SEQ ID NO:54) is occupied by the same amino acid residue as the corresponding position in the other sequence (e.g., a mutant form of the sequence selected from the polypeptide of SEQ ID NO:54), then the molecules are identical at that position. The same type of comparison can be made between two nucleic acid sequences.

The percent sequence identity between the two sequences is a function of the number of identical positions shared by the sequences (i.e., percent sequence identity = numbers of identical positions/total numbers of positions x 100). Preferably, the isolated amino acid homologs included in the present invention are at least about 80%, 80-85%, 85-90% or 90-95%, and most preferably at least about 96%, 97%, 98%, 99% or more identical to an entire amino acid sequence shown in SEQ ID NO:54. In yet another embodiment, the isolated amino acid homologs included in the present invention are at least about 80%, 80-85%, 85-90% or 90-95%, and most preferably at least about 96%, 97%, 98%, 99% or more identical to an entire amino acid sequence encoded by a nucleic acid sequence according to SEQ ID NO:53.

In another preferred embodiment, a nucleic acid homolog of the invention comprises a nucleotide sequence which is at least about 80-85%, 85-90% or 90-95%, and even more preferably at least about 95%, 96%, 97%, 98%, 99% or more identical to a nucleotide sequence according to SEQ ID NO:53.

It is further preferred that the isolated nucleic acid homolog of the invention encodes a SRP, or portion thereof, that is at least 85% identical to an amino acid sequence of SEQ ID NO:54 and that functions as a modulator of an environmental stress response in a plant. In a more preferred embodiment, overexpression of the nucleic acid homolog in a plant increases the tolerance of the plant to an environmental stress.

For the purposes of the invention, the percent sequence identity between two nucleic acid or polypeptide sequences may be determined using the Vector NTI 6.0 (PC) software package (InforMax, 7600 Wisconsin Ave., Bethesda, MD 20814). A gap opening penalty of 15 and a gap extension penalty of 6.66 are used for determining the percent identity of two nucleic acids. A gap opening penalty of 10 and a gap extension penalty of 0.1 are used for determining the percent identity of two polypeptides. All other parameters are set at the default settings. For purposes of a multiple alignment (Clustal W algorithm), the gap opening penalty is 10, and the gap extension penalty is 0.05 with blosum62 matrix. It is to be understood that for the purposes of determining sequence identity when comparing a DNA sequence to an RNA sequence, a thymidine nucleotide is equivalent to a uracil nucleotide.

As used herein with regard to hybridization for DNA to DNA blot, the term "stringent conditions" refers in one embodiment to hybridization overnight at 60°C in 10X Denharts solution, 6X SSC, 0.5% SDS and 100 µg/ml denatured salmon sperm DNA. Blots are washed sequentially at 62°C for 30 minutes each time in 3X SSC/0.1% SDS, followed by 1X SSC/0.1% SDS and finally 0.1X SSC/0.1% SDS. As also used herein, "highly stringent conditions" refers to hybridization overnight at 65°C in 10X Denharts solution, 6X SSC, 0.5% SDS and 100 µg/ml denatured salmon sperm DNA. Blots are washed sequentially at 65°C for 30 minutes each time in 3X SSC/0.1% SDS, followed by 1X SSC/0.1% SDS and finally 0.1X SSC/0.1% SDS. Methods for nucleic acid hybridizations are described in Meinkoth and Wahl, 1984, Anal. Biochem. 138:267-284; Ausubel et al. eds, 1995, Current Protocols in Molecular Biology, Chapter 2, Greene Publishing and Wiley-Interscience, New York; and Tijssen, 1993, Laboratory Techniques in Biochemistry and Molecular Biology: Hybridization with Nucleic Acid Probes, Part I, Chapter 2, Elsevier, New York. Preferably, an nucleic acid molecule of the invention that hybridizes under stringent or highly stringent conditions to a sequence of SEQ ID NO:53 corresponds to a naturally occurring nucleic acid molecule. As used herein, a "naturally occurring" nucleic acid molecule refers to an RNA or DNA molecule having a nucleotide sequence that occurs in nature (e.g., encodes a natural polypeptide). In one embodiment, the nucleic acid encodes a naturally occurring Arabidopsis thaliana, Brassica napus, Glycine max, Zea mays or Oryza sativa protein whose reduction or deletion results in increased tolerance and/or resistance to an environmental stress.

Using the above-described methods, and others known to those of skill in the art, one of ordinary skill in the art can isolate homologs of the protein whose reduction or deletion results in increased tolerance and/or resistance to an environmental stress comprising amino acid sequences shown in SEQ ID NO:53. One subset of these homologs are allelic variants. As used herein, the term "allelic variant" refers to a nucleotide sequence containing polymorphisms that lead to changes in the amino acid sequences of said protein and that exist within a natural population (e.g., a plant species or variety). Such natural allelic variations can typically result in 1-5% variance in a nucleic acid of the invention. Allelic variants can be identified by sequencing the nucleic acid sequence of interest in a number of different plants, which can be readily carried out by using hybridization probes to identify the same SRP genetic locus in those plants. Any and all such nucleic acid variations and resulting amino acid polymorphisms or variations in a protein whose reduction or deletion results in increased tolerance and/or resistance to an environmental stress that are the result of natural allelic variation and that do not alter the functional activity of said protein, are intended to be within the scope of the invention.

A nucleic acid molecule encoding a protein whose reduction or deletion results in increased tolerance and/or resistance to an environmental stress having at least 80% sequence identity with a polypeptide sequence according to SEQ ID NO:54 can be created by introducing one or more nucleotide substitutions, additions or deletions into a nucleotide sequence of SEQ ID NO:53, respectively, such that one or more amino acid substitutions, additions, or deletions are introduced into the encoded polypeptide. Mutations can be introduced into one of the sequences of SEQ ID NO:53 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain.

To knock the mutation is carried out preferably at essential positions.

Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a protein of the invention is preferably replaced with another amino acid residue from the same side chain family. Alternatively, in another embodiment, mutations can be introduced randomly along all or part of a protein of the invention coding sequence, such as by saturation mutagenesis, and the resultant mutants can be screened for a SRP activity described herein to identify mutants that retain protein activity. Following mutagenesis of one of the sequences of SEQ ID NO:53, the encoded polypeptide can be expressed recombinantly and the activity of the polypeptide can be determined by analyzing the stress tolerance of a plant expressing the polypeptide as described herein.

In addition to the nucleic acid molecules encoding the protein whose reduction or deletion results in increased tolerance and/or resistance to an environmental stress described above, another aspect of the invention pertains to isolated nucleic acid molecules that are antisense thereto. Antisense polynucleotides are thought to inhibit gene expression of a target polynucleotide by specifically binding the target polynucleotide and interfering with transcription, splicing, transport, translation, and/or stability of the target polynucleotide. Methods are described in the prior art for targeting the antisense polynucleotide to the chromosomal DNA, to a primary RNA transcript, or to a processed mRNA. Preferably, the target regions include splice sites, translation initiation codons, translation termination codons, and other sequences within the open reading frame.

The term "antisense," for the purposes of the invention, refers to a nucleic acid comprising a polynucleotide that is sufficiently complementary to all or a portion of a gene, primary transcript, or processed mRNA, so as to interfere with expression of the endogenous gene. "Complementary" polynucleotides are those that are capable of base pairing according to the standard Watson-Crick complementarity rules. specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. The term "antisense nucleic acid" includes single stranded RNA as well as double-stranded DNA expression cassettes that can be transcribed to produce an antisense RNA. "Active" antisense nucleic acids are antisense RNA molecules that are capable of selectively hybridizing with a primary transcript or mRNA encoding a polypeptide having at least 80% sequence identity with the polypeptide of SEQ ID NO:54.

The antisense nucleic acid can be complementary to an entire SRP coding strand, or to only a portion thereof. In one embodiment, an antisense nucleic acid molecule is antisense to a "coding region" of the coding strand of a nucleotide sequence encoding a SRP. The term "coding region" refers to the region of the nucleotide sequence comprising codons that are translated into amino acid residues. In another embodiment, the antisense nucleic acid molecule is antisense to a "noncoding region" of the coding strand of a nucleotide sequence encoding a SRP. The term "noncoding region" refers to 5' and 3' sequences that flank the coding region that are not translated into amino acids (i.e., also referred to as 5' and 3' untranslated regions). The antisense nucleic acid molecule can be complementary to the entire coding region of SRP mRNA, but more preferably is an oligonucleotide which is antisense to only a portion of the coding or noncoding region of SRP mRNA. For example, the antisense oligonucleotide can be complementary to the region surrounding the translation start site of PKSRP mRNA. An antisense oligonucleotide can be, for example, about 5, 10, 15, 20, 25, 30, 35, 40, 45 or 50 nucleotides in length. Typically, the antisense molecules of the present invention comprise an RNA having 60-100% sequence identity with at least 14 consecutive nucleotides of SEQ ID NO:53. Preferably, the sequence identity will be at least 70%, more preferably at least 75%, 80%, 85%, 90%, 95%, 98% and most preferably 99%.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-ca1rboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al., 1987, Nucleic Acids. Res. 15:6625-6641). The antisense nucleic acid molecule can also comprise a 2'-o-methylribonucleotide (Inoue et al., 1987, Nucleic Acids Res. 15:6131-6148) or a chimeric RNA-DNA analogue (Inoue et al., 1987, FEBS Lett. 215:327-330).

The antisense nucleic acid molecules of the invention are typically administered to a cell or generated in situ such that they hybridize with or bind to cellular mRNA and/or genomic DNA encoding a SRP to thereby inhibit expression of the polypeptide, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve sufficient intracellular concentrations of the antisense molecules, vector constructs in which the antisense nucleic acid molecule is placed under the control of a strong prokaryotic, viral, or eukaryotic (including plant) promoter are preferred.

As an alternative to antisense polynucleotides, ribozymes, sense polynucleotides, or double stranded RNA (dsRNA) can be used to reduce expression of a SRP polypeptide. By "ribozyme" is meant a catalytic RNA-based enzyme with ribonuclease activity which is capable of cleaving a single-stranded nucleic acid, such as an mRNA, to which it has a complementary region. Ribozymes (e.g., hammerhead ribozymes described in Haselhoff and Gerlach, 1988, Nature 334:585-591) can be used to catalytically cleave proteins of the invention mRNA transcripts to thereby inhibit translation of SRP mRNA. A ribozyme having specificity for a nucleic acid of the invention can be designed based upon the nucleotide sequence of a cDNA, as disclosed herein (i.e., sequences according to SEQ ID NO:53) or on the basis of a heterologous sequence to be isolated according to methods taught in this invention. For example, a derivative of a Tetrahymena L-19 IVS RNA can be constructed in which the nucleotide sequence of the active site is complementary to the nucleotide sequence to be cleaved in a SRP-encoding mRNA. See, e.g., U.S. Patent Nos. 4,987,071 and 5,116,742 to Cech et al. Alternatively, SRP mRNA can be used to select a catalytic RNA having a specific ribonuclease activity from a pool of RNA molecules. See, e.g., Bartel, D. and Szostak, J.W., 1993, Science 261:1411-1418. In preferred embodiments, the ribozyme will contain a portion having at least 7, 8, 9, 10, 12, 14, 16, 18 or 20 nucleotides, and more preferably 7 or 8 nucleotides, that have 100% complementarity to a portion of the target RNA. Methods for making ribozymes are known to those skilled in the art. See, e.g., U.S. Patent Nos. 6,025,167; 5,773,260; and 5,496,698.

The term "dsRNA," as used herein, refers to RNA hybrids comprising two strands of RNA. The dsRNAs can be linear or circular in structure. In a preferred embodiment, dsRNA is specific for a polynucleotide encoding either the polypeptide of SEQ ID NO:54 or a polypeptide having at least 70% sequence identity with SEQ ID NO:54. The hybridizing RNAs may be substantially or completely complementary. By "substantially complementary," is meant that when the two hybridizing RNAs are optimally aligned using the BLAST program as described above, the hybridizing portions are at least 95% complementary. Preferably, the dsRNA will be at least 100 base pairs in length. Typically, the hybridizing RNAs will be of identical length with no over hanging 5' or 3' ends and no gaps. However, dsRNAs having 5' or 3' overhangs of up to 100 nucleotides may be used in the methods of the invention.

The dsRNA may comprise ribonucleotides or ribonucleotide analogs, such as 2'-O-methyl ribosyl residues, or combinations thereof. See, e.g., U.S. Patent Nos. 4,130,641 and 4,024,222. A dsRNA polyriboinosinic acid:polyribocytidylic acid is described in U.S. patent 4,283,393. Methods for making and using dsRNA are known in the art. One method comprises the simultaneous transcription of two complementary DNA strands, either in vivo, or in a single in vitro reaction mixture. See, e.g., U.S. Patent No. 5,795,715. In one embodiment, dsRNA can be introduced into a plant or plant cell directly by standard transformation procedures. Alternatively, dsRNA can be expressed in a plant cell by transcribing two complementary RNAs. Other methods for the inhibition of endogenous gene expression, such as triple helix formation (Moser et al., 1987, Science 238:645-650 and Cooney et al., 1988, Science 241:456-459) and cosuppression (Napoli et al., 1990, The Plant Cell 2:279-289) are known in the art. Partial and full-length cDNAs have been used for the cosuppression of endogenous plant genes. See, e.g., U.S. Patent Nos. 4,801,340, 5,034,323, 5,231,020, and 5,283,184; Van der Kroll et al., 1990, The Plant Cell 2:291-299; Smith et al., 1990, Mol. Gen. Genetics 224:477-481 and Napoli et al., 1990, The Plant Cell 2:279-289.

For sense suppression, it is believed that introduction of a sense polynucleotide blocks transcription of the corresponding target gene. The sense polynucleotide will have at least 65% sequence identity with the target plant gene or RNA. Preferably, the percent identity is at least 80%, 90%, 95% or more. The introduced sense polynucleotide need not be full length relative to the target gene or transcript. Preferably, the sense polynucleotide will have at least 65% sequence identity with at least 100 consecutive nucleotides of SEQ ID NO:53. The regions of identity can comprise introns and and/or exons and untranslated regions. The introduced sense polynucleotide may be present in the plant cell transiently, or may be stably integrated into a plant chromosome or extrachromosomal replicon.

Moreover, nucleic acid molecules encoding proteins from the same or other species such as protein analogs, orthologs and paralogs, are intended to be within the scope of the present invention. As used herein, the term "analogs" refers to two nucleic acids that have the same or similar function, but that have evolved separately in unrelated organisms. As used herein, the term "orthologs" refers to two nucleic acids from different species that have evolved from a common ancestral gene by speciation. Normally, orthologs encode proteins having the same or similar functions. As also used herein, the term "paralogs" refers to two nucleic acids that are related by duplication within a genome. Paralogs usually have different functions, but these functions may be related (Tatusov, R.L. et al. 1997 Science 278(5338):631-637). Analogs, orthologs and paralogs of naturally occurring proteins can differ from the naturally occurring proteins by post-translational modifications, by amino acid sequence differences, or by both. Post-translational modifications include in vivo and in vitro chemical derivatisation of polypeptides, e.g., acetylation, carboxylation, phosphorylation, or glycosylation, and such modifications may occur during polypeptide synthesis or processing or following treatment with isolated modifying enzymes. In particular, orthologs of the invention will generally exhibit at least 80% and most preferably 90%, 91%, 92%, 93%, 94%, 95%, 96%, 97%, 98% or even 99% identity or homology with all or part of a naturally occurring protein amino acid sequence and will exhibit a function similar to a protein.

Such homologs, analogs, orthologs and paralogs will be referred to in general as homologs or being homologous throughout the present application. Homologs of the sequences given in SEQ ID NO:54 are furthermore to be understood as meaning, for example, homologs, analogs, orthologs and paralogs which have at least 30% homology (= identity) at the derived amino acid level, preferably at least 50 %, 60 %, 70 % or 80 % homology, especially preferably at least 85 % homology, very especially preferably 90 %, 91%, 92%, 93%, 94%, homology, most preferably 95 %, 96 %, 97 %, 98 % or 99 % homology. The homology (= identity) was calculated over the entire amino acid range. The program used was PileUp (J. Mol. Evolution., 25 (1987), 351 - 360, Higgens et al., CABIOS, 5 1989: 151 -153) or the program Gap and BestFit [Needleman and Wunsch (J. Mol. Biol. 48; 443 - 453 (1970) and Smith and Waterman respectively (Adv. Appl. Math. 2; 482 - 489 (1981)] which are part of the GCG software package [Genetics Computer Group, 575 Science Drive, Madison, Wisconsin, USA 53711 (1991)]. The above mentioned percentages of sequence homology are calculated with the program BestFit or Gap, preferably Gap, over the total sequence length with the following parameters used: Gap Weight: 8, Length Weight: 2.
Furthermore the invention provides a method of producing a transformed plant, wherein inactivation or down-regulation of a gene in the transformed plant results in increased tolerance and/or resistance to environmental stress as compared to a corresponding non-transformed wild type plant, comprising
(a) transforming a plant cell by inactivation or down-regulation of one or more genes, encoded by one or more nucleic acids selected from
   aa) nucleic acid molecule encoding the polypeptide-according to SEQ ID NO:54;
   bb) nucleic acid molecule comprising the nucleic acid molecule according to SEQ ID NO:53;
   cc) nucleic acid molecule comprising a nucleic acid sequence, which, as a result of the degeneracy of the genetic code, can be derived from a polypeptide sequence depicted in SEQ ID NO:54;
   dd) nucleic acid molecule encoding a polypeptide having at least 80% identity with the amino acid sequence of the polypeptide encoded by the nucleic acid molecule of (aa) to (cc) and having the biological activity represented by protein of SEQ ID NO:54;
   and
(b) generating from the plant cell a transformed plant with an increased tolerance and/or resistance to environmental stress as compared to a corresponding wild type plant.
Furthermore the invention provides a transformed plant cell with enhanced photosynthetic yield as compared to a corresponding non-transformed wild type plant cell, wherein the enhanced photosynthetic yield is increased by an inactivated or down-regulated gene encoded by one or more nucleic acid sequences selected from the group consisting of:
a) nucleic acid molecule encoding the polypeptide according to SEQ ID NO:54;
b) nucleic acid molecule comprising the nucleic acid molecule according to SEQ ID NO:53;
c) nucleic acid molecule comprising a nucleic acid sequence, which, as a result of the degeneracy of the genetic code, can be derived from a polypeptide sequence depicted in SEQ ID NO:54;
d) nucleic acid molecule encoding a polypeptide having at least 80% identity with the amino acid sequence of the polypeptide encoded by the nucleic acid molecule of (a) to (c) and having the biological activity represented by protein of SEQ ID NO:54;
or which comprises a sequence which is complementary thereto.

The invention also incorporates a method of inducing increased tolerance and/or resistance to environmental stress as compared to a corresponding non-transformed wild type plant in said plant cell or said plant by inactivation or down-regulation of one or more genes encoded by one or more nucleic acids selected from a group consisting of the nucleic acids according to SEQ ID NO:53 and/or homologs thereof.

Preferably the nucleic acid is at least 80% homologous to said sequence (see above). It is also possible that the homolog sequence stems form a plant selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

Inactivation or down-regulation of said gene or genes may be achieved by all methods known to one skilled in the art, preferably by double-stranded RNA interference (dsRNAi), introduction of an antisense nucleic acid, a ribozyme, an antisense nucleic acid combined with a ribozyme, a nucleic acid encoding a co-suppressor, a nucleic acid encoding a dominant negative protein, DNA- or protein-binding factors targeting said gene or -RNA or -proteins, RNA degradation inducing viral nucleic acids and expression systems, systems for inducing a homolog recombination of said genes, mutations in said genes or a combination of the above.

The nucleic acid sequences of the invention or their homologs are isolated nucleic acid sequences which encode polypeptides. These nucleic acids or the polypeptides encoded by them and their biological and enzymatic activity are inactivated or downregulated in the method according to the invention which leads to increased resistance and/or tolerance to environmental stress.

In this context, inactivation means that the enzymatic or biological activity of the polypeptides encoded is no longer detectable in the organism or in the cell such as, for example, within the plant or plant cell. For the purposes of the invention, downregulation (= reduction) means that the enzymatic or biological activity of the polypeptides encoded is partly or essentially completely reduced in comparison with the activity of the untreated organism. This can be achieved by different cell-biological mechanisms. In this context, the activity can be downregulated in the entire organism or, in the case of multi-celled organisms, in individual parts of the organism, in the case of plants for example in tissues such as the seed, the leaf, the root or other parts. In this context, the enzymatic activity or biological activity is reduced by at least 10%, advantageously at least 20%, preferably at least 30%, especially preferably at least 40%, 50% or 60%, very especially preferably at least 70%, 80%, 90% or 95%, 99% or even 100% in comparison with the untreated organism. A particularly advantageous embodiment is the inactivation of the nucleic acids or of the polypeptides encoded by them.

Various strategies for reducing the quantity (= expression), the activity or the function of proteins encoded by the nucleic acids according to the invention are encompassed in accordance with the invention. The skilled worker will recognize that a series of different methods are available for influencing the quantity of a protein, the activity or the function in the desired manner.

A reduction in the activity or the function is preferably achieved by a reduced expression of a gene encoding an endogenous protein.

A reduction in the protein quantity, the activity or function can be achieved using the following methods:
a) introduction of a double-stranded RNA nucleic acid sequence (dsRNA) or of an expression cassette, or more than one expression cassette, ensuring the expression of the latter;
b) introduction of an antisense nucleic acid sequence or of an expression cassette ensuring the expression of the latter. Encompassed are those methods in which the antisense nucleic acid sequence is directed against a gene (i.e. genomic DNA sequences) or a gene transcript (i.e. RNA sequences). Also encompassed are α-anomeric nucleic acid sequences.
c) introduction of an antisense nucleic acid sequence in combination with a ribozyme or of an expression cassette ensuring the expression of the former
d) introduction of sense nucleic acid sequences for inducing cosuppression or of an expression cassette ensuring the expression of the former
e) introduction of a nucleic acid sequence encoding dominant-negative protein or of an expression cassette ensuring the expression of the latter
f) introduction of DNA-, RNA- or protein-binding factors against genes, RNA's or proteins or of an expression cassette ensuring the expression of the latter
g) introduction of viral nucleic acid sequences and expression constructs which bring about the degradation of RNA, or of an expression cassette ensuring the expression of the former
h) introduction of constructs for inducing homologous recombination on endogenous genes, for example for generating knockout mutants.
i) introduction of mutations into endogenous genes for generating a loss of function (e.g. generation of stop codons, reading-frame shifts and the like)

Each of these methods may bring about a reduction in the expression, the activity or the function for the purposes of the invention. A combined use is also feasible. Further methods are known to the skilled worker and may encompass hindering or preventing processing of the protein, transport of the protein or its mRNA, inhibition of ribosomal attachment, inhibition of RNA splicing, induction of an enzyme which degrades RNA and/or inhibition of translational elongation or termination.

The term "protein quantity" refers to the amount of a polypeptide in an organism, a tissue, a cell or cell compartment. The term "reduction" of the protein quantity refers to the quantitative reduction of the amount of a protein in an organism, a tissue, a cell or a cell compartment - for example by one of the methods described herein below - in comparison with the wild type of the same genus and species to which this method has not been applied under otherwise identical conditions (such as, for example, culture conditions, age of the plants and the like). In this context, a reduction of at least 10%, advantageously of at least 20%, preferably at least 30%, especially preferably of at least 40%, 50% or 60%, very especially preferably of at least 70%, 80%, 90% or 95%, 99% or even 100% in comparison with the untreated organism is advantageous. An especially advantageous embodiment is the inactivation of the nucleic acids, or of the polypeptides encoded by them.

The term "activity" preferably refers to the activity of a polypeptide in an organism, a tissue, a cell or a cell compartment. The term "reduction" in the activity refers to the reduction in the overall activity of a protein in an organism, a tissue, a cell or a cell compartment - for example by one of the methods described herein below - in comparison with the wild type of the same genus and species, to which this method has not been applied, under otherwise identical conditions (such as, for example, culture conditions, age of the plants and the like). In this context, a reduction in activity of at least 10%, advantageously of at least 20%, preferably at least 30%, especially preferably of at least 40%, 50% or 60%, very especially preferably of at least 70%, 80%, 90% or 95%, 99% or even 100% in comparison with the untreated organism is advantageous. A particularly advantageous embodiment is the inactivation of the nucleic acids or of the polypeptides encoded by them.

The term "function" preferably refers to the enzymatic or regulatory function of a peptide in an organism, a tissue, a cell or a cell compartment. Suitable substrates are low-molecular-weight compounds and also the protein interaction partners of a protein. The term "reduction" of the function refers, for example, to the quantitative reduction in binding capacity or binding strength of a protein for at least one substrate in an organism, a tissue, a cell or a cell compartment - for example by one of the methods described herein below - in comparison with the wild type of the same genus and species to which this method has not been applied, under otherwise identical conditions (such as, for example, culture conditions, age of the plants and the like). Reduction is also understood as meaning the modification of the substrate specificity as can be expressed for example, by the kcat/Km value. In this context, a reduction of the function of at least 10%, advantageously of at least 20%, preferably at least 30%, especially preferably of at least 40%, 50% or 60%, very especially preferably of at least 70%, 80%, 90% or 95%, 99% or even 100% in comparison with the untreated organism is advantageous. A particularly advantageous embodiment is the inactivation of the function. Binding partners for the protein can be identified in the manner with which the skilled worker is familiar, for example by the yeast 2-hybrid system.

What follows is a brief description of the individual preferred methods:
A) Introduction of a double-stranded RNA nucleic acid sequence (dsRNA)
   The method of regulating genes by means of double-stranded RNA ("double-stranded RNA interference"; dsRNAi) has been described extensively for animal and plant organisms (for example Matzke MA et al. (2000) Plant Mol. Biol. 43: 401-415; Fire A. et al. (1998) Nature 391: 806-811; WO 99/32619; WO 99/53050; WO 00/68374; WO 00/44914; WO 00/44895; WO 00/49035; WO 00/63364). The techniques and methods described in the above references are expressly referred to. Efficient gene suppression can also be observed in the case of transient expression or following transient transformation, for example as the consequence of a biolistic transformation (Schweizer P et al. (2000) Plant J 2000 24: 895-903). dsRNAi methods are based on the phenomenon that the simultaneous introduction of complementary strand and counterstrand of a gene transcript brings about highly effective suppression of the expression of the gene in question. The resulting phenotype is very similar to that of an analogous knock-out mutant (Waterhouse PM et al. (1998) Proc. Natl. Acad. Sci. USA 95: 13959-64).
   Tuschl et al. [Gens Dev., 1999, 13 (24): 3191 - 3197] was able to show that the efficiency of the RNAi method is a function of the length of the duplex, the length of the 3'-end overhangs, and the sequence in these overhangs. Based on the work of Tuschl et al. and assuming that the underlining principles are conserved between different species the following guidelines can be given to the skilled worker:
   - to achieve good results the 5' and 3' untranslated regions of the used nucleic acid sequence and regions close to the start codon should be ingeneral avoided as this regions are richer in regulatory protein binding sites and interactions between RNAi sequences and such regulatory proteins might lead to undesired interactions;
   - in plants the 5' and 3' untranslated regions of the used nucleic acid sequence and regions close to the start codon preferably 50 to 100 nt upstream of the start codon give good results and therefore should not be avoided;
   - preferably a region of the used mRNA is selected, which is 50 to 100 nt (= nucleotides or bases) downstream of the AUG start codon;
   - only dsRNA (= double-stranded RNA) sequences from exons are useful for the method, as sequences from introns have no effect;
   - the G/C content in this region should be greater than 30% and less than 70% ideally around 50%;
   - a possible secondary structure of the target mRNA is less important for the effect of the RNAi method.

   The dsRNAi method has proved to be particularly effective and advantageous for reducing the expression of the nucleic acid sequenceaccording to SEQ ID NO:53 and/or homologs thereof. As described inter alia in WO 99/32619, dsRNAi approaches are clearly superior to traditional antisense approaches.
   The invention therefore furthermore relates to double-stranded RNA molecules (dsRNA molecules) which, when introduced into an organism, advantageously into a plant (or a cell, tissue, organ or seed derived therefrom), bring about the reduction in the expression of the nucleic acid sequences of SEQ ID NO:53 and/or homologs thereof. In a double-stranded RNA molecule for reducing the expression of an protein encoded by a nucleic acid sequence SEQ ID NO:53 and/or homologs thereof,
   i) one of the two RNA strands is essentially identical to at least part of a nucleic acid sequence, and
   ii) the respective other RNA strand is essentially identical to at least part of the complementary strand of a nucleic acid sequence.

   The term "essentially identical" refers to the fact that the dsRNA sequence may also include insertions, deletions and individual point mutations in comparison to the target sequence while still bringing about an effective reduction in expression. Preferably, the homology as defined above amounts to at least 75%, preferably at least 80%, very especially preferably at least 90%, most preferably 100%, between the "sense" strand of an inhibitory dsRNA and a part-segment of a nucleic acid sequence of the invention (or between the "antisense" strand and the complementary strand of a nucleic acid sequence, respectively). The part-segment amounts to at least 10 bases, preferably at least 25 bases, especially preferably at least 50 bases, very especially preferably at least 100 bases, most preferably at least 200 bases or at least 300 bases in length. As an alternative, an "essentially identical" dsRNA may also be defined as a nucleic acid sequence which is capable of hybridizing with part of a gene transcript (for example in 400 mM NaCl, 40 mM PIPES pH 6.4, 1 mM EDTA at 50°C or 70°C for 12 to 16 h).
   The dsRNA may consist of one or more strands of polymerized ribonucleotides. Modification of both the sugar-phosphate backbone and of the nucleosides may furthermore be present. For example, the phosphodiester bonds of the natural RNA can be modified in such a way that they encompass at least one nitrogen or sulfur hetero atom. Bases may undergo modification in such a way that the activity of, for example, adenosine deaminase is restricted. These and other modifications are described herein below in the methods for stabilizing antisense RNA.
   The dsRNA can be prepared enzymatically; it may also be synthesized chemically, either in full or in part.
   Short dsRNA up to 30 bp, which effectively mediate RNA interference, can be for example efficiently generated by partial digestion of long dsRNA templates using E. coli ribonuclease III (RNase III). (Yang, D., et al. (2002) Proc. Natl. Acad. Sci. USA 99, 9942.)
   The double-stranded structure can be formed starting from a single, self-complementary strand or starting from two complementary strands. In a single, self-complementary strand, "sense" and "antisense" sequence can be linked by a linking sequence ("linker") and form for example a hairpin structure. Preferably, the linking sequence may take the form of an intron, which is spliced out following dsRNA synthesis. The nucleic acid sequence encoding a dsRNA may contain further elements such as, for example, transcription termination signals or polyadenylation signals. If the two strands of the dsRNA are to be combined in a cell or an organism advantageously in a plant, this can be brought about in a variety of ways:
   a) transformation of the cell or of the organism, advantageously of a plant, with a vector encompassing the two expression cassettes,
   b) cotransformation of the cell or of the organism, advantageously of a plant, with two vectors, one of which encompasses the expression cassettes with the "sense" strand while the other encompasses the expression cassettes with the "antisense" strand.
   c) hybridization of two organisms, advantageously of plants, each of which has been transformed with one vector, one of which encompasses the expression cassettes with the "sense" strand while the other encompasses the expression cassettes with the "antisense" strand.
   d) supertransformation of the cell or of the organism, advantageously of a plant, with a vector encompassing the expression cassettes with the "sense" strand, after the cell or the organism had already been transformed with a vector encompassing the expression cassettes with the "antisense" strand;
   e) introduction of a construct comprising two promoters that lead to transcription of the desired sequence from both directions; and/or
   f) infecting of the cell or of the organism with, advantageously of a plant, with an engeniered virus, which is able to produce the disered dsRNA molecule.
   Formation of the RNA duplex can be initiated either outside the cell or within the cell.
   If the dsRNA is synthesized outside the target cell or organism it can be introduced into the organism or a cell of the organism by injection, microinjection, electroporation, high velocity particles, by laser beam or mediated by chemical compounds (DEAE-dextran, calciumphosphate, liposomes) or in case of animals it is also possible to feed bacteria such as E. coli strains engineered to express double-stranded RNAi to the animals.
   As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.
   As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.
   The expression cassettes encoding the "antisense" or the "sense" strand of the dsRNA or the self-complementary strand of the dsRNA are preferably inserted into a vector and stably inserted into the genome of a plant, using the methods described herein below (for example using selection markers), in order to ensure permanent expression of the dsRNA.
   The dsRNA can be introduced using an amount which makes possible at least one copy per cell. A larger amount (for example at least 5, 10, 100, 500 or 1 000 copies per cell) may bring about more efficient reduction.
   As has already been described, 100 % sequence identity between the dsRNA and a gene transcript of a nucleic acid sequence according to SEQ ID NO:53 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the nucleic acid according to SEQ ID NO:53 or homologs thereof of the one organism, may be used to suppress the corresponding expression in another organism.
   Due to the high degree of sequence homology between the sequences according to SEQ ID NO:53 from various organisms (e. g. plants), i. e. proteins may be conserved to a high degree within, for example other plants, it is optionally possible that the expression of a dsRNA derived from one of the disclosed sequences according to SEQ ID NO:53 or homologs thereof also has an advantageous effect in other plant species.
   The dsRNA can be synthesized either in vivo or in vitro. To this end, a DNA sequence encoding a dsRNA can be introduced into an expression cassette under the control of at least one genetic control element (such as, for example, promoter, enhancer, silencer, splice donor or splice acceptor or polyadenylation signal). Suitable advantageous constructs are described herein below. Polyadenylation is not required, nor do elements for initiating translation have to be present.
   A dsRNA can be synthesized chemically or enzymatically. Cellular RNA polymerases or bacteriophage RNA polymerases (such as, for example T3, T7 or SP6 RNA polymerase) can be used for this purpose. Suitable methods for the in-vitro expression of RNA are described (WO 97/32016; US 5,593,874; US 5,698,425, US 5,712,135, US 5,789,214, US 5,804,693). Prior to introduction into a cell, tissue or organism, a dsRNA which has been synthesized in vitro either chemically or enzymatically can be isolated to a higher or lesser degree from the reaction mixture, for example by extraction, precipitation, electrophoresis, chromatography or combinations of these methods. The dsRNA can be introduced directly into the cell or else be applied extracellularly (for example into the interstitial space).
   Stable transformation of the plant with an expression construct which brings about the expression of the dsRNA is preferred, however. Suitable methods are described herein below.
B) Introduction of an antisense nucleic acid sequence
   Methods for suppressing a specific protein by preventing the accumulation of its mRNA by means of "antisense" technology can be used widely and has been described extensively, including for plants (Sheehy et al. (1988) Proc. Natl. Acad. Sci. USA 85: 8805-8809; US 4,801,100; Mol JN et al. (1990) FEBS Lett 268(2): 427-430). The antisense nucleic acid molecule hybridizes with, or binds to, the cellular mRNA and/or the genomic DNA encoding the target protein to be suppressed. This process suppresses the transcription and/or translation of the target protein. Hybridization can be brought about in the conventional manner via the formation of a stable duplex or, in the case of genomic DNA, by the antisense nucleic acid molecule binding to the duplex of the genomic DNA by specific interaction in the large groove of the DNA helix.
   An antisense nucleic acid sequence which is suitable for reducing the activity of a protein can be deduced using the nucleic acid sequence encoding this protein, for example the nucleic acid sequence as shown in SEQ ID NO:53 (or homologs, analogs, paralogs, orthologs thereof), by applying the base-pair rules of Watson and Crick. The antisense nucleic acid sequence can be complementary to all of the transcribed mRNA of the protein; it may be limited to the coding region, or it may only consist of one oligonucleotide which is complementary to part of the coding or noncoding sequence of the mRNA. Thus, for example, the oligonucleotide can be complementary to the nucleic acid region which encompasses the translation start for the protein. Antisense nucleic acid sequences may have an advantageous length of, for example, 5, 10, 15, 20, 25, 30, 35, 40, 45 or 50 nucleotides but they may also be longer and encompass at least 100, 200, 500, 1000, 2000 or 5000 nucleotides. Prefered is a length of 15-35, more preferd a length of 15, 20, 2, 30 or 35 nucleotides. Antisense nucleic acid sequences can be expressed recombinantly or synthesized chemically or enzymatically using methods known to the skilled worker. In the case of chemical synthesis, natural or modified nucleotides may be used. Modified nucleotides may confer increased biochemical stability to the antisense nucleic acid sequence and lead to an increased physical stability of the duplex formed by antisense nucleic acid sequence and sense target sequence. Examples of substances which can be used are phosphorothioate derivatives and acridine-substituted nucleotides such as 5-fluorouracil, 5-bromouracil, 5-chlorouracil, 5-iodouracil, hypoxanthin, xanthin, 4-acetylcytosine, 5-(carboxyhydroxymethyl)uracil, 5-carboxymethylaminomethyl-2-thiouridine, 5-carboxymethylaminomethyluracil, dihydrouracil, β-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, β-D-mannosylqueosine, 5'-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, methyl uracil-5-oxyacetate, uracil-5-oxyacetic acid, 5-methyl-2-thiouracil, 3-(3-amino-3-N-2-carboxypropyl)uracil and 2,6-diaminopurine.
   In a further preferred embodiment, the expression of a protein encoded by SEQ ID NO:54 or homologs, analogs, paralogs, orthologs thereof can be inhibited by nucleotide sequences which are complementary to the regulatory region of a gene (for example a promoter and/or enhancer) and which form triplex structures with the DNA double helix in this region so that the transcription of the gene is reduced. Such methods have been described (Helene C (1991) Anticancer Drug Res. 6(6): 569-84; Helene C et al. (1992) Ann. NY Acad. Sci. 660: 27-36; Maher LJ (1992) Bioassays 14(12): 807-815).
   In a further embodiment, the antisense nucleic acid molecule can be an α-anomeric nucleic acid. Such α-anomeric nucleic acid molecules form specific double-stranded hybrids with complementary RNA in which - as opposed to the conventional β-nucleic acids - the two strands run in parallel with one another (Gautier C et al. (1987) Nucleic Acids Res. 15: 6625-6641). Furthermore, the antisense nucleic acid molecule can also comprise 2'-O-methylribonucleotides (Inoue et al. (1987) Nucleic Acids Res. 15: 6131-6148) or chimeric RNA-DNA analogs (Inoue et al. (1987) FEBS Lett 215: 327-330).
   The antisense nucleic acid molecules of the invention are typically administered to a cell or generated in situ such that they hybridize with or bind to cellular mRNA and/or genomic DNA encoding a polypeptide having the biological activity of protein of the invention thereby inhibit expression of the protein, e.g., by inhibiting transcription and/or translation and leading to the aforementioned compound X increasing activity.
   The antisense molecule of the present invention comprises also a nucleic acid molecule comprising a nucleotide sequences complementary to the regulatory region of an nucleotide sequence encoding the natural occurring polypeptide of the invention, e.g. the polypeptide sequences shown in the sequence listing, or identified according to the methods described herein, e.g., its promoter and/or enhancers, e.g. to form triple helical structures that prevent transcription of the gene in target cells. See generally, Helene, C. (1991) Anticancer Drug Des. 6(6):569-84; Helene, C. et al. (1992) Ann. N.Y. Acad. Sci. 660:27-36; and Maher, L.J. (1992) Bioassays 14(12):807-15.
C) Introduction of an antisense nucleic acid sequence combined with a ribozyme
   It is advantageous to combine the above-described antisense strategy with a ribozyme method. Catalytic RNA molecules or ribozymes can be adapted to any target RNA and cleave the phosphodiester backbone at specific positions, thus functionally deactivating the target RNA (Tanner NK (1999) FEMS Microbiol. Rev. 23(3): 257-275). The ribozyme per se is not modified thereby, but is capable of cleaving further target RNA molecules in an analogous manner, thus acquiring the properties of an enzyme. The incorporation of ribozyme sequences into "antisense" RNAs imparts this enzyme-like RNA-cleaving property to precisely these "antisense" RNAs and thus increases their efficiency when inactivating the target RNA. The preparation and the use of suitable ribozyme "antisense" RNA molecules is described, for example, by Haseloff et al. (1988) Nature 310: 585-591.
   In this manner, ribozymes [for example "Hammerhead" ribozymes; Haselhoff and Gerlach (1988) Nature 310: 585-591] can be used to catalytically cleave the mRNA of an enzyme to be suppressed and to prevent translation. The ribozyme technology can increase the efficacy of an antisense strategy. Methods for expressing ribozymes for reducing specific proteins are described in (EP 0 291 533, EP 0 321 201, EP 0 360 257). Ribozyme expression has also been described for plant cells (Steinecke P et al. (1992) EMBO J 11(4): 1525-1530; de Feyter R et al. (1996) Mol. Gen. Genet. 250(3): 329-338). Suitable target sequences and ribozymes can be identified for example as described by Steinecke P, Ribozymes, Methods in Cell Biology 50, Galbraith et al. eds, Academic Press, Inc. (1995), pp. 449-460 by calculating the secondary structures of ribozyme RNA and target RNA and by their interaction [Bayley CC et al. (1992) Plant Mol. Biol. 18(2): 353-361; Lloyd AM and Davis RW et al. (1994) Mol. Gen. Genet. 242(6): 653-657]. For example, derivatives of the tetrahymena L-19 IVS RNA which have complementary regions to the mRNA of the protein to be suppressed can be constructed (see also US 4,987,071 and US 5,116,742). As an alternative, such ribozymes can also be identified from a library of a variety of ribozymes via a selection process (Bartel D and Szostak JW (1993) Science 261: 1411-1418).
D) Introduction of a (sense) nucleic acid sequence for inducing cosuppression
   The expression of a nucleic acid sequence in sense orientation can lead to cosuppression of the corresponding homologous, endogenous genes. The expression of sense RNA with homology to an endogenous gene can reduce or indeed eliminate the expression of the endogenous gene, in a similar manner as has been described for the following antisense approaches: Jorgensen et al. [(1996) Plant Mol. Biol. 31(5): 957-973], Goring et al. [(1991) Proc. Natl. Acad. Sci. USA 88: 1770-1774], Smith et al. [(1990) Mol. Gen. Genet. 224: 447-481], Napoli et al. [(1990) Plant Cell 2: 279-289] or Van der Krol et al. [(1990) Plant Cell 2: 291-99]. In this context, the construct introduced may represent the homologous gene to be reduced either in full or only in part. The application of this technique to plants has been described for example by Napoli et al. [(1990) The Plant Cell 2: 279-289 and in US 5,010,323].
E) Introduction of nucleic acid sequences encoding a dominant-negative protein
   The function or activity of a protein can efficiently also be reduced by expressing a dominant-negative variant of said protein. The skilled worker is familiar with methods for reducing the function or activity of a protein by means of coexpression of its dominant-negative form [Lagna G and Hemmati-Brivanlou A (1998) Current Topics in Developmental Biology 36: 75-98; Perlmutter RM and Alberola-IIa J (1996-Current Opinion in Immunology 8(2): 285-90; Sheppard D (1994) American Journal of Respiratory Cell & Molecular Biology 11(1): 1-6; Herskowitz I (1987) Nature 329 (6136): 219-22].
   A dominant-negative variant can be realized for example by changing of an amino acid in the proteins encoded by SEQ ID NO:54 or homologs thereof. This change can be determined for example by computer aided comparison ("alignment"). These mutations for achieving a dominant-negative variant are preferably carried out at the level of the nucleic acid sequences. A corresponding mutation can be performed for example by PCR-mediated in-vitro mutagenesis using suitable oligonucleotide primers by means of which the desired mutation is introduced. To this end, methods are used with which the skilled worker is familiar. For example, the "LA PCR in vitro Mutagenesis Kit" (Takara Shuzo, Kyoto) can be used for this purpose. It is also possible and known to those skilled in the art that deleting or changing of functional domains, e. g. TF or other signaling components which can bind but not activate may achieve the reduction of protein activity.
F) Introduction of DNA- or protein-binding factors against genes, RNAs or proteins
   A reduction in the expression of a gene encoded by SEQ ID NO:54 or homologs thereof according to the invention can also be achieved with specific DNA-binding factors, for example factors of the zinc finger transcription factor type. These factors attach to the genomic sequence of the endogenous target gene, preferably in the regulatory regions, and bring about repression of the endogenous gene. The use of such a method makes possible the reduction in the expression of an endogenous gene without it being necessary to recombinantly manipulate the sequence of the latter. Such methods for the preparation of relevant factors are described in Dreier B et al. [(2001) J. Biol. Chem. 276(31): 29466-78 and (2000) J. Mol. Biol. 303(4): 489-502], Beerli RR et al. [(1998) Proc. Natl. Acad. Sci. USA 95(25): 14628-14633; (2000) Proc. Natl. Acad. Sci. USA 97(4): 1495-1500 and (2000) J. Biol. Chem. 275(42): 32617-32627)], Segal DJ and Barbas CF [3rd (2000) Curr. Opin. Chem. Biol. 4(1): 10-39], Kang JS and Kim JS [(2000) J. Biol. Chem. 275(12): 8742-8748], Kim JS et al. [(1997) Proc. Natl. Acad. Sci. USA 94(8): 3616-3620], Klug A [(1999) J. Mol. Biol. 293(2): 215-218], Tsai SY et al. [(1998) Adv. Drug Deliv. Rev. 30(1-3): 23-31], Mapp AK et al. [(2000) Proc. Natl. Acad. Sci. USA 97(8): 3930-3935], Sharrocks AD et al. [(1997) Int. J. Biochem. Cell Biol. 29(12): 1371-1387] and Zhang L et al. [(2000) J. Biol. Chem. 275(43): 33850-33860]. Examples for the application of this technology in plants have been described in WO 01/52620, Ordiz MI et al., (Proc. Natl. Acad. Sci. USA, Vol. 99, Issue 20, 13290 - 13295, 2002) or Guan et al., (Proc. Natl. Acad. Sci. USA, Vol. 99, Issue 20, 13296 - 13301, 2002)
   These factors can be selected using any portion of a gene. This segment is preferably located in the promoter region. For the purposes of gene suppression, however, it may also be located in the region of the coding exons or introns. The skilled worker can obtain the relevant segments from Genbank by database search or starting from a cDNA whose gene is not present in Genbank by screening a genomic library for corresponding genomic clones.
   It is also possible to first identify sequences in a target crop which are encoded by SEQ ID NO:53 then find the promoter and reduce expression by the use of the above mentioned factors.
   The skilled worker is familiar with the methods required for doing so.
   Furthermore, factors which are introduced into a cell may also be those which themselves inhibit the target protein. The protein-binding factors can, for example, be aptamers [Famulok M and Mayer G (1999) Curr. Top Microbiol. Immunol. 243: 123-36] or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants [Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272].
   Gene expression may also be suppressed by tailor-made low-molecular-weight synthetic compounds, for example of the polyamide type [Dervan PB and Bürli RW (1999) Current Opinion in Chemical Biology 3: 688-693; Gottesfeld JM et al. (2000) Gene Expr. 9(1-2): 77-91]. These oligomers consist of the units 3-(dimethylamino)propylamine, N-methyl-3-hydroxypyrrole, N-methylimidazole and N-methylpyrroles; they can be adapted to each portion of double-stranded DNA in such a way that they bind sequence-specifically to the large groove and block the expression of the gene sequences located in this position. Suitable methods have been described in Bremer RE et al. [(2001) Bioorg. Med. Chem. 9(8): 2093-103], Ansari AZ et al. [(2001) Chem. Biol. 8(6): 583-92], Gottesfeld JM et al. [(2001) J. Mol. Biol. 309(3): 615-29], Wurtz NR et al. [(2001) Org. Lett 3(8): 1201-3], Wang CC et al. [(2001) Bioorg. Med. Chem. 9(3): 653-7], Urbach AR and Dervan PB [(2001) Proc. Natl. Acad. Sci. USA 98(8): 4103-8] and Chiang SY et al. [(2000) J. Biol. Chem. 275(32): 24246-54].
G) Introduction of viral nucleic acid sequences and expression constructs which bring about the degradation of RNA
   Inactivation or downregulation can also be efficiently brought about by inducing specific RNA degradation by the organism, advantageously in the plant, with the aid of a viral expression system (Amplikon) [Angell, SM et al. (1999) Plant J. 20(3): 357-362]. Nucleic acid sequences with homology to the transcripts to be suppressed are introduced into the plant by these systems - also referred to as "VIGS" (viral induced gene silencing) with the aid of viral vectors. Then, transcription is switched off, presumably mediated by plant defense mechanisms against viruses. Suitable techniques and methods are described in Ratcliff F et al. [(2001) Plant J. 25(2): 237-45], Fagard M and Vaucheret H [(2000) Plant Mol. Biol. 43(2-3): 285-93], Anandalakshmi R et al. [(1998) Proc. Natl. Acad. Sci. USA 95(22): 13079-84] and Ruiz MT [(1998) Plant Cell 10(6): 937-46].
H) Introduction of constructs for inducing a homologous recombination on endogenous genes, for example for generating knock-out mutants
   To generate a homologously-recombinant organism with reduced activity, a nucleic acid construct is used which, for example, comprises at least part of an endogenous gene which is modified by a deletion, addition or substitution of at least one nucleotide in such a way that the functionality is reduced or completely eliminated. The modification may also affect the regulatory elements (for example the promoter) of the gene so that the coding sequence remains unmodified, but expression (transcription and/or translation) does not take place and is reduced.
   In the case of conventional homologous recombination, the modified region is flanked at its 5' and 3' end by further nucleic acid sequences which must be sufficiently long for allowing recombination. Their length is, as a rule, in a range of from one hundred bases up to several kilobases [Thomas KR and Capecchi MR (1987) Cell 51: 503; Strepp et al. (1998) Proc. Natl. Acad. Sci. USA 95(8): 4368-4373]. In the case of homologous recombination, the host organism - for example a plant - is transformed with the recombination construct using the methods described herein below, and clones which have successfully undergone recombination are selected using for example a resistance to antibiotics or herbicides. Using the cotransformation technique, the resistance to antibiotics or herbicides can subsequently advantageously be re-eliminated by performing crosses. An example for an efficient homologous recombination system in plants has been published in Nat. Biotechnol. 2002 Oct; 20(10):1030-4, Terada R et al.: Efficient gene targeting by homologous recombination in rice.
   Homologous recombination is a relatively rare event in higher eukaryotes, especially in plants. Random integrations into the host genome predominate. One possibility of removing the randomly integrated sequences and thus increasing the number of cell clones with a correct homologous recombination is the use of a sequence-specific recombination system as described in US 6,110,736, by means of which unspecifically integrated sequences can be deleted again, which simplifies the selection of events which have integrated successfully via homologous recombination. A multiplicity of sequence-specific recombination systems may be used, examples which may be mentioned being Cre/lox system of bacteriophage P1, the FLP/FRT system from yeast, the Gin recombinase of phage Mu, the Pin recombinase from E. coli and the R/RS system of the pSR1 plasmid. The bacteriophage P1 Cre/lox system and the yeast FLP/FRT system are preferred. The FLP/FRT and the cre/lox recombinase system have already been applied to plant systems [Odell et al. (1990) Mol. Gen. Genet. 223: 369-378].
I) Introduction of mutations into endogenous genes for bringing about a loss of function (for example generation of stop codons, reading-frame shifts and the like)
   Further suitable methods for reducing activity are the introduction of nonsense mutations into endogenous genes, for example by introducing RNA/DNA oligonucleotides into the plant [Zhu et al. (2000) Nat. Biotechnol. 18(5): 555-558], and the generation of knock-out mutants with the aid of, for example, T-DNA mutagenesis [Koncz et al. (1992) Plant Mol. Biol. 20(5): 963-976], ENU-(N-ethyl-N- nitrosourea) - mutagenesis or homologous recombination [ENU - (N-ethyl-N-nitrosourea) - mutagenesis or homologous recombination [Hohn B and Puchta (1999) H. Proc. Natl. Acad. Sci. USA 96: 8321-8323]. Point mutations may also be generated by means of DNA-RNA hybrids also known as "chimeraplasty" [Cole-Strauss et al. (1999) Nucl. Acids Res. 27(5): 1323-1330; Kmiec (1999) Gene Therapy American Scientist 87(3): 240-247]. The mutation sites may be specifically targeted or randomly selected.
   Nucleic acid sequences as described in item B) to I) are expressed in the cell or organism by transformation/transfection of the cell or organism or are introduced in the cell or organism by known methods, for example as disclosed in item A).
   Other suitable method for reducing activity is the introduction of a nucleic acid in the plant cell, which interacts with a gene encoded by one or more nucleic acid sequences consisting of SEQ ID NO:53 and/or homologs thereof. The interaction of the introduced nucleic acid, which can be active itself, with said gene leads by deletion, inversion or insertion finally to inactivation, i. e. by frameshift, or destruction of said gene.
   In particular, the invention provides a method of producing a transformed plant with a gene encoding nucleic acid, wherein inactivation or down-regulation of said gene(s) in the plant results in increased tolerance to environmental stress as compared to a wild type plant, comprising the inactivation or down-regulation by mutation of a nucleic acid sequence of SEQ ID NO:53 or homologs thereof.
   For such plant transformation, binary vectors such as pBinAR can be used (Höfgen and Willmitzer, 1990 Plant Science 66:221-230). Moreover suitable binary vectors are such as pBIN19, pBI101, pGPTV or pPZP (Hajukiewicz, P. et al., 1994, Plant Mol. Biol., 25: 989-994). An overview of binary vectors and their specific features is given in Hellens et al., 2000, Trends in plant science, 5: 446 - 451.
   Construction of the binary vectors can be performed by ligation of the cDNA in sense or antisense orientation into the T-DNA. 5-prime to the cDNA a plant promoter activates transcription of the cDNA. A polyadenylation sequence is located 3-prime to the cDNA. Tissue-specific expression can be achieved by using a tissue specific promoter as listed below. Also, any other promoter element can be used. For constitutive expression within the whole plant, the CaMV 35S promoter can be used. The expressed protein can be targeted to a cellular compartment using a signal peptide, for example for plastids, mitochondria or endoplasmic reticulum (Kermode, 1996 Crit. Rev. Plant Sci. 4(15):285-423). The signal peptide is cloned 5-prime in frame to the cDNA to archive subcellular localization of the fusion protein. Additionally, promoters that are responsive to abiotic stresses can be used with, such as the Arabidopsis promoter RD29A. One skilled in the art will recognize that the promoter used should be operatively linked to the nucleic acid such that the promoter causes transcription of the nucleic acid which results in the synthesis of a mRNA which encodes a polypeptide. Alternatively, the RNA can be an antisense RNA for use in affecting subsequent expression of the same or another gene or genes.
   Alternate methods of transfection include the direct transfer of DNA into developing flowers via electroporation or Agrobacterium mediated gene transfer. Agrobacterium mediated plant transformation can be performed using for example the GV3101 (pMP90) (Koncz and Schell, 1986 Mol. Gen. Genet. 204:383-396) or LBA4404 (Ooms et al., Plasmid, 1982, 7: 15-29; Hoekema et al., Nature, 1983, 303: 179-180) Agrobacterium tumefaciens strain. Transformation can be performed by standard transformation and regeneration techniques (Deblaere et al., 1994 Nucl. Acids. Res. 13:4777-4788; Gelvin and Schilperoort, Plant Molecular Biology Manual, 2nd Ed. - Dordrecht: Kluwer Academic Publ., 1995. - in Sect., Ringbuch Zentrale Signatur: BT11-P ISBN 0-7923-2731-4; Glick, B R and Thompson, J E, Methods in Plant Molecular Biology and Biotechnology, Boca Raton : CRC Press, 1993. - 360 S., ISBN 0-8493-5164-2). For example, rapeseed can be transformed via cotyledon or hypocotyl transformation (Moloney et al., 1989 Plant Cell Reports 8:238-242; De Block et al., 1989 Plant Physiol. 91:694-701). Use of antibiotics for Agrobacterium and plant selection depends on the binary vector and the Agrobacterium strain used for transformation. Rapeseed selection is normally performed using kanamycin as selectable plant marker. Agrobacterium mediated gene transfer to flax can be performed using, for example, a technique described by Mlynarova et al., 1994 Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,376,543 or U.S. Patent No. 5,169,770. Transformation of maize can be achieved by particle bombardment, polyethylene glycol mediated DNA uptake or via the silicon carbide fiber technique. (See, for example, Freeling and Walbot "The maize handbook" Springer Verlag: New York (1993) ISBN 3-540-97826-7). A specific example of maize transformation is found in U.S. Patent No. 5,990,387 and a specific example of wheat transformation can be found in PCT Application No. WO 93/07256.
   In particular, a useful method to ascertain the level of transcription or activity of the gene (an indicator of the amount of mRNA available for translation to the gene product) is to perform a Northern blot (for reference see, for example, Ausubel et al., 1988 Current Protocols in Molecular Biology, Wiley: New York). This information at least partially demonstrates the degree of transcription of the transformed gene. Total cellular RNA can be prepared from cells, tissues or organs by several methods, all well-known in the art, such as that described in Bormann, E.R. et al., 1992 Mol. Microbiol. 6:317-326. To assess the presence or relative quantity of protein translated from this mRNA, standard techniques, such as a Western blot, may be employed. These techniques are well known to one of ordinary skill in the art. (See for example Ausubel et al., 1988 Current Protocols in Molecular Biology, Wiley: New York). The use of Real Time PCR is also possible.
   The invention may further be combined with an isolated recombinant expression vector comprising a stress related protein encoding nucleic acid, wherein expression of the vector or stress related protein encoding nucleic acid, respectively in a host cell results in increased tolerance and/or resistance to environmental stress as compared to the wild type of the host cell. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses), which serve equivalent functions.
   A plant expression cassette comprising a nucleic acid construct, which when expressed allows inactivation or down-regulation of a gene encoded by a nucleic acid consisting of sequences according to SEQ ID NO:53 and/or homologs thereof and/or parts thereof by a method mentioned above leading to increased stress tolerance and/or resistance is also included in the scope of the present invention.
   The plant expression cassette preferably contains regulatory sequences capable of driving gene expression in plant cells and operably linked so that each sequence can fulfill its function, for example, termination of transcription by polyadenylation signals. Preferred polyadenylation signals are those originating from Agrobacterium tumefaciens T-DNA such as the gene 3 known as octopine synthase of the Ti-plasmid pTiACH5 (Gielen et al., 1984 EMBO J. 3:835) or functional equivalents thereof but also all other terminators functionally active in plants are suitable.
   Plant gene expression must be operably linked to an appropriate promoter conferring gene expression in a time, cell or tissue specific manner. Preferred promoters are such that drive constitutive expression (Benfey et al., 1989 EMBO J. 8:2195-2202) like those derived from plant viruses like the 35S CaMV (Franck et al., 1980 Cell 21:285-294), the 19S CaMV (see also U.S. Patent No. 5352605 and PCT Application No. WO 8402913) or plant promoters like those from Rubisco small subunit described in U.S. Patent No. 4,962,028.
   Additional advantageous regulatory sequences are, for example, included in the plant promoters such as CaMV/35S [Franck et al., Cell 21 (1980) 285 - 294], PRP1 [Ward et al., Plant. Mol. Biol. 22 (1993)], SSU, OCS, lib4, usp, STLS1, B33, LEB4, nos or in the ubiquitin, napin or phaseolin promoter. Additional useful plant promoters are the cytosolic FBPase promoter or ST-LSI promoter of the potato (Stockhaus et al., EMBO J. 8, 1989, 2445), the phosphorybosyl phyrophoshate amido transferase promoter of Glycine max (gene bank accession No. U87999) or the noden specific promoter described in EP-A-0 249 676. Additional particularly advantageous promoters are seed specific promoters which can be used for monocotyledons or dicotyledons. Described in US 5,608,152 (napin promoter from rapeseed), WO 98/45461 (phaseolin promoter from Arobidopsis), US 5,504,200 (phaseolin promoter from Phaseolus vulgaris), WO 91/13980 (Bce4 promoter from Brassica) and Baeumlein et al., Plant J., 2, 2, 1992: 233-239 (LEB4 promoter from leguminosa) are promoters useful in dicotyledons. The following promoters are useful for example in monocotyledons lpt-2-or lpt-1- promoter from barley (WO 95/15389 and WO 95/23230) or hordein promoter from barley. Other useful promoters described in WO 99/16890.
   It is possible in principle to inactivate or down-regulate all natural promoters with their regulatory sequences like those mentioned above in order to e. g. reduce the level of production of a targeted protein.
   The construct may also comprise further genes which are to be inserted into the organisms and which are for example involved in stress resistance, i.e. next to inactivating certain genes or incorporating inactivated genes at their place, it is possible to introduce favorable genes that are related to production of proteins which actively increase stress tolerance or resistance. It is therefore feasible and advantageous to insert and express in host organisms regulatory genes such as genes for inducers, repressors or enzymes which intervene by their enzymatic activity in the regulation of one or more or all genes of a biosynthetic pathway. These genes can be heterologous or homologous in origin. The inserted genes may have their own promoter or else be under the control of same promoter as sequences of SEQ ID NO:53 or their homologs.
   The gene construct advantageously comprises, for expression of the other genes present, additionally 3' and/or 5' terminal regulatory sequences to enhance expression, which are selected for optimal expression depending on the selected host organism and gene or genes.
   These regulatory sequences are intended to make specific expression of the genes and protein expression possible as mentioned above. This may mean, depending on the host organism, for example that the gene is expressed or overexpressed only after induction, or that it is immediately expressed and/or overexpressed.
   The regulatory sequences or factors may moreover preferably have a beneficial effect on expression of the introduced genes, and thus increase it. It is possible in this way for the regulatory elements to be enhanced advantageously at the transcription level by using strong transcription signals such as promoters and/or enhancers. However, in addition, it is also possible to enhance translation by, for example, improving the stability of the mRNA.
   Other preferred sequences for use in plant gene expression cassettes are targeting-sequences necessary to direct the gene product in its appropriate cell compartment (for review see Kermode, 1996 Crit. Rev. Plant Sci. 15(4):285-423 and references cited therein) such as the vacuole, the nucleus, all types of plastids like amyloplasts, chloroplasts, chromoplasts, the extracellular space, mitochondria, the endoplasmic reticulum, oil bodies, peroxisomes and other compartments of plant cells.

**Tab. 1: Examples of Tissue-specific and Stress inducible promoters in plants**

| Expression | Reference |
|---|---|
| Cor78- Cold, drought, salt, ABA, wounding-inducible | Ishitani, et al., Plant Cell 9:1935-1949 (1997). |
| | Yamaguchi-Shinozaki and Shinozaki, Plant Cell 6:251-264 (1994). |
| Rci2A - Cold, dehydration-inducible | Capel et al., Plant Physiol 115:569-576 (1997) |
| Rd22 - Drought, salt | Yamaguchi-Shinozaki and Shinozaki, Mol Gen Genet 238:17-25 (1993). |
| Cor15A - Cold, dehydration, ABA | Baker et al., Plant Mol. Biol. 24:701-713 (1994). |
| GH3- Auxin inducible | Liu et al., Plant Cell 6:645-657 (1994) |
| ARSK1-Root, salt inducible | Hwang and Goodman, Plant J 8:37-43 (1995). |
| PtxA - Root, salt inducible | GenBank accession X67427 |
| SbHRGP3 - Root specific | Ahn et al., Plant Cell 8:1477-1490 (1998). |
| KST1 - Guard cell specific | Plesch et al., Plant Journal 28(4):455-64(2001). |
| KAT1 - Guard cell specific | Plesch et al., Gene 249:83-89 (2000) |
| | Nakamura et al., Plant Physiol. 109:371-374 (1995) |
| salicylic acid inducible | PCT Application No. WO 95/19443 |
| tetracycline inducible | Gatz et al. Plant J. 2:397-404 (1992) |
| Ethanol inducible | PCT Application No. WO 93/21310 |
| pathogen inducible PRP1 | Ward et al., 1993 Plant. Mol. Biol. 22:361-366 |
| heat inducible hsp80 | U.S. Patent No. 5187267 |
| cold inducible alpha-amylase | PCT Application No. WO 96/12814 |
| Wound-inducible pinII | European Patent No. 375091 |
| RD29A - salt-inducible | Yamaguchi-Shinozalei et al. (1993) Mol. Gen. Genet. 236:331-100 |
| plastid-specific viral RNA-polymerase | PCT Application No. WO 95/16783 and. WO 97/06250 |

Selection marker systems, like the AHAS marker or other promotors, e.g. superpromotor (Ni et al,., Plant Journal 7, 1995: 661-676), Ubiquitin promotor (Callis et al., J. Biol. Chem., 1990, 265: 12486-12493; US 5,510,474; US 6,020,190; Kawalleck et al., Plant. Molecular Biology, 1993, 21: 673-684) or 10S promotor (GenBank Accession numbers M59930 and X16673) may be similar useful for the combination with the present invention and are known to a person skilled in the art.

In particular, the present invention describes the use of comparing particular attributes or traits, estimating the general appearance or comparing the altered metabolic activity by inactivation or down-regulation of genes to engineer stress-tolerant and/or resistant, i.e. drought-, salt- and/or cold-tolerant and/or resistant plants. This strategy has herein been demonstrated for Arabidopsis thaliana, but its application is not restricted to these plants. Accordingly, the invention provides a transformed plant containing one or more (stress related protein encoding) genes selected from sequences of SEQ ID NO:53 or homologs thereof, that are inactivated or down-regulated and stress tolerance and/or resistance, wherein the (environmental) stress is drought, increased salt or decreased or increased temperature but its application is not restricted to these adverse environments. Protection against other adverse conditions such as heat, air pollution, heavy metals and chemical toxicants, for example, may be obtained. In particullary preferred embodiments, the environmental stress is drought.

Growing the modified plants under stress conditions and then screening and analyzing the growth characteristics and/or metabolic activity assess the effect of the genetic modification in plants on stress tolerance and/or resistance. Such analysis techniques are well known to one skilled in the art. They include next to screening (Römpp Lexikon Biotechnologie, Stuttgart/New York: Georg Thieme Verlag 1992, "screening" p. 701) dry weight, wet weight, protein synthesis, carbohydrate synthesis, lipid synthesis, evapotranspiration rates, general plant and/or crop yield, flowering, reproduction, seed setting, root growth, respiration rates, photosynthesis rates, etc. (Applications of HPLC in Biochemistry in: Laboratory Techniques in Biochemistry and Molecular Biology, vol. 17; Rehm et al., 1993 Biotechnology, vol. 3, Chapter III: Product recovery and purification, page 469-714, VCH: Weinheim; Belter, P.A. et al., 1988 Bioseparations: downstream processing for biotechnology, John Wiley and Sons; Kennedy, J.F. and Cabral, J.M.S., 1992 Recovery processes for biological materials, John Wiley and Sons; Shaeiwitz, J.A. and Henry, J.D., 1988 Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications).

The methods of the invention may be used to detect environmental stress in plant cells or plants by screening the plant cells for particular attributes or traits, estimating the general appearance, analyzing the growth characteristics or screening for altered metabolic activity as compared to non-stress conditions, which allows for selection of resistant or tolerant plants or plant cells and also provides detection of stress in plants or plant cells before symptoms are visable and damage is high.

The methods of the invention also allow breeding of plant cells or plants towards increased tolerance and/or resistance to environmental stress by screening the plant cells under stress conditions for particular attributes or traits, estimating the general appearance, analyzing the growth characteristics or screening for altered metabolic activity as compared to non-stress conditions and selecting those with increased tolerance and/or resistance to environmental stress for further replication.

The engineering of one or more stress related genes of the invention may also result in stress related proteins having altered activities which indirectly impact the stress response and/or stress tolerance of plants. For example, the normal biochemical processes of metabolism result in the production of a variety of products (e.g., hydrogen peroxide and other reactive oxygen species) which may actively interfere with these same metabolic processes (for example, peroxynitrite is known to react with tyrosine side chains, thereby inactivating some enzymes having tyrosine in the active site (Groves, J.T., 1999 Curr. Opin. Chem. Biol. 3(2):226-235). By optimizing the inactivation or down-regulation of one or more stress related genes of the invention, it may be possible to improve the metabolic activity leading to higher stress tolerance and/or resistance of the cell.

Additionally, the sequences disclosed herein, or fragments thereof, can be targeted to generate knockout mutations in the genomes of various other plant cells (Girke, T., 1998 The Plant Journal 15:39-48). The resultant knockout cells can then be evaluated for their ability or capacity to tolerate various stress conditions, their response to various stress conditions, and the effect on the phenotype and/or genotype of the mutation. For other methods of gene inactivation see U.S. Patent No. 6004804 "Non-Chimeric Mutational Vectors" and Puttaraju et al., 1999 Spliceosome-mediated RNA trans-splicing as a tool for gene therapy Nature Biotechnology 17:246-252. Throughout this application, various publications are referenced. The disclosures of all of these publications and those references cited within those publications in their entireties are hereby incorporated by reference into this application in order to more fully describe the state of the art to which this invention pertains.

This invention is not limited to specific nucleic acids, specific polypeptides, specific cell types, specific host cells, specific conditions, or specific methods, etc., as such may, of course, vary, and the numerous modifications and variations therein will be apparent to those skilled in the art. It is also to be understood that the terminology used herein is for the purpose of describing specific embodiments only and is not intended to be limiting.

It should also be understood that the foregoing relates to preferred embodiments of the present invention and that numerous changes may be made therein without departing from the scope of the invention. The invention is further illustrated by the following examples, which are not to be construed in any way as imposing limitations upon the scope thereof. On the contrary, it is to be clearly understood that resort may be had to various other embodiments, modifications, and equivalents thereof, which, after reading the description herein, may suggest themselves to those skilled in the art without departing from the spirit of the present invention and/or the scope of the appended claims.

### Example

### Engineering stress-tolerant Arabidopsis plants by inactivation or down-regulation stress related genes.

### Transformation of Arabidopsis thaliana

### Vector preparation

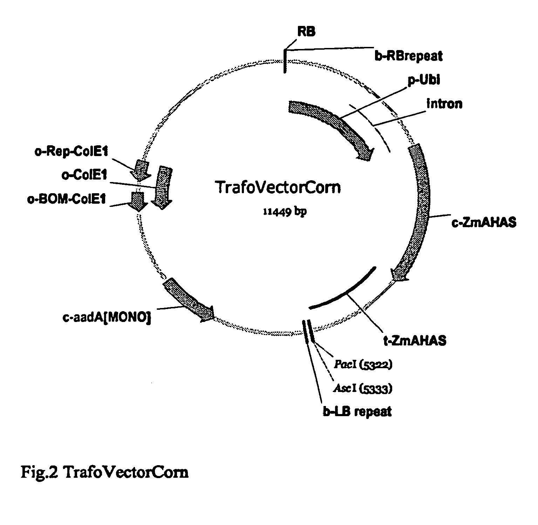

A binary vector was constructed based on the modified pPZP binary vector backbone (comprising the kanamycin-gene for bacterial selection; Hajdukiewicz, P. et al., 1994, Plant Mol. Biol., 25: 989-994) and the selection marker bar-gene (De Block et al., 1987, EMBO J. 6, 2513-2518) driven by the mas2'1' and mas271f promoters (Velten et al., 1984, EMBO J. 3, 2723-2730; Mengiste, Amedeo and Paszkowski, 1997, Plant J., 12, 945-948). The complete vector (Fig. 2) and plasmid are shown in the annex.
Examples of other usable binary vectors for insertional mutagenesis are pBIN19, pBI101, pBinAR or pGPTV. An overview over binary vectors and their specific features is given in Hellens et al., 2000, Trends in plant Science, 5:446-451 and in Guerineau F., Mullineaux P., 1993, Plant transformation and expression vectors in plant molecular biology, LABFAX Series, (Croy R.R.D., ed.) pp. 121-127 Bios Scientific Publishers, Oxford.

### Transformation of Agrobacteria

The plasmid was transformed into Agrobacterium tumefaciens (GV3101pMP90; Koncz and Schell, 1986 Mol. Gen. Genet. 204:383-396) using heat shock or electroporation protocols. Transformed colonies were grown on YEB medium and selected by respective antibiotics (Rif/Gent/Km) for 2 d at 28 °C. These agrobacteria cultures were used for the plant transformation.
Arabidopsis thaliana of the ecotype C24 were grown and transformed according to standard conditions (Bechtold, N., Ellis, J., Pelletier, G. 1993. In planta Agrobacterium mediated gene transfer by infiltration of Arabidopsis thaliana plants, C. R. Acad. Sci. Paris 316:1194-1199; Bent, A. F., Clough, J. C., 1998; Floral dip: a simplified method for Agrobacterium-mediated transformation of Arabidopsis thaliana, PLANT J. 16:735 - 743).
Transformed plants (F1) were selected by the use of their respective resistance marker. In case of BASTA®-resistance, plantlets were sprayed four times at an interval of 2 to 3 days with 0.02 % BASTA® and transformed plants were allowed to set seeds. 50-100 seedlings (F2) were subjected again to marker selection, in case of BASTA-resistance by spaying with 0.1 % BASTA® on 4 consecutive days during the plantlet phase. Plants segregating for a single resistance locus (approximately 3:1 resistant seedling to sensitive seedlings) were chosen for further analysis. From these lines three of the resistant seedlings (F2) were again allowed to set seeds and were tested for homozygosis through in-vitro germination of their seeds (F3) on agar medium containing the selection agent (BASTA®, 15 mg/L ammonium glufosinate, Pestanal, Riedel de Haen, Seelze, Germany). Those F2 lines which showed nearly 100% resistant offspring (F3) were considered homozygote and taken for functional analysis.

### Measurement of Stress Tolerance

Transformed A. thaliana plants were grown individually in pots containing a 4:1 (v/v) mixture of soil and quartz sand in a growth chamber (York Industriekälte GmbH, Mannheim, Germany). To induce germination, sown seeds were kept at 4°C, in the dark, for 3 days. Standard growth conditions were: photoperiod of 16 h light and 8 h dark, 20 °C, 60% relative humidity, and a photon flux density of 150 µE. Plants were watered daily until they were approximately 3 weeks old at which time drought was imposed by withholding water. Simultaneously, the relative humidity was reduced in 10% increments every second day to 20%. After approximately 12 days of withholding water, most plants showed visual symptoms of injury, such as wilting and leaf browning, whereas tolerant or resistant plants were identified as being visually turgid and healthy green in color. Plants were scored for symptoms of drought injury in comparison to wild type and neighboring plants for 3 - 5 days in succession.
In one experiment (table 2), at least 4, but usually 20 - 25 replicates of each confirmed tolerant line, i.e. those that had been scored as tolerant or resistant in precoursery experiments, were grown and treated as before. In this experiment the average and maximum number of days of drought survival after the wild-type control had visually died was determined. Additionally measurements of chlorophyll fluorescence (table 3) were made in stressed and non-stressed plants using a Mini-PAM (Heinz Walz GmbH, Effeltrich, Germany).
In the drought tolerance experiment (table 2) the control (non-transformed Arabidopsis thaliana) and most transformed lines in the test showed extreme visual symptoms of stress including necrosis and cell death. Several transformed plants retained viability as shown by their turgid appearance and maintenance of green color.
Chlorophyll fluorescence measurements of photosynthetic yield (in non-dark adapted plants) confirmed that 14 days of drought stress completely inhibited photosynthesis in the control plants. In most cases the transformed lines maintained photosynthetic function longer (table 3).

### Analysis of the selected stress resistant lines

Since the lines were preselected for single insertion loci and a homozygous situation of the resistance marker, the disruption (or mutation) of single genes through the integration of the T-DNA were expected to have lead to the stress-resistant phenotype. Lines which showed a consistent phenotype were chosen for molecular analysis.
Genomic DNA was purified from approximately 100 mg of leaf tissue from these lines using standard procedures (either spins columns from Qiagen, Hilden, Germany or the Nucleon Phytopure Kit from Amersham Biosciences, Freiburg, Germany). The amplification of the insertion side of the T-DNA was achieved using two different methods. Either by an adaptor PCR-method according to Spertini D, Béliveau C. and Bellemare G., 1999, Biotechniques, 27, 308 - 314 using T-DNA specific primers LB1 (5' - TGA CGC CAT TTC GCC TTT TCA - 3'; SEQ ID NO: 83) for the first and LB2 (5' - CAG AAA TGG ATA AAT AGC CTT GCT TCC - 3'; SEQ ID NO: 84) or RB4-2 (5' - AGC TGG CGT AAT AGC GAA GAG - 3'; SEQ ID NO: 85) for the second round of PCR. Alternatively TAIL-PCR (Liu Y-G, Mitsukawa N, Oosumi T and Whittier RF, 1995, Plant J. 8, 457-463 was preformed. In this case in the first round PCR LB1 (5' - TGA CGC CAT TTC GCC TTT TCA - 3' SEQ ID NO: 83) or RB1-2 (5'- CAA CTT AAT CGC CTT GCA GCA CA-3'; SEQ ID NO: 86), for the second round LB2 (5' - CAG AAA TGG ATA AAT AGC CTT GCT TCC - 3' SEQ ID NO: 84) or RB4-2 (5' - AGC TGG CGT AAT AGC GAA GAG - 3' SEQ ID NO: 87) and in the last round LB3 (5' - CCA ATA CAT TAC ACT AGC ATC TG - 3'; SEQ ID NO: 88) or RB5 (5' - AAT GCT AGA GCA GCT TGA - 3'; SEQ ID NO: 89) were used as T-DNA specific primers for left or right T-DNA borders respectively.
Appropriate PCR-products were identified on agarose gels and purified using columns and standard procedures (Qiagen, Hilden, Germany). PCR-products were sequenced with additional T-DNA-specific primers located towards the borders relative to the primers used for amplification. For PCR products containing left border sequences primer LBseq (5' - CAA TAC ATT ACA CTA GCA TCT G - 3'; SEQ ID NO: 90) and for sequences containing right border sequences primer RBseq (5' - AGA GGC CCG CAC CGA TCG - 3'; SEQ ID NO: 91) was used for sequencing reactions. The resulting sequences were taken for comparison with the available Arabidopsis genome sequence from Genbank using the blast algorithm (Altschul et al., 1990. J Mol Biol, 215:403-410).
Details on PCR products used to identify the genomic locus are given in table 4. Indicated are the identified annotated open reading frame in the Arabidopsis genome, the estimated size of the obtained PCR product (in base pairs), the T-DNA border (LB: left border, RB: right border) for which the amplification was achieved, the method which resulted in the indicated PCR product (explanation see text above), the respective restriction enzymes in case of adaptor PCR, and the degenerated primer in the case of TAIL PCR. Routinely degenerated primers ADP3 (5'-WGTGNAGWANCANAGA-3'; SEQ ID NO: 92), ADP6 (5'-AGWGNAGWANCANAGA-3'; SEQ ID NO: 93) and ADP8 (5'-NTGCGASWGANWAGAA-3'; SEQ ID NO: 94) were used next to the other known primers given in table 4.
The identification of the insertion locus in each case was confirmed by a control PCR, using a T-DNA-specific primer and a primer deduced from the identified genomic locus, near to the insertion side. The amplification of a PCR-product of the expected size from the insertion line using these two primers proved the disruption of the identified locus by the T-DNA integration.

### Tables 2 - 5:

**Table 2: Duration of survival of transformed Arabidopsis thaliana after imposition of drought stress on 3-week-old plants. Drought tolerance was measured visually at daily intervals. Survival duration is the average of all plants that survived longer than the wild type control. The Maximum duration is the longest period that any single transformed plant survived longer than the wild type control.**

| SEQ ID No. | Gene | Plants tested | Average days of survival after WT | Maximum days of survival |
|---|---|---|---|---|
| | CONTROL | | 0 | 0 |
| 53 | At3g55990 | 40 | 7.03 | 20 |

**Table 3: Photosynthetic yield as determined by chlorophyll florescence of transformed Arabidopsis thaliana after imposition of drought stress on 3-week-old plants. Measurements were taken at intervals after withholding water and reported as photosynthetic yield (Y). Values are the average of 5 randomly selected plants. These are compared to the MC24 strain for reference.**

| SEQ ID No. | Gene | Photosynt hetic yield 6 days after final watering | MC24 Referen ce | Photosynth etic yield 10 days after final watering | MC24 Reference | Photosynt hetic yield 14 days after final watering | MC24 Reference |
|---|---|---|---|---|---|---|---|
| 53 | At3g55990 | 774 | 794 | 776 | 413 | 747 | 54 |

**Table 4: Details on PCR products used to identify the down-regulated genomic locus.**

| SEQ ID No. | Gene | Length PCR Product | Sequence length | Border | Method | Restriction enzyme or deg. Primer |
|---|---|---|---|---|---|---|
| 53 | At3g55990 | 1300 | 579 | LB | Adaptor | Psp1406 I/Bsp119 I |

Construction of antisense constructs for repression of the genes of the invention Fragments of SEQ ID NO: 95 or other nucleic acids of the invention are amplified by PCR using pairs of gene specific primers. To enable cloning of the PCR product, restriction sites may be added to the primers used for the amplification. Alternatively recombination sites may be added to the primers to enable a recombination reaction. The PCR fragment is either cloned or recombined into a binary vector, preferently under control of a strong constitutive, tissue or developmental specific promoters in a way, that the orientation to the promoter is opposite of the direction the gens has in its original genomic position.

The amplification of the fragment of the SEQ ID NO: 95 was performed using the oligonucleotides that have been deduced from the gene sequence:
Seq1antifw: 5' - atagaattcatgcttcgactgatcgacga - 3' (SEQ ID NO: 869)
Seq1antirev: 5' - atagtcgaccaccgggcacattgagcaat - 3' (SEQ ID NO: 870)
The Oligonucleotides have been solved in water to give a concentration of 20 µM. The standard protocol of RNA extraction (Qiagen) and cDNA production (Invitrogen) is used for the isolation procedure of RNA from different tissues of Brassica napus, Glycine max, Zea mays or Oryza sativa and sysnthesis of ther respective cDNA. The PCR reaction contained 5 µl ThermalAce buffer (Invitrogen), 0,4 µl dNTPs (25 mM each) (Invitrogen), 0,5 µl Primer Seq1antifw, 0,5 µl Primer Seq1antirev, 0,5 µl ThermalAce (Invitrogen), 0,5 µl cDNA and 42,6 µl water. The PCR was performed on MJ-Cycler Tetrad (BioZym) with the following programm: 4 min 94 °C, followed by 30 cycles of 1 min 94 °C, 1 min 57 °C, 1 min 72 °C followed by 10 min 72 °C and cooling to 25 °C.

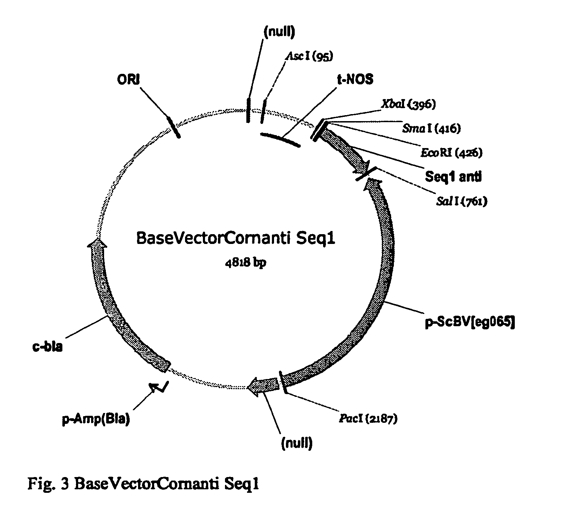

The PCR product has been purified using a Kit from Qiagen. The DNA was subsequently digested with EcoRI / SalI (NEB) at 37 °C over night. The fragment was then cloned into the vector BaseVectorCorn (see figure 1) which has been digested with EcoRI / SalI. The resulting construct was named BaseVectorCorn_antiSeq1. From this vector the cassette comprising the ScBV Promoter, the antisense fragment of Seq1 and the NOS terminator has been cut out using the restriction enzymes Ascl /PacI and has been ligated in the vector TrafoVectorCorn (see figure 2) yielding the vector TrafoVectorCorn_antiSeq1 (Fig. 3).

### Construction of RNAi constructs for repression of the genes of the invention

Two fragments of SEQ ID NO: 95 are amplified by PCR . To enable cloning of the PCR product, restriction sites may be added to the primers used for the amplification. Alternatively recombination sites may be added to the primers to enable a recombination reaction. The PCR fragment is either cloned or recombined into a binary vector, preferently under control of a strong constitutive, tissue or developmental specific promoters in a way, that the fragment is introduced twice in the vector as an inverted repeat, the repeats separated by a DNA spacer.

The amplification of the fragments of the SEQ ID NO: 95 was performed using the oligonucleotides that have been deduced from the gene sequence:
Seq1rifw1: 5' - atagtcgacatgcttcgactgatcgacga - 3' (SEQ ID NO: 871)
Seq1rirev1: 5' - atagaattccaccgggcacattgagcaat - 3' (SEQ ID NO: 872)
Seq1rifw2: 5' - atacccgggatgcttcgactgatcgacga - 3' (SEQ ID NO: 873)
Seq 1 rirev2: 5' - atatctagacaccgggcacattgagcaat - 3' (SEQ I D NO: 874)
The Oligonucleotides have been solved in water to give a concentration of 20 µM. The PCR reactions contained 5 µl ThermalAce buffer (Invitrogen), 0,4 µl dNTPs (25 mM each) (Invitrogen), 0,5 µl Primer Seq1rifw1, 0,5 µl Primer Seq1rirev1, 0,5 µl ThermalAce (Invitrogen), 0,5 µl cDNA and 42,6 µl water and 5 µl ThermalAce buffer (Invitrogen), 0,4 µl dNTPs (25 mM each) (Invitrogen), 0,5 µl Primer Seq1rifw2, 0,5 µl Primer Seq1rirev2, 0,5 µl ThermalAce (Invitrogen), 0,5 µl cDNA and 42,6 µl water respectively. The PCRs were performed on MJ-Cycler Tetrad (BioZym) with the following programm: 4 min 94 °C, followed by 30 cycles of 1 min 94 °C, 1 min 57 °C, 1 min 72 °C followed by 10 min 72 °C and cooling to 25 °C.

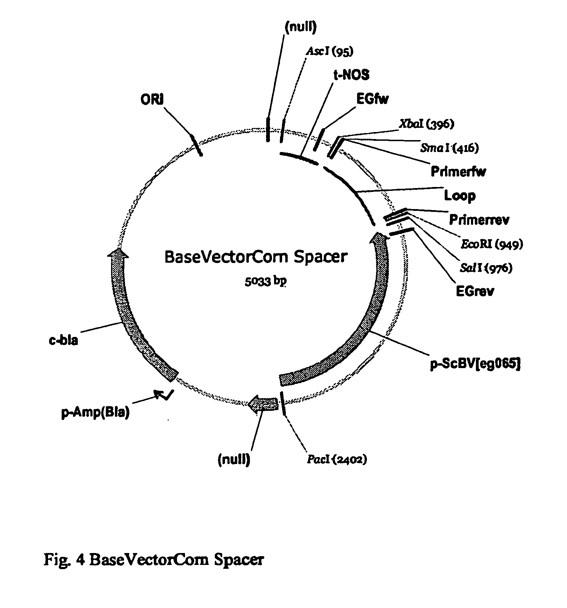

The PCR products have been purified using a Kit from Qiagen. The DNA was subsequently digested with SalI / EcoRI at 37 °C over night and Smal for 6h at 28 °C followed by digestion with Xbal overnight at 37 °C respectively. The SmaI / Xbal digested fragment was then cloned into the vector BaseVectorCorn_Spacer (see figure 4) wich has been digested with SmaI/XbaI. The resulting construct was digested with EcoRI / SalI and the EcoRI / SalI PCR fragment was ligated into this vector. Subsequently, the expression cassette comprising the ScBV promoter, inverted repeat of Seq1 separated by the spacer sequence and NOS terminator has been cut out using the restriction enzymes Ascl/Pacl and has been ligated in the vector TrafoVectorCorn (see figure 2) yielding the vector.

### Construction of Cosuppression constructs for repression of the genes of the invention

A fragment of SEQ ID NO: 95 is amplified by PCR . To enable cloning of the PCR product, restriction sites may be added to the primers used for the amplification. Alternatively recombinationsites may be added to the primers to enable a recombination reaction. The PCR fragment is either cloned or recombined into a binary vector, preferently under control of a strong constitutive, tissue or developmental specific promoters in a way, that the orientation to the promoter is identical to the direction the gen has in its original genomic position.

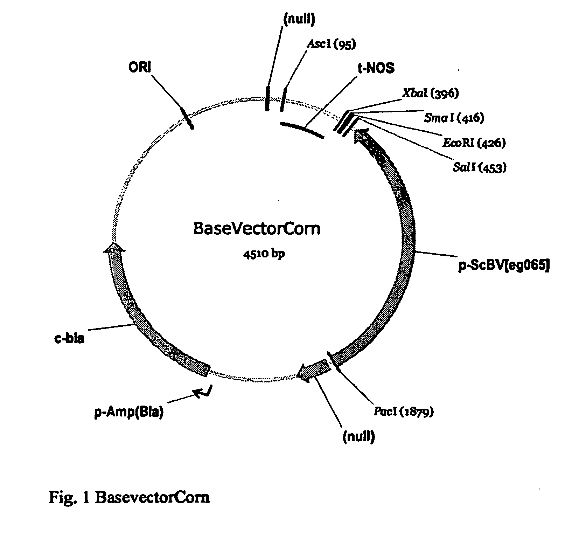

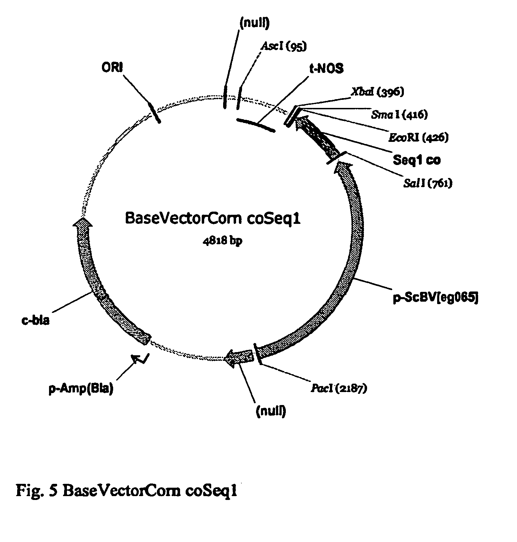

The amplification of the fragment of the SEQ ID NO: 95 was performed using the oligonucleotides that have been deduced from the gene sequence:
Seq1cofw: 5' - atagtcgacatgcttcgactgatcgacga - 3' (SEQ ID NO:875)
Seq1corev: 5' - atagaattcccaccgggcacattgagcaat - 3' (SEQ ID NO:876)
The Oligonucleotides have been solved in water to give a concentration of 20 µM. The PCR reactions contained 5 µl ThermalAce buffer (Invitrogen), 0,4 µl dNTPs (25 mM each) (Invitrogen), 0,5 µl Primer Seq1cofw, 0,5 µl Primer Seq1corev, 0,5 µl ThermalAce (Invitrogen), 0,5 µl cDNA and 42,6 µl water. The PCR was performed on MJ-Cycler Tetrad (BioZym) with the following programm: 4 min 94 °C, followed by 30 cycles of 1 min 94 °C, 1 min 57 °C, 1 min 72 °C followed by 10 min 72 °C and cooling to 25 °C.
The PCR product has been purified using a Kit from Qiagen. The DNA was subsequently digested with EcoRI / SalI (NEB) at 37 °C over night. The fragment was then cloned into the vector BaseVectorCorn (see figure 1) which has been digested with EcoRI / SalI. The resulting construct was named BaseVectorCorn_coSeq1.
From this vector the cassette comprising the ScBV Promoter, the cosuppression fragment of Seq1 and the NOS terminator has been cut out using the restriction enzymes AscI / PacI and has been ligated in the vector TrafoVectorCorn (see figure 2) yielding the vector TrafoVectorCorn_coSeq1 (Fig. 5).

Engineering stress-tolerant corn plants by reducing the expression of stress related genes from Zea mays in corn.
Transformation of corn (Zea mays L.) is performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors are constructed as described. Various selection marker genes can be used including the corn gene encoding a mutated acetohydroxy acid synthase (AHAS) enzyme (US patent 6025541). Similarly, various promoters can be used to provide constitutive, developmental, tissue or environmental regulation of gene suppression construct, eg the cosuppresion or the antisense or the RNAi construct.. In this example, the 34S promoter (GenBank Accession numbers M59930 and X16673) is used to provide constitutive expression of the cosuppression or antisense or RNAi constructs.
Excised embryos are grown on callus induction medium, then corn regeneration medium, containing imidazolinone as a selection agent. The Petri plates were incubated in the light at 25 °C for 2-3 weeks, or until shoots develop. The green shoots from each embryo are transferred to corn rooting medium and incubated at 25 °C for 2-3 weeks, until roots develop. The rooted shoots are transplanted to soil in the greenhouse. T1 seeds are produced from plants that exhibit tolerance to the imidazolinone herbicides and are PCR positive for the transgenes.

The T1 transgenic plants are then evaluated for their improved stress tolerance according to the standard methods eg the withdraw of water and the performance othe the plant in comparison the wildtype or mock transformed plants.. The seedlings receive no water for a period up to 3 weeks at which time the plant and soil are desiccated. At various times after withholding water, a normal watering schedule is resumed. At one week after resuming watering, the lengths of leaf blades, and the fresh and dry weights of the shoots is determined. At an equivalent degree of drought stress, tolerant plants are able to resume normal growth whereas susceptible plants have died or suffer significant injury resulting in shorter leaves and less dry matter. The T1 generation of single locus insertions of the T-DNA will segregate for the transgene in a 1:2:1 ratio. Those progeny containing one or two copies of the transgene (3/4 of the progeny) are tolerant of the imidazolinone herbicide, and exhibit greater tolerance of drought stress than those progeny lacking the transgenes. Tolerant plants have higher seed yields, maintained their stomatal aperture and showed only slight changes in osmotic potential and proline levels, whereas the susceptible non-transgenic control plants closed their stomata and exhibited increased osmotic potential and proline levels. Homozygous T2 plants exhibited similar phenotypes. Hybrid plants (F1 progeny) of homozygous transgenic plants and non-transgenic plants also exhibited increased environmental stress tolerance.

Engineering stress-tolerant soybean plants by reducing the expression of stress related genes from Glycine max in Glycine max.
In order to reduce the expression of the stress related soybean gene ins Soybean, antisense, cosuppression or RNAi constructs are constructed in a way similar to what has been described in the previous examples. Soybean is transformed according to the following modification of the method described in the Texas A&M patent US 5,164,310. Several commercial soybean varieties are amenable to transformation by this method. The cultivar Jack (available from the Illinois Seed Foundation) is a commonly used for transformation. Seeds are sterilized by immersion in 70% (v/v) ethanol for 6 min and in 25 % commercial bleach (NaOCl) supplemented with 0.1 % (v/v) Tween for 20 min, followed by rinsing 4 times with sterile double distilled water. Seven-day old seedlings are propagated by removing the radicle, hypocotyl and one cotyledon from each seedling. Then, the epicotyl with one cotyledon is transferred to fresh germination media in petri dishes and incubated at 25 °C under a 16-hr photoperiod (approx. 100 µE-m-2s-1) for three weeks. Axillary nodes (approx. 4 mm in length) are cut from 3 - 4 week-old plants. Axillary nodes are excised and incubated in Agrobacterium LBA4404 culture.
Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey) and can be used to carry the antisense, cosuppression or RNAi constructs. Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two components - a selection marker gene with a suitable promoter and a plant promoter regulating the transcription of the cDNA or the antisense fragment or the RNAi construct. Various selection marker genes can be used including the Arabidopsis gene encoding a mutated acetohydroxy acid synthase (AHAS) enzyme (US patents 57673666 and 6225105). Similarly, various promoters can be used to regulate the cosuppression, RNAi oder antisense construct to provide constitutive, developmental, tissue or environmental regulation. In this example, the 34S promoter (GenBank Accession numbers M59930 and X16673) is used to provide constitutive expression the gene repression constructs.
After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.
The primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.
The transgenic plants are then evaluated for their improved stress tolerance according to the method standard methods, or like described in the following. The seedlings receive no water for a period up to 3 weeks at which time the plant and soil are desiccated. At various times after withholding water, a normal watering schedule is resumed. At one week after resuming watering, the lengths of leaf blades, and the fresh and dry weights of the shoots is determined. At an equivalent degree of drought stress, tolerant plants are able to resume normal growth whereas susceptible plants have died or suffer significant injury resulting in shorter leaves and less dry matter.

Stress-tolerant soybean plants reduced in the expression of the stress related genes from soybean have higher seed yields, maintain their stomatal aperture and show only slight changes in osmotic potential and proline levels, whereas the susceptible non-transgenic control plants close their stomata and exhibit increased osmotic potential and proline levels.
Transgenic plants that display tolerance to salinity or cold have higher seed yields, photosynthesis and dry matter production than susceptible plants.

Engineering stress-tolerant Rapeseed/Canola plants by by reducing the expression of stress related genes from Brassica napus, in Brassica napus. Cotyledonary petioles and hypocotyls of 5-6 day-old young seedlings are used as explants for tissue culture and transformed according to Babic et al. (1998, Plant Cell Rep 17: 183-188). The commercial cultivar Westar (Agriculture Canada) is the standard variety used for transformation, but other varieties can be used.
Agrobacterium tumefaciens LBA4404 containing a binary vector carrying an antisense or an cosuppression or an RNAi constructs as described in the examples above is used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two components - a selection marker gene with a suitable promoter and a plant promoter regulating the transcription of the cDNA or a fragment of it for cosupression or the antisense fragment or the RNAi construct.. Various selection marker genes can be used including the Arabidopsis gene encoding a mutated acetohydroxy acid synthase (AHAS) enzyme (US patents 57673666 and 6225105). Similarly, various promoters can be used to regulate the constructs for reducing gene expression to provide constitutive, developmental, tissue or environmental regulation of transcription. In this example, the 34S promoter (GenBank Accession numbers M59930 and X16673) is used to provide constitutive expression.of the gene repression constructs.
Canola seeds are surface-sterilized in 70% ethanol for 2 min., and then in 30% Clorox with a drop of Tween-20 for 10 min, followed by three rinses with sterilized distilled water. Seeds are then germinated in vitro 5 days on half strength MS medium without hormones, 1% sucrose, 0.7% Phytagar at 23oC, 16 hr. light. The cotyledon petiole explants with the cotyledon attached are excised from the in vitro seedlings, and inoculated with Agrobacterium by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MSO) for root induction.
Samples of the primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.
The transgenic plants are then evaluated for their improved stress tolerance according to the method standard methods, or like described in the following. The seedlings receive no water for a period up to 3 weeks at which time the plant and soil are desiccated. At various times after withholding water, a normal watering schedule is resumed. At one week after resuming watering, the lengths of leaf blades, and the fresh and dry weights of the shoots is determined. At an equivalent degree of drought stress, tolerant plants are able to resume normal growth whereas susceptible plants have died or suffer significant injury resulting in shorter leaves and less dry matter. It is found that transgenic Rapeseed/Canola reduced in the expression of stress related genes from Brassica napus, are more tolerant to environmental stress than non-transgenic control plants. Tolerant plants have higher seed yields, maintain their stomatal aperture and show only slight changes in osmotic potential and proline levels, whereas the susceptible non-transgenic control plants close their stomata and exhibited increased osmotic potential and proline levels. Plants that have tolerance to salinity or cold have higher seed yields, photosynthesis and dry matter production than susceptible plants.

### Identification of Identical and Heterologous Genes

Gene sequences can be used to identify identical or heterologous genes from cDNA or genomic libraries. Identical genes (e. g. full-length cDNA clones) can be isolated via nucleic acid hybridization using for example cDNA libraries. Depending on the abundance of the gene of interest, 100,000 up to 1,000,000 recombinant bacteriophages are plated and transferred to nylon membranes. After denaturation with alkali, DNA is immobilized on the membrane by e. g. UV cross linking. Hybridization is carried out at high stringency conditions. In aqueous solution, hybridization and washing is performed at an ionic strength of 1 M NaCl and a temperature of 68°C. Hybridization probes are generated by e.g. radioactive (³²P) nick transcription labeling (High Prime, Roche, Mannheim, Germany). Signals are detected by autoradiography.
Partially identical or heterologous genes that are similar but not identical can be identified in a manner analogous to the above-described procedure using low stringency hybridization and washing conditions. For aqueous hybridization, the ionic strength is normally kept at 1 M NaCl while the temperature is progressively loared from 68 to 42°C.
Isolation of gene sequences with homology (or sequence identity/similarity) in only a distinct domain of for example 10-20 amino acids can be carried out using synthetic radio labeled oligonucleotide probes. Radiolabeled oligonucleotides are prepared by phosphorylation of the 5-prime end of two complementary oligonucleotides with T4 polynucleotide kinase. The complementary oligonucleotides are annealed and ligated to form concatemers. The double stranded concatemers are then radiolabeled by, for example, nick transcription. Hybridization is normally performed at low stringency conditions using high oligonucleotide concentrations.
Oligonucleotide hybridization solution:
6 x SSC
0.01 M sodium phosphate
1 mM EDTA (pH 8)
0.5 % SDS
100 µg/ml denatured salmon sperm DNA
0.1 % nonfat dried milk
During hybridization, temperature is lowered stepwise to 5-10°C below the estimated oligonucleotide Tₘ or down to room temperature followed by washing steps and autoradiography. Washing is performed with low stringency such as 3 washing steps using 4 x SSC. Further details are described by Sambrook, J. et al., 1989, "Molecular Cloning: A Laboratory Manual," Cold Spring Harbor Laboratory Press or Ausubel, F.M. et al., 1994, "Current Protocols in Molecular Biology," John Wiley & Sons.

### Identification of Identical or homologous Genes by Screening Expression Libraries with Antibodies

c-DNA clones can be used to produce recombinant polypeptide for example in E. coli (e.g. Qiagen QIAexpress pQE system). Recombinant polypeptides are then normally affinity purified via Ni-NTA affinity chromatography (Qiagen). Recombinant polypeptides are then used to produce specific antibodies for example by using standard techniques for rabbit immunization. Antibodies are affinity purified using a Ni-NTA column saturated with the recombinant antigen as described by Gu et al., 1994, BioTechniques 17:257-262. The antibody can then be used to screen expression cDNA libraries to identify identical or heterologous genes via an immunological screening (Sambrook, J. et al., 1989, "Molecular Cloning: A Laboratory Manual," Cold Spring Harbor Laboratory Press or Ausubel, F.M. et al., 1994, "Current Protocols in Molecular Biology", John Wiley & Sons).

### Reducing the expression of the genes of the invention by artificial transcription factors

The genes of the invention and their homologous ORFs in other species may also be down regulated by introducing a synthetic specific repressor. For this purpose, a gene for a chimeric zinc finger protein, which binds to a specific region in the regulatory or coding region of the genes of interests or its homologs in other spezies is constructed. The artificial zinc finger protein comprises a specific DNA-binding domain consting for example of zinc finger and optional an repression like the EAR domain (Hiratsu et al., 2003. Plant J. 34(5), 733-739 Dominant repression of target genes by chimeric repressors that include the EAR motif, a repression domain, in Arabidopsis.)
Expression of this chimeric repressor for example in plants then results in specific repression of the target gene or of its homologs in other plant species lead to increased metabolite production The experimental details expecially about the desing and construction of specific zinc finger domains may be carried out as described, or WO 01/52620 or Ordiz MI, (Proc. Natl. Acad. Sci. USA, 2002, Vol. 99, Issue 20, 13290) or Guan, (Proc. Natl. Acad. Sci. USA, 2002, Vol. 99, Issue 20, 13296).

Knock out of the genes of the invention by homologous recombination Identifying mutations in the genes of the invention in random mutagenized populations
a) In chemically or radiation mutated population
   Production of chemically or radiation mutated populations is a common technique and known to the skilled worker. Methods are described by Koorneef et al. 1982 and the citations therein and by Lightner and Caspar in "Methods in Molecular Biology" Vol 82. These techniques usually induce point mutations that can be identified in any known gene using methods such as TILLING (Colbert et al. 2001).
b) in T-DNA or transposon mutated population by reserve genetics
   Reverse genetic strategies to identify insertion mutants in genes of interest have been described for various cases expecially Arabidopsis eg. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999 ,11 , 1853-1866). Also for crops insertional Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (e.g. T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290). Rescreening of lower levels DNA pools lead to the identification of individual plants in which the gene of interest is disrupted by the insertional mutagen. These methods can as well be applied to crop populations that are mutated with insertional mutagenesis such as corn (Fernandes J, Dong Q, Schneider B, Morrow DJ, Nan GL, Brendel V, Walbot V.Genome Biol. 2004;5(10):R82. Epub 2004 Sep 23 Genome-wide mutagenesis of Zea mays L. using RescueMu transposons) or rice (Sallaud C, Gay C, Larmande P, Bes M, Piffanelli P, Piegu B, Droc G, Regad F, Bourgeois E, Meynard D, Perin C, Sabau X, Ghesquiere A, Glaszmann JC, Delseny M, Guiderdoni E. Plant J. 2004 Aug;39(3):450-64. High throughput T-DNA insertion mutagenesis in rice: a first step towards in silico reverse genetics)
   Furthermore systematic sequencing of insertions sides in T-DNA or transposon mutagenized population is under way also in crops allowing the simple in silico search for crop lines having mutated the genes of the invention (Sallaud C, Gay C, Larmande P, Bes M, Piffanelli P, Piegu B, Droc G, Regad F, Bourgeois E, Meynard D, Perin C, Sabau X, Ghesquiere A, Glaszmann JC, Delseny M, Guiderdoni E. Plant J. 2004 Aug;39(3):450-64. High throughput T-DNA insertion mutagenesis in rice: a first step towards in silico reverse genetics; Ryu CH, You JH, Kang HG, Hur J, Kim YH, Han MJ, An K, Chung BC, Lee CH, An G., Plant Mol Biol. 2004 Mar;54(4):489-502., Generation of T-DNA tagging lines with a bidirectional gene trap vector and the establishment of an insertion-site database.)

## Claims

1. A transformed plant cell with tolerance and/or resistance to an environmental stress as compared to a corresponding non-transformed wild type plant cell, wherein the tolerance and/or resistance to an environmental stress is increased by an inactivated or down-regulated gene encoded by one or more nucleic acid sequences selected from the group consisting of:
a) nucleic acid molecule encoding the polypeptide according to SEQ ID NO:54;
b) nucleic acid molecule comprising the nucleic acid molecule according to SEQ ID NO:53;
c) nucleic acid molecule comprising a nucleic acid sequence, which, as a result of the degeneracy of the genetic code, can be derived from a polypeptide sequence depicted in SEQ ID NO:54;
d) nucleic acid molecule encoding a polypeptide having at least 80% identity with the amino acid sequence of the polypeptide encoded by the nucleic acid molecule of (a) to (c) and having the biological activity represented by protein of SEQ ID NO:54;
or which comprises a sequence which is complementary thereto.

2. The transformed plant cell of 1 derived from a monocotyledonous plant.

3. The transformed plant cell of 1 derived from a dicotyledonous plant.

4. The transformed plant cell of one of the claims 1-3, wherein the plant is selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

5. The transformed plant cell of claim 1, derived from a gymnosperm plant.

6. A transformed plant generated from a plant cell according to of one of the claims 1-4 and which is a monocot or dicot plant.

7. A transformed plant of claim 6, which is selected from the group comprised of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops and Arabidopsis thaliana, preferably Brassica napus, Glycine max, Zea mays or Oryza sativa.

8. A transformed plant generated from a plant cell according to claim 1 or 5 and which is a gymnosperm plant.

9. A seed produced by a transformed plant of one of the claims 6 to 8, wherein the seed is at least genetically heterozygous for a gene
a. comprising a nucleic acid as defined in claim 1, that when down-regulated confers increased tolerance to environmental stress as compared to a wild type plant, or
b. the seed is at least genetically heterozygous for an inactivated gene that before inactivation comprised a nucleic acid as defined in claim 1.

10. A method of producing a transformed plant with increased tolerance and/or resistance to environmental stress as compared to a corresponding non-transformed wild type plant by inactivation or down-regulation of a gene in the transformed plant resulting in increased tolerance and/or resistance to environmental stress as compared to a corresponding non-transformed wild type plant, comprising
(a) transforming a plant cell by inactivation or down-regulation of one or more genes, encoded by one or more nucleic acids selected from
aa) nucleic acid molecule encoding the polypeptide according to SEQ ID NO:54;
bb) nucleic acid molecule comprising the nucleic acid molecule according to SEQ ID NO:53;
cc) nucleic acid molecule comprising a nucleic acid sequence, which, as a result of the degeneracy of the genetic code, can be derived from a polypeptide sequence depicted in SEQ ID NO:54;
dd) nucleic acid molecule encoding a polypeptide having at least 80% identity with the amino acid sequence of the polypeptide encoded by the nucleic acid molecule of (aa) to (cc) and having the biological activity represented by protein of SEQ ID NO:54;
and
(b) generating from the plant cell a transformed plant with an increased tolerance and/or resistance to environmental stress as compared to a corresponding wild type plant.

11. A method of inducing increased tolerance and/or resistance to environmental stress as compared to a corresponding non-transformed wild type plant in a plant cell of one of the claims 1 - 6 or plant of one of the claims 7 - 9 by inactivation or down-regulation of one or more genes encoded by one or more nucleic acids selected from a group consisting of the nucleic acid molecules as defined in claim 1.

12. A plant expression cassette comprising a nucleic acid construct, which when expressed allows inactivation or down-regulation of one or more genes encoded by one or more nucleic acids selected from the group consisting of the nucleic acid molecules as defined in claim 1 by a method of one of the claims 10 - 11.

13. A method of detecting environmental stress in plant cells according to claims 1 - 5 or plants according to claims 6-8 comprising screening the plant cells for increased tolerance and/or resistance to environmental stress as compared to non-stress conditions.

14. A method of screening plant cells according to claims 1 - 5 or plants according to claims 6 - 8 for increased tolerance and/or resistance to environmental stress comprising screening the plant cells under stress conditions for increased tolerance and/or resistance to environmental stress as compared to non-stress conditions.

15. The method of one of the claims 13 - 14, wherein the increased tolerance and/or resistance to environmental stress is due to one or more inactivated or down-regulated genes encoded by one or more nucleic acid sequences selected from the group consisting of
a) nucleic acid molecule encoding the polypeptide according to SEQ ID NO:54;
b) nucleic acid molecule comprising the nucleic acid molecule according to SEQ ID NO:53;
c) nucleic acid molecule comprising a nucleic acid sequence, which, as a result of the degeneracy of the genetic code, can be derived from a polypeptide sequence depicted in SEQ ID NO:54;
d) nucleic acid molecule encoding a polypeptide having at least 80% identity with the amino acid sequence of the polypeptide encoded by the nucleic acid molecule of (a) to (c) and having the biological activity represented by protein of SEQ ID NO:54.

16. The method of one of the claims 13 - 15, wherein tolerance and/or resistance to environmental stress is increased by one or more inactivated or down-regulated genes encoded by one or more nucleic acid sequences selected from the group consisting of the nucleic acid molecules as defined in claim 1.

17. A transformed plant cell with an inactivated or down-regulated gene encoded by a nucleic acid sequence selected from the group consisting of the nucleic acid molecules as defined in claim 1.

18. An nucleic acid molecule which comprises a nucleic acid molecule selected from the group consisting of:
a) nucleic acid molecule which encodes a polypeptide comprising the polypeptide according to SEQ ID NO:54;
b) nucleic acid molecule which comprising the polynucleotide shown in SEQ ID NO:53;
c) nucleic acid molecule comprising a nucleic acid sequence, which, as a result of the degeneracy of the genetic code, can be derived from a polypeptide sequence depicted (b) and having the biological activity represented by protein according to SEQ ID NO:54;
d) nucleic acid molecule encoding a polypeptide having at least 80% identity with the amino acid sequence of the polypeptide encoded by the nucleic acid molecule of (a) or (c) and having a biological activity represented by protein according to SEQ ID NO:54;
e) a nucleic acid molecule having at least 80% sequence identity to polynucleotide selected from the groups consisting of the polynucleotides shown in SEQ ID NO:53;
or which comprises a sequence which is complementary thereto; whereby the nucleic acid molecule according to (a) to (e) is at least in one or more nucleotides different from the sequence depicted in SEQ ID NO:53 and which encodes a protein which differs at least in one or more amino acids from the protein sequences depicted in SEQ ID NO:54.

19. A polypeptide encoded by a nucleic acid molecule as claimed in claim 18.

20. An antibody, which specifically binds to the polypeptide as claimed in claim 19.

21. Use of the nucleic acid according to claim 18 for producing a transformed plant with increased tolerance and/or resistance to environmental stress as compared to a corresponding non-transformed wild type plant.

## Patentansprüche

1. Transformierte Pflanzenzelle mit erhöhter Toleranz und/oder Resistenz gegen einen Umweltstress verglichen mit einer entsprechenden nicht transformierten Wildtyppflanzenzelle, wobei die Toleranz und/oder Resistenz gegen einen Umweltstress durch ein inaktiviertes oder hinunterreguliertes Gen erhöht wird, das von einer oder mehreren Nukleinsäuresequenzen, ausgewählt aus der folgenden Gruppe:
a) Nukleinsäuremolekül, das für das Polypeptid nach SEQ ID NO: 54 codiert;
b) Nukleinsäuremolekül, das das Nukleinsäuremolekül gemäß SEQ ID NO: 53 umfasst;
c) Nukleinsäuremolekül, umfassend eine Nukleinsäuresequenz, die sich aufgrund der Degeneration des genetischen Codes von einer Polypeptidsequenz gemäß SEQ ID NO: 54 ableiten lässt;
d) Nukleinsäuremolekül, das für ein Polypeptid mit mindestens 80% Identität zu der Aminosäuresequenz des von dem Nukleinsäuremolekül gemäß (a) bis (c) codierten Polypeptids codiert und das die biologische Wirksamkeit gemäß dem Protein von SEQ ID NO: 54 aufweist;
codiert wird oder das eine Sequenz umfasst, die hierzu komplementär ist.

2. Transformierte Pflanzenzelle nach Anspruch 1, die von einer monokotylen Pflanze abgeleitet ist.

3. Transformierte Pflanzenzelle nach Anspruch 1, die von einer dikotylen Pflanze abgeleitet ist.

4. Transformierte Pflanzenzelle nach einem der Ansprüche 1-3, wobei die Pflanze aus der Gruppe umfassend Mais, Weizen, Roggen, Hafer, Triticale, Reis, Gerste, Sojabohne, Erdnuss, Baumwolle, Raps, Canola, Maniok, Pfeffer, Sonnenblume, Flachs, Borretsch, Saflor, Lein, Nachtkerze, Rübsen, Tagetes, Solanaceen, Kartoffel, Tabak, Aubergine, Tomate, Vicia-Arten, Erbse, Luzerne, Kaffee, Kakao, Tee, Salix-Arten, Ölpalme, Kokosnuss, mehrjähriges Gras, Futterkulturen und Arabidopsis thaliana, vorzugsweise Brassica napus, Glycine max, Zea mays oder Oryza sativa ausgewählt ist.

5. Transformierte Pflanzenzelle nach Anspruch 1, die von einer nacktsamigen Pflanze abgeleitet ist.

6. Transformierte Pflanze, die ausgehend von einer Pflanzenzelle nach einem der Ansprüche 1 - 4 erzeugt ist und bei der es sich um eine monokotyle oder dikotyle Pflanze handelt.

7. Transformierte Pflanze nach Anspruch 6, die aus der Gruppe umfassend Mais, Weizen, Roggen, Hafer, Triticale, Reis, Gerste, Sojabohne, Erdnuss, Baumwolle, Raps, Canola, Maniok, Pfeffer, Sonnenblume, Flachs, Borretsch, Saflor, Lein, Nachtkerze, Rübsen, Tagetes, Solanaceen, Kartoffel, Tabak, Aubergine, Tomate, Vicia-Arten, Erbse, Luzerne, Kaffee, Kakao, Tee, Salix-Arten, Ölpalme, Kokosnuss, mehrjähriges Gras, Futterkulturen und Arabidopsis thaliana, vorzugsweise Brassica napus, Glycine max, Zea mays oder Oryza sativa ausgewählt ist.

8. Transformierte Pflanze, die ausgehend von einer Pflanzenzelle nach Anspruch 1 oder 5 erzeugt ist und bei der es sich um eine nacktsamige Pflanze handelt.

9. Samen, der von einer transformierten Pflanze nach einem der Ansprüche 6 bis 8 produziert wird, wobei der Samen mindestens für ein Gen genetisch heterozygot ist,
a. umfassend eine Nukleinsäure wie in Anspruch 1 definiert, die, wenn sie hinunterreguliert wird, eine erhöhte Toleranz gegen Umweltstress verglichen mit einer Wildtyppflanze verleiht, oder
b. der Samen mindestens für ein inaktiviertes Gen, das vor der Inaktivierung eine Nukleinsäure wie in Anspruch 1 definiert umfasst hat, genetisch heterozygot ist.

10. Verfahren zur Herstellung einer transformierten Pflanze mit erhöhter Toleranz und/oder Resistenz gegen Umweltstress verglichen mit einer entsprechenden nicht transformierten Wildtyppflanze durch Inaktivieren oder Hinunterregulieren eines Gens in der transformierten Pflanze, was zu einer erhöhten Toleranz und/oder Resistenz gegen Umweltstress verglichen mit einer entsprechenden nicht transformierten Wildtyppflanze führt, das Folgendes umfasst:
(a) Transformieren einer Pflanzenzelle durch Inaktivieren oder Hinunterregulieren von einem oder mehreren Genen, die von einer oder mehreren Nukleinsäuren, ausgewählt aus:
aa) Nukleinsäuremolekül, das für das Polypeptid nach SEQ ID NO: 54 codiert;
bb) Nukleinsäuremolekül, das das Nukleinsäuremolekül gemäß SEQ ID NO: 53 umfasst;
cc) Nukleinsäuremolekül, umfassend eine Nukleinsäuresequenz, die sich aufgrund der Degeneration des genetischen Codes von einer Polypeptidsequenz gemäß SEQ ID NO: 54 ableiten lässt;
dd) Nukleinsäuremolekül, das für ein Polypeptid mit mindestens 80% Identität zu der Aminosäuresequenz des von dem Nukleinsäuremolekül gemäß (aa) bis (cc) codierten Polypeptids codiert und das die biologische Wirksamkeit gemäß dem Protein von SEQ ID NO: 54 aufweist;
codiert werden,
und,
(b) ausgehend von der Pflanzenzelle, Erzeugen einer transformierten Pflanze mit einer erhöhten Toleranz und/oder Resistenz gegen Umweltstress verglichen mit einer entsprechenden Wildtyppflanze.

11. Verfahren zum Induzieren von erhöhter Toleranz und/oder Resistenz gegen Umweltstress verglichen mit einer entsprechenden nicht transformierten Wildtyppflanze in einer Pflanzenzelle nach einem der Ansprüche 1 - 6 oder in einer Pflanze nach einem der Ansprüche 7 - 9 durch Inaktivieren oder Hinunterregulieren von einem oder mehreren Genen, die von einer oder mehreren Nukleinsäuren, ausgewählt aus einer Gruppe bestehend aus den Nukleinsäuremolekülen wie in Anspruch 1 definiert, codiert werden.

12. Pflanzenexpressionskassette, umfassend ein Nukleinsäurekonstrukt, das, wenn es exprimiert wird, das Inaktivieren oder Hinunterregulieren von einem oder mehreren Genen, die von einer oder mehreren Nukleinsäuren, ausgewählt aus der Gruppe bestehend aus den Nukleinsäuremolekülen wie in Anspruch 1 definiert, codiert werden, nach einem Verfahren nach einem der Ansprüche 10 - 11 gestattet.

13. Verfahren zum Nachweisen von Umweltstress in Pflanzenzellen nach den Ansprüchen 1 - 5 oder in Pflanzen nach den Ansprüchen 6 - 8, bei dem man die Pflanzenzellen auf erhöhte Toleranz und/oder Resistenz gegen Umweltstress verglichen mit Nichtstressbedingungen durchmustert.

14. Verfahren zum Durchmustern von Pflanzenzellen nach den Ansprüchen 1 - 5 oder von Pflanzen nach den Ansprüchen 6 - 8 auf erhöhte Toleranz und/oder Resistenz gegen Umweltstress, bei dem man die Pflanzenzellen unter Stressbedingungen auf erhöhte Toleranz und/oder Resistenz gegen Umweltstress verglichen mit Nichtstressbedingungen durchmustert.

15. Verfahren nach einem der Ansprüche 13 - 14, wobei die erhöhte Toleranz und/oder Resistenz gegen Umweltstress auf einem oder mehreren inaktivierten oder hinunterregulierten Genen, codiert von einer oder mehreren Nukleinsäuresequenzen, ausgewählt aus der Gruppe bestehend aus
a) Nukleinsäuremolekül, das für das Polypeptid nach SEQ ID NO: 54 codiert;
b) Nukleinsäuremolekül, das das Nukleinsäuremolekül gemäß SEQ ID NO: 53 umfasst;
c) Nukleinsäuremolekül, umfassend eine Nukleinsäuresequenz, die sich aufgrund der Degeneration des genetischen Codes von einer Polypeptidsequenz gemäß SEQ ID NO: 54 ableiten lässt;
d) Nukleinsäuremolekül, das für ein Polypeptid mit mindestens 80% Identität zu der Aminosäuresequenz des von dem Nukleinsäuremolekül gemäß (a) bis (c) codierten Polypeptids codiert und das die biologische Wirksamkeit gemäß dem Protein von SEQ ID NO: 54 aufweist;
beruht.

16. Verfahren nach einem der Ansprüche 13 - 15, wobei die Toleranz und/oder Resistenz gegen Umweltstress durch ein oder mehrere inaktivierte oder hinunterregulierte Gene, codiert von einer oder mehreren Nukleinsäuresequenzen, ausgewählt aus der Gruppe bestehend aus den Nukleinsäuremolekülen wie in Anspruch 1 definiert, erhöht wird.

17. Transformierte Pflanzenzelle mit einem inaktivierten oder hinunterregulierten Gen, das von einer Nukleinsäuresequenz, ausgewählt aus der Gruppe bestehend aus den Nukleinsäuremolekülen wie in Anspruch 1 definiert, codiert wird.

18. Nukleinsäuremolekül, das ein Nukleinsäuremolekül, ausgewählt aus der Gruppe bestehend aus:
a) Nukleinsäuremolekül, das für ein das Polypeptid nach SEQ ID NO: 54 umfassendes Polypeptid codiert;
b) Nukleinsäuremolekül, das das Polynukleotid gemäß SEQ ID NO: 53 umfasst;
c) Nukleinsäuremolekül, umfassend eine Nukleinsäuresequenz, die sich aufgrund der Degeneration des genetischen Codes von einer dargestellten Polypeptidsequenz (b) ableiten lässt, die die biologische Wirksamkeit gemäß dem Protein von SEQ ID NO: 54 aufweist;
d) Nukleinsäuremolekül, das für ein Polypeptid mit mindestens 80% Identität zu der Aminosäuresequenz des von dem Nukleinsäuremolekül gemäß (a) bis (c) codierten Polypeptids codiert und das die biologische Wirksamkeit gemäß dem Protein von SEQ ID NO: 54 aufweist;
e) Nukleinsäuremolekül mit mindestens 80% Sequenzidentität zu einem Polynukleotid, ausgewählt aus den Gruppen bestehend aus den Polynukleotiden gemäß SEQ ID NO: 53;
umfasst
oder das eine Sequenz, die hierzu komplementär ist, umfasst; wobei sich das Nukleinsäuremolekül nach (a) bis (e) in mindestens einem oder mehreren Nukleotiden, das/die zu der in SEQ ID NO: 53 angegebenen Sequenz unterschiedlich ist/sind, unterscheidet, und das für ein Protein codiert, das sich in mindestens einer oder mehreren Aminosäuren aus den in SEQ ID NO: 54 angegebenen Proteinsequenzen unterscheidet.

19. Polypeptid, das von einem Nukleinsäuremolekül nach Anspruch 18 codiert wird.

20. Antikörper, der spezifisch an das Polypeptid nach Anspruch 19 bindet.

21. Verwendung der Nukleinsäure nach Anspruch 18 zur Herstellung einer transformierten Pflanze mit erhöhter Toleranz und/oder Resistenz gegen Umweltstress verglichen mit einer entsprechenden nicht transformierten Wildtyppflanze.

## Revendications

1. Cellule végétale transformée ayant une tolérance et/ou une résistance accrues envers un stress environnemental par rapport à une cellule végétale correspondante de type sauvage et non transformée, dans laquelle la tolérance et/ou la résistance envers un stress environnemental est accrue par un gène inactivé ou régulé à la baisse codé par une ou plusieurs séquences d'acide nucléique sélectionnées dans le groupe constitué par :
a) une molécule d'acide nucléique codant pour le polypeptide selon SEQ ID n° 54 ;
b) une molécule d'acide nucléique comprenant la molécule d'acide nucléique selon SEQ ID n° 53 ;
c) une molécule d'acide nucléique comprenant une séquence d'acide nucléique, laquelle peut, du fait de la dégénérescence du code génétique, être dérivée d'une séquence polypeptidique illustrée dans SEQ ID n° 54 ;
d) une molécule d'acide nucléique codant pour un polypeptide ayant au moins 80% d'identité avec la séquence d'acides aminés du polypeptide codé par la molécule d'acide nucléique de (a) à (c) et ayant l'activité biologique représentée par la protéine de SEQ ID n° 54 ;
ou qui comprend une séquence qui est complémentaire à celle-ci.

2. Cellule végétale transformée selon la revendication 1, dérivée d'une plante monocotylédone.

3. Cellule végétale transformée selon la revendication 1, dérivée d'une plante dicotylédone.

4. Cellule végétale transformée selon l'une des revendications 1-3, dans laquelle la plante est sélectionnée dans le groupe constitué par le maïs, le blé, le seigle, l'avoine, le triticale, le riz, l'orge, le soja, l'arachide, le coton, le colza, le canola, le manioc, le poivre, le tournesol, le lin, la bourrache, le carthame, la graine de lin, l'onagre, le navet, le tagète, les solanacées, la pomme de terre, le tabac, l'aubergine, la tomate, les espèces de Vicia, le pois, la luzerne, le café, le cacao, le thé, les espèces de Salix, le palmier à huile, la noix de coco, les herbes vivaces, les cultures fourragères et *Arabidopsis thaliana,* de préférence *Brassica napus, Glycine max, Zea mays* ou *Oryza sativa.*

5. Cellule végétale transformée selon la revendication 1, dérivée d'une gymnosperme.

6. Plante transformée générée à partir d'une cellule végétale selon l'une des revendications 1-4 et qui est une plante monocotylédone ou dicotylédone.

7. Plante transformée selon la revendication 6, qui est sélectionnée dans le groupe constitué par le maïs, le blé, le seigle, l'avoine, le triticale, le riz, l'orge, le soja, l'arachide, le coton, le colza, le canola, le manioc, le poivre, le tournesol, le lin, la bourrache, le carthame, la graine de lin, l'onagre, le navet, le tagète, les solanacées, la pomme de terre, le tabac, l'aubergine, la tomate, les espèces de Vicia, le pois, la luzerne, le café, le cacao, le thé, les espèces de Salix, le palmier à huile, la noix de coco, les herbes vivaces, les cultures fourragères et *Arabidopsis thaliana,* de préférence *Brassica napus, Glycine max, Zea mays* ou *Oryza sativa.*

8. Plante transformée générée à partir d'une cellule végétale selon la revendication 1 ou 5 et qui est une gymnosperme.

9. Graine produite par une plante transformée selon l'une des revendications 6 à 8, dans laquelle la graine est génétiquement hétérozygote pour au moins un gène
a. comprenant un acide nucléique tel que défini selon la revendication 1, qui, lorsqu'il est régulé à la baisse, confère une tolérance accrue envers un stress environnemental par rapport à une plante de type sauvage, ou
b. la graine est au moins génétiquement hétérozygote pour un gène inactivé qui, avant l'inactivation, comprenait un acide nucléique tel que défini selon la revendication 1.

10. Méthode de production d'une plante transformée ayant une tolérance et/ou une résistance accrue envers un stress environnemental par rapport à une plante correspondante de type sauvage et non transformée, par inactivation ou régulation à la baisse d'un gène dans la plante transformée, résultant en une tolérance et/ou une résistance accrue envers un stress environnemental par rapport à une plante correspondante de type sauvage et non transformée, comprenant
(a) la transformation d'une cellule végétale par inactivation ou régulation à la baisse d'un ou plusieurs gènes, codés par un ou plusieurs acides nucléiques sélectionnés parmi
aa) une molécule d'acide nucléique codant pour le polypeptide selon SEQ ID n° 54 ;
bb) une molécule d'acide nucléique comprenant la molécule d'acide nucléique selon SEQ ID n° 53 ;
cc) une molécule d'acide nucléique comprenant une séquence d'acide nucléique, laquelle peut, du fait de la dégénérescence du code génétique, être dérivée d'une séquence polypeptidique illustrée dans SEQ ID n° 54 ;
dd) une molécule d'acide nucléique codant pour un polypeptide ayant au moins 80% d'identité avec la séquence d'acides aminés du polypeptide codé par la molécule d'acide nucléique de (aa) à (cc) et ayant l'activité biologique représentée par la protéine de SEQ ID n° 54 ;
et
(b) la génération à partir de la cellule végétale d'une plante transformée ayant une tolérance et/ou une résistance accrue envers un stress environnemental par rapport à une plante correspondante de type sauvage.

11. Méthode d'induction d'une tolérance et/ou d'une résistance accrue envers un stress environnemental par rapport à une plante correspondante de type sauvage et non transformée dans une cellule végétale selon l'une des revendications 1-6 ou dans une plante selon l'une des revendications 7-9 par inactivation ou régulation à la baisse d'un ou plusieurs gènes codés par un ou plusieurs acides nucléiques sélectionnes dans un groupe constitué par les molécules d'acide nucléique telles que définies selon la revendication 1.

12. Cassette d'expression végétale, comprenant un produit de recombinaison d'acide nucléique, laquelle, lors de son expression, permet l'inactivation ou la régulation à la baisse d'un ou plusieurs gènes codés par un ou plusieurs acides nucléiques sélectionnés dans le groupe constitué par les molécules d'acide nucléique telles que définies selon la revendication 1, par une méthode selon l'une des revendications 10-11.

13. Méthode de détection d'un stress environnemental dans des cellules végétales selon les revendications 1-5 ou dans des plantes selon les revendications 6-8, comprenant le criblage des cellules végétales pour une tolérance et/ou une résistance accrue envers un stress environnemental par rapport à des conditions dépourvues de stress.

14. Méthode de criblage de cellules végétales selon les revendications 1-5 ou de plantes selon les revendications 6-8 pour une tolérance et/ou une résistance accrue envers un stress environnemental, comprenant le criblage des cellules végétales dans des conditions de stress pour une tolérance et/ou une résistance accrue envers un stress environnemental par rapport à des conditions dépourvues de stress.

15. Méthode selon l'une des revendications 13-14, dans laquelle la tolérance et/ou la résistance accrue envers un stress environnemental est due à un ou plusieurs gènes inactivés ou régulés à la baisse codés par une ou plusieurs séquences d'acide nucléique sélectionnées dans le groupe constitué par
a) une molécule d'acide nucléique codant pour le polypeptide selon SEQ ID n° 54 ;
b) une molécule d'acide nucléique comprenant la molécule d'acide nucléique selon SEQ ID n° 53 ;
c) une molécule d'acide nucléique comprenant une séquence d'acide nucléique, laquelle peut, du fait de la dégénérescence du code génétique, être dérivée d'une séquence polypeptidique illustrée dans SEQ ID n° 54 ;
d) une molécule d'acide nucléique codant pour un polypeptide ayant au moins 80% d'identité avec la séquence d'acides aminés du polypeptide codé par la molécule d'acide nucléique de (a) à (c) et ayant l'activité biologique représentée par la protéine de SEQ ID n° 54.

16. Méthode selon l'une des revendications 13-15, dans laquelle la tolérance et/ou la résistance au stress environnemental est accrue par un ou plusieurs gènes inactivés ou régulés à la baisse codés par une ou plusieurs séquences d'acide nucléique sélectionnées dans le groupe constitué par les molécules d'acide nucléique telles que définies selon la revendication 1.

17. Cellule végétale transformée ayant un gène inactivé ou régulé à la baisse codé par une séquence d'acide nucléique sélectionnée dans le groupe constitué par les molécules d'acide nucléique telles que définies selon la revendication 1.

18. Molécule d'acide nucléique qui comprend une molécule d'acide nucléique sélectionnée dans le groupe constitué par :
a) une molécule d'acide nucléique codant pour un polypeptide comprenant le polypeptide selon SEQ ID n° 54 ;
b) une molécule d'acide nucléique comprenant le polynucléotide illustré dans SEQ ID n° 53 ;
c) une molécule d'acide nucléique comprenant une séquence d'acide nucléique, laquelle peut, du fait de la dégénérescence du code génétique, être dérivée d'une séquence polypeptidique présentée en (b) et ayant l'activité biologique représentée par la protéine selon SEQ ID n° 54 ;
d) une molécule d'acide nucléique codant pour un polypeptide ayant au moins 80% d'identité avec la séquence d'acides aminés du polypeptide codé par la molécule d'acide nucléique de (a) à (c) et ayant une activité biologique représentée par la protéine selon SEQ ID n° 54 ;
e) une molécule d'acide nucléique ayant au moins 80% d'identité de séquence avec un polynucléotide sélectionné dans le groupe constitué par les polynucléotides illustrés dans SEQ ID n° 53 ;
ou qui comprend une séquence complémentaire à celle-ci ; ce par quoi la molécule d'acide nucléique selon (a) à (e) est différente au niveau d'au moins un ou plusieurs nucléotides par rapport à la séquence illustrée dans SEQ ID n° 53 et qui code pour une protéine qui est différente au niveau d'au moins un ou plusieurs acides aminés par rapport aux séquences protéiques illustrées dans SEQ ID n° 54.

19. Polypeptide codé par une molécule d'acide nucléique selon la revendication 18.

20. Anticorps qui se fixe spécifiquement au polypeptide selon la revendication 19.

21. Utilisation de l'acide nucléique selon la revendication 18 pour la production d'une plante transformée ayant une tolérance et/ou une résistance accrue envers un stress environnemental par rapport à une plante correspondante de type sauvage et non transformée.


### SEQUENCE LISTING

<110> BASF Plant Science GmbH
<120> PLANT CELLS AND PLANTS WITH INCREASED TOLERANCE TO ENVIRONMENTAL STRESS
<130> BASFPF56031PCTEP
<160> 876
<170> PatentIn version 3.2
<210> 1
   <211> 1458
   <212> DNA
   <213> Arabidopsis thaliana
<400> 1
   "000"
<210> 2
   <211> 485
   <212> PRT
   <213> Arabidopsis thaliana
<400> 2
   "000"
<210> 3
   <211> 402
   <212> DNA
   <213> Arabidopsis thaliana
<400> 3
   "000"
<210> 4
   <211> 133
   <212> PRT
   <213> Arabidopsis thaliana
<400> 4
   "000"
<210> 5
   <211> 1470
   <212> DNA
   <213> Arabidopsis thaliana
<400> 5
   "000"
<210> 6
   <211> 489
   <212> PRT
   <213> Arabidopsis thaliana
<400> 6
   "000"
<210> 7
   <211> 1584
   <212> DNA
   <213> Arabidopsis thaliana
<400> 7
   "000"
<210> 8
   <211> 527
   <212> PRT
   <213> Arabidopsis thaliana
<400> 8
   "000"
<210> 9
   <211> 1836
   <212> DNA
   <213> Arabidopsis thaliana
<400> 9
   "000"
<210> 10
   <211> 611
   <212> PRT
   <213> Arabidopsis thaliana
<400> 10
   "000"
<210> 11
   <211> 1437
   <212> DNA
   <213> Arabidopsis thaliana
<400> 11
   "000"
<210> 12
   <211> 478
   <212> PRT
   <213> Arabidopsis thaliana
<400> 12
   "000"
<210> 13
   <211> 1584
   <212> DNA
   <213> Arabidopsis thaliana
<400> 13
   "000"
<210> 14
   <212> PRT
   <213> Arabidopsis thaliana
<400> 14
   "000"
<210> 15
   <211> 798
   <212> DNA
   <213> Arabidopsis thaliana
<400> 15
   "000"
<210> 16
   <211> 265
   <212> PRT
   <213> Arabidopsis thaliana
<400> 16
   "000"
<210> 17
   <211> 2265
   <212> DNA
   <213> Arabidopsis thaliana
<400> 17
   "000"
<210> 18
   <211> 754
   <212> PRT
   <213> Arabidopsis thaliana
<400> 18
   "000"
<210> 19
   <211> 2304
   <212> DNA
   <213> Arabidopsis thaliana
<400> 19
   "000"
<210> 20
   <211> 767
   <212> PRT
   <213> Arabidopsis thaliana
<400> 20
   "000"
<210> 21
   <211> 1512
   <212> DNA
   <213> Arabidopsis thaliana
<400> 21
   "000"
<210> 22
   <211> 503
   <212> PRT
   <213> Arabidopsis thaliana
<400> 22
   "000"
<210> 23
   <211> 1359
   <212> DNA
   <213> Arabidopsis thaliana
<400> 23
   "000"
<210> 24
   <211> 452
   <212> PRT
   <213> Arabidopsis thaliana
<400> 24
   "000"
<210> 25
   <211> 1875
   <212> DNA
   <213> Arabidopsis thaliana
<400> 25
   "000"
<210> 26
   <211> 624
   <212> PRT
   <213> Arabidopsis thaliana
<400> 26
   "000"
<210> 27
   <211> 1074
   <212> DNA
   <213> Arabidopsis thaliana
<400> 27
   "000"
<210> 28
   <211> 357
   <212> PRT
   <213> Arabidopsis thaliana
<400> 28
   "000"
<210> 29
   <211> 1074
   <212> DNA
   <213> Arabidopsis thaliana
<400> 29
   "000"
<210> 30
   <211> 357
   <212> PRT
   <213> Arabidopsis thaliana
<400> 30
   "000"
<210> 31
   <211> 597
   <212> DNA
   <213> Arabidopsis thaliana
<400> 31
   "000"
<210> 32
   <211> 198
   <212> PRT
   <213> Arabidopsis thaliana
<400> 32
   "000"
<210> 33
   <211> 5163
   <212> DNA
   <213> Arabidopsis thaliana
<400> 33
   "000"
<210> 34
   <211> 1720
   <212> PRT
   <213> Arabidopsis thaliana
<400> 34
   "000"
<210> 35
   <211> 1719
   <212> DNA
   <213> Arabidopsis thaliana
<400> 35
   "000"
<210> 36
   <211> 572
   <212> PRT
   <213> Arabidopsis thaliana
<400> 36
   "000"
<210> 37
   <211> 465
   <212> DNA
   <213> Arabidopsis thaliana
<400> 37
   "000"
<210> 38
   <211> 154
   <212> PRT
   <213> Arabidopsis thaliana
<400> 38
   "000"
<210> 39
   <211> 1662
   <212> DNA
   <213> Arabidopsis thaliana
<400> 39
   "000"
<210> 40
   <211> 553
   <212> PRT
   <213> Arabidopsis thaliana
<400> 40
   "000"
<210> 41
   <211> 936
   <212> DNA
   <213> Arabidopsis thaliana
<400> 41
   "000"
<210> 42
   <211> 311
   <212> PRT
   <213> Arabidopsis thaliana
<400> 42
   "000"
<210> 43
   <211> 1431
   <212> DNA
   <213> Arabidopsis thaliana
<400> 43
   "000"
<210> 44
   <211> 476
   <212> PRT
   <213> Arabidopsis thaliana
<400> 44
   "000"
<210> 45
   <211> 477
   <212> DNA
   <213> Arabidopsis thaliana
<400> 45
   "000"
<210> 46
   <211> 158
   <212> PRT
   <213> Arabidopsis thaliana
<400> 46
   "000"
<210> 47
   <211> 744
   <212> DNA
   <213> Arabidopsis thaliana
<400> 47
   "000"
<210> 48
   <211> 247
   <212> PRT
   <213> Arabidopsis thaliana
<400> 48
   "000"
<210> 49
   <211> 1218
   <212> DNA
   <213> Arabidopsis thaliana
<400> 49
   "000"
<210> 50
   <211> 405
   <212> PRT
   <213> Arabidopsis thaliana
<400> 50
   "000"
<210> 51
   <211> 1152
   <212> DNA
   <213> Arabidopsis thaliana
<400> 51
   "000"
<210> 52
   <211> 383
   <212> PRT
   <213> Arabidopsis thaliana
<400> 52
   "000"
<210> 53
   <211> 1464
   <212> DNA
   <213> Arabidopsis thaliana
<220>
   <221> CDS
   <222> (1)..(1464)
<400> 53
   atg caa cct tgg aga aga aaa ttt cca ttg ttc gaa aca gga gtt aca       48
   Met Gln Pro Trp Arg Arg Lys Phe Pro Leu Phe Glu Thr Gly Val Thr
   1               5                   10                  15
   atg aaa cag agg aag aac agt aat ctc tca atc ttc gtc gta gtc ttc       96
   Met Lys Gln Arg Lys Asn Ser Asn Leu Ser Ile Phe Val Val Val Phe
               20                  25                  30
   tct gtt ttc ctc ttc ggg atc ttc atg tac aat gaa gac gtc aag tcc      144
   Ser Val Phe Leu Phe Gly Ile Phe Met Tyr Asn Glu Asp Val Lys Ser
           35                  40                  45
   atc gca gag ttt cct ttc tcc acc tca aaa cct cac gac gtc cac gac      192
   Ile Ala Glu Phe Pro Phe Ser Thr Ser Lys Pro His Asp Val His Asp
       50                  55                  60
   gaa gcg aca ccg atc aca gag atc aca aca cta ccg gtt caa gag tcg      240
   Glu Ala Thr Pro Ile Thr Glu Ile Thr Thr Leu Pro Val Gln Glu Ser
   65                  70                  75                  80
   atc aag aac tca gac cca att cag gaa tcg atc aaa aac gca gac tca      288
   Ile Lys Asn Ser Asp Pro Ile Gln Glu Ser Ile Lys Asn Ala Asp Ser
                   85                  90                  95
   gtt caa gat tca gtt aaa gac gta gca gaa cca gtt caa gag gag gta      336
   Val Gln Asp Ser Val Lys Asp Val Ala Glu Pro Val Gln Glu Glu Val
               100                 105                 110
   tcg aaa aca gag gaa gtt aaa aag att gag ctt ttc gct gcg acg gag      384
   Ser Lys Thr Glu Glu Val Lys Lys Ile Glu Leu Phe Ala Ala Thr Glu
           115                 120                 125
   gat gaa gaa gac gtg gaa ttg ccg ccg gag gaa tgc gat ttg ttc acc      432
   Asp Glu Glu Asp Val Glu Leu Pro Pro Glu Glu Cys Asp Leu Phe Thr
       130                 135                 140
   gga gaa tgg gtt ttt gat aac gag acg cat cca ttg tat aaa gag gat      480
   Gly Glu Trp Val Phe Asp Asn Glu Thr His Pro Leu Tyr Lys Glu Asp
   145                 150                 155                 160
   caa tgt gag ttc ttg acg gcg caa gtc act tgc atg aga aat gga aga      528
   Gln Cys Glu Phe Leu Thr Ala Gln Val Thr Cys Met Arg Asn Gly Arg
                   165                 170                 175
   aga gat tct ctg tat cag aac tgg aga tgg caa cct aga gat tgt tct      576
   Arg Asp Ser Leu Tyr Gln Asn Trp Arg Trp Gln Pro Arg Asp Cys Ser
               180                 185                 190
   tta cca aag ttc aaa gcg aaa ttg ctg ttg gag aag cta agg aac aag      624
   Leu Pro Lys Phe Lys Ala Lys Leu Leu Leu Glu Lys Leu Arg Asn Lys
           195                 200                 205
   aga atg atg ttc gtt gga gat tcc cta aac cgg aac caa tgg gaa tca      672
   Arg Met Met Phe Val Gly Asp Ser Leu Asn Arg Asn Gln Trp Glu Ser
       210                 215                 220
   atg gtt tgt ttg gtt caa tca gtg gtt cct ccc ggt cga aaa agc ttg      720
   Met Val Cys Leu Val Gln Ser Val Val Pro Pro Gly Arg Lys Ser Leu
   225                 230                 235                 240
   aac aaa acc ggt tct ctc tcc gtc ttc cga gtc gag gat tat aat gcg      768
   Asn Lys Thr Gly Ser Leu Ser Val Phe Arg Val Glu Asp Tyr Asn Ala
                   245                 250                 255
   acg gtg gag ttt tat tgg gca cca ttt ttg gtg gaa tcg aat tca gat      816
   Thr Val Glu Phe Tyr Trp Ala Pro Phe Leu Val Glu Ser Asn Ser Asp
               260                 265                 270
   gat cca aat atg cat agt ata ctt aac cgt ata ata atg cct gag tcc      864
   Asp Pro Asn Met His Ser Ile Leu Asn Arg Ile Ile Met Pro Glu Ser
           275                 280                 285
   att gaa aag cat gga gtc aac tgg aaa ggc gtt gac ttt ctt gtt ttc      912
   Ile Glu Lys His Gly Val Asn Trp Lys Gly Val Asp Phe Leu Val Phe
       290                 295                 300
   aac act tac att tgg tgg atg aac aca ttc gct atg aaa gtc cta cga       960
   Asn Thr Tyr Ile Trp Trp Met Asn Thr Phe Ala Met Lys Val Leu Arg
   305                 310                 315                 320
   gga tcg ttc gat aaa gga gac acc gag tat gag gag atc gaa cgg cca      1008
   Gly Ser Phe Asp Lys Gly Asp Thr Glu Tyr Glu Glu Ile Glu Arg Pro
                   325                 330                 335
   gta gcg tat agg aga gtg atg agg act tgg gga gat tgg gtg gaa cga      1056
   Val Ala Tyr Arg Arg Val Met Arg Thr Trp Gly Asp Trp Val Glu Arg
               340                 345                 350
   aat att gat cct cta cgt acc act gtc ttc ttt gct agc atg tct cct      1104
   Asn Ile Asp Pro Leu Arg Thr Thr Val Phe Phe Ala Ser Met Ser Pro
           355                 360                 365
   ctt cac atc aag agc ttg gat tgg gag aat cca gat ggg atc aag tgt      1152
   Leu His Ile Lys Ser Leu Asp Trp Glu Asn Pro Asp Gly Ile Lys Cys
       370                 375                 380
   gca tta gag acg aca cca atc cta aac atg tca atg cca ttc agc gta      1200
   Ala Leu Glu Thr Thr Pro Ile Leu Asn Met Ser Met Pro Phe Ser Val
   385                 390                 395                 400
   gga aca gac tac aga ctg ttt tca gta gcg gaa aac gtc acg cat tct      1248
   Gly Thr Asp Tyr Arg Leu Phe Ser Val Ala Glu Asn Val Thr His Ser
                   405                 410                 415
   ctt aat gtt ccg gtt tac ttt ctc aac att acc aaa ctc tcc gaa tac      1296
   Leu Asn Val Pro Val Tyr Phe Leu Asn Ile Thr Lys Leu Ser Glu Tyr
               420                 425                 430
   cgg aaa gat gct cac act tcg gtt cac aca atc cga caa ggc aaa atg      1344
   Arg Lys Asp Ala His Thr Ser Val His Thr Ile Arg Gln Gly Lys Met
           435                 440                 445
   ctg acg ccg gag caa caa gcc gat ccc aac act tac gcc gat tgt atc      1392
   Leu Thr Pro Glu Gln Gln Ala Asp Pro Asn Thr Tyr Ala Asp Cys Ile
       450                 455                 460
   cat tgg tgt ctt ccc ggt tta cct gac acg tgg aat gag ttc ctt tac      1440
   His Trp Cys Leu Pro Gly Leu Pro Asp Thr Trp Asn Glu Phe Leu Tyr
   465                 470                 475                 480
   aca cgt atc att tcc cgt tcg tga                                      1464
   Thr Arg Ile Ile Ser Arg Ser
                   485
<210> 54
   <211> 487
   <212> PRT
   <213> Arabidopsis thaliana
<400> 54
   Met Gln Pro Trp Arg Arg Lys Phe Pro Leu Phe Glu Thr Gly Val Thr
   1               5                   10                  15
   Met Lys Gln Arg Lys Asn Ser Asn Leu Ser Ile Phe Val Val Val Phe
               20                  25                  30
   Ser Val Phe Leu Phe Gly Ile Phe Met Tyr Asn Glu Asp Val Lys Ser
           35                  40                  45
   Ile Ala Glu Phe Pro Phe Ser Thr Ser Lys Pro His Asp Val His Asp
       50                  55                  60
   Glu Ala Thr Pro Ile Thr Glu Ile Thr Thr Leu Pro Val Gln Glu Ser
   65                  70                  75                  80
   Ile Lys Asn Ser Asp Pro Ile Gln Glu Ser Ile Lys Asn Ala Asp Ser
                   85                  90                  95
   Val Gln Asp Ser Val Lys Asp Val Ala Glu Pro Val Gln Glu Glu Val
               100                 105                 110
   Ser Lys Thr Glu Glu Val Lys Lys Ile Glu Leu Phe Ala Ala Thr Glu
           115                 120                 125
   Asp Glu Glu Asp Val Glu Leu Pro Pro Glu Glu Cys Asp Leu Phe Thr
       130                 135                 140
   Gly Glu Trp Val Phe Asp Asn Glu Thr His Pro Leu Tyr Lys Glu Asp
   145                 150                 155                 160
   Gln Cys Glu Phe Leu Thr Ala Gln Val Thr Cys Met Arg Asn Gly Arg
                   165                 170                 175
   Arg Asp Ser Leu Tyr Gln Asn Trp Arg Trp Gln Pro Arg Asp Cys Ser
               180                 185                 190
   Leu Pro Lys Phe Lys Ala Lys Leu Leu Leu Glu Lys Leu Arg Asn Lys
           195                 200                 205
   Arg Met Met Phe Val Gly Asp Ser Leu Asn Arg Asn Gln Trp Glu Ser
       210                 215                 220
   Met Val Cys Leu Val Gln Ser Val Val Pro Pro Gly Arg Lys Ser Leu
   225                 230                 235                 240
   Asn Lys Thr Gly Ser Leu Ser Val Phe Arg Val Glu Asp Tyr Asn Ala
                   245                 250                 255
   Thr Val Glu Phe Tyr Trp Ala Pro Phe Leu Val Glu Ser Asn Ser Asp
               260                 265                 270
   Asp Pro Asn Met His Ser Ile Leu Asn Arg Ile Ile Met Pro Glu Ser
           275                 280                 285
   Ile Glu Lys His Gly Val Asn Trp Lys Gly Val Asp Phe Leu Val Phe
       290                 295                 300
   Asn Thr Tyr Ile Trp Trp Met Asn Thr Phe Ala Met Lys Val Leu Arg
   305                 310                 315                 320
   Gly Ser Phe Asp Lys Gly Asp Thr Glu Tyr Glu Glu Ile Glu Arg Pro
                   325                 330                 335
   Val Ala Tyr Arg Arg Val Met Arg Thr Trp Gly Asp Trp Val Glu Arg
               340                 345                 350
   Asn Ile Asp Pro Leu Arg Thr Thr Val Phe Phe Ala Ser Met Ser Pro
           355                 360                 365
   Leu His Ile Lys Ser Leu Asp Trp Glu Asn Pro Asp Gly Ile Lys Cys
       370                 375                 380
   Ala Leu Glu Thr Thr Pro Ile Leu Asn Met Ser Met Pro Phe Ser Val
   385                 390                 395                 400
   Gly Thr Asp Tyr Arg Leu Phe Ser Val Ala Glu Asn Val Thr His Ser
                   405                 410                 415
   Leu Asn Val Pro Val Tyr Phe Leu Asn Ile Thr Lys Leu Ser Glu Tyr
               420                 425                 430
   Arg Lys Asp Ala His Thr Ser Val His Thr Ile Arg Gln Gly Lys Met
           435                 440                 445
   Leu Thr Pro Glu Gln Gln Ala Asp Pro Asn Thr Tyr Ala Asp Cys Ile
       450                 455                 460
   His Trp Cys Leu Pro Gly Leu Pro Asp Thr Trp Asn Glu Phe Leu Tyr
   465                 470                 475                 480
   Thr Arg Ile Ile Ser Arg Ser
                   485
<210> 55
   <211> 759
   <212> DNA
   <213> Arabidopsis thaliana
<400> 55
   "000"
<210> 56
   <211> 252
   <212> PRT
   <213> Arabidopsis thaliana
<400> 56
   "000"
<210> 57
   <211> 867
   <212> DNA
   <213> Arabidopsis thaliana
<400> 57
   "000"
<210> 58
   <211> 288
   <212> PRT
   <213> Arabidopsis thaliana
<400> 58
   "000"
<210> 59
   <211> 1500
   <212> DNA
   <213> Arabidopsis thaliana
<400> 59
   "000"
<210> 60
   <211> 499
   <212> PRT
   <213> Arabidopsis thaliana
<400> 60
   "000"
<210> 61
   <211> 513
   <212> DNA
   <213> Arabidopsis thaliana
<400> 61
   "000"
<210> 62
   <211> 170
   <212> PRT
   <213> Arabidopsis thaliana
<400> 62
   "000"
<210> 63
   <211> 1296
   <212> DNA
   <213> Arabidopsis thaliana
<400> 63
   "000"
<210> 64
   <211> 431
   <212> PRT
   <213> Arabidopsis thaliana
<400> 64
   "000"
<210> 65
   <211> 3165
   <212> DNA
   <213> Arabidopsis thaliana
<400> 65
   "000"
<210> 66
   <211> 1054
   <212> PRT
   <213> Arabidopsis thaliana
<400> 66
   "000"
<210> 67
   <211> 1434
   <212> DNA
   <213> Arabidopsis thaliana
<400> 67
   "000"
<210> 68
   <211> 477
   <212> PRT
   <213> Arabidopsis thaliana
<400> 68
   "000"
<210> 69
   <211> 525
   <212> DNA
   <213> Arabidopsis thaliana
<400> 69
   "000"
<210> 70
   <211> 174
   <212> PRT
   <213> Arabidopsis thaliana
<400> 70
   "000"
<210> 71
   <211> 1596
   <212> DNA
   <213> Arabidopsis thaliana
<400> 71
   "000"
<210> 72
   <211> 531
   <212> PRT
   <213> Arabidopsis thaliana
<400> 72
   "000"
<210> 73
   <211> 1161
   <212> DNA
   <213> Arabidopsis thaliana
<400> 73
   "000"
<210> 74
   <211> 386
   <212> PRT
   <213> Arabidopsis thaliana
<400> 74
   "000"
<210> 75
   <211> 396
   <212> DNA
   <213> Arabidopsis thaliana
<400> 75
   "000"
<210> 76
   <211> 131
   <212> PRT
   <213> Arabidopsis thaliana
<400> 76
   "000"
<210> 77
   <211> 1413
   <212> DNA
   <213> Arabidopsis thaliana
<400> 77
   "000"
<210> 78
   <211> 470
   <212> PRT
   <213> Arabidopsis thaliana
<400> 78
   "000"
<210> 79
   <211> 1302
   <212> DNA
   <213> Arabidopsis thaliana
<400> 79
   "000"
<210> 80
   <211> 433
   <212> PRT
   <213> Arabidopsis thaliana
<400> 80
   "000"
<210> 81
   <211> 1083
   <212> DNA
   <213> Arabidopsis thaliana
<400> 81
   "000"
<210> 82
   <211> 360
   <212> PRT
   <213> Arabidopsis thaliana
<400> 82
   "000"
<210> 83
   <211> 21
   <212> DNA
   <213> Artificial
<220>
   <223> primer
<400> 83
   tgacgccatt tcgccttttc a 21
<210> 84
   <211> 27
   <212> DNA
   <213> Artificial
<220>
   <223> primer
<400> 84
   cagaaatgga taaatagcct tgcttcc 27
<210> 85
   <211> 21
   <212> DNA
   <213> Artificial
<220>
   <223> primer
<400> 85
   agctggcgta atagcgaaga g 21
<210> 86
   <211> 23
   <212> DNA
   <213> Artificial
<220>
   <223> primer
<400> 86
   caacttaatc gccttgcagc aca 23
<210> 87
   <211> 21
   <212> DNA
   <213> Artificial
<220>
   <223> primer
<400> 87
   agctggcgta atagcgaaga g 21
<210> 88
   <211> 23
   <212> DNA
   <213> Artificial
<220>
   <223> primer
<400> 88
   ccaatacatt acactagcat ctg 23
<210> 89
   <211> 18
   <212> DNA
   <213> Artificial
<220>
   <223> primer
<400> 89
   aatgctagag cagcttga 18
<210> 90
   <211> 22
   <212> DNA
   <213> Artificial
<220>
   <223> primer
<400> 90
   caatacatta cactagcatc tg 22
<210> 91
   <211> 18
   <212> DNA
   <213> Artificial
<220>
   <223> primer
<400> 91
   agaggcccgc accgatcg 18
<210> 92
   <211> 16
   <212> DNA
   <213> Artificial
<220>
   <223> primer
<220>
   <221> misc_feature
   <222> (5)..(5)
   <223> n is a, c, g, or t
<220>
   <221> misc_feature
   <222> (10)..(10)
   <223> n is a, c, g, or t
<220>
   <221> misc_feature
   <222> (13)..(13)
   <223> n is a, c, g, or t
<400> 92
   wgtgnagwan canaga 16
<210> 93
   <211> 16
   <212> DNA
   <213> Artificial
<220>
   <223> primer
<220>
   <221> misc_feature
   <222> (5)..(5)
   <223> n is a, c, g, or t
<220>
   <221> misc_feature
   <222> (10)..(10)
   <223> n is a, c, g, or t
<220>
   <221> misc_feature
   <222> (13)..(13)
   <223> n is a, c, g, or t
<400> 93
   agwgnagwan canaga 16
<210> 94
   <211> 16
   <212> DNA
   <213> Artificial
<220>
   <223> primer
<220>
   <221> misc_feature
   <222> (1)..(1)
   <223> n is a, c, g, or t
<220>
   <221> misc_feature
   <222> (11)..(11)
   <223> n is a, c, g, or t
<400> 94
   ntgcgaswga nwagaa 16
<210> 95
   <211> 693
   <212> DNA
   <213> Zea mays
<220>
   <221> CDS
   <222> (1)..(693)
<400> 95
   atg ctt cga ctg atc gac gat ttc gag agc gtg cca gcg ctg ctg ggc          48
   Met Leu Arg Leu Ile Asp Asp Phe Glu Ser Val Pro Ala Leu Leu Gly
   1               5                   10                  15
   tgg tgt cag tca cct gag caa gga aga gag atg gcc acg ctg cct cgc          96
   Trp Cys Gln Ser Pro Glu Gln Gly Arg Glu Met Ala Thr Leu Pro Arg
               20                  25                  30
   gag ggc gcc gcg gcg gcg gac tcc gac cag ctt ttg gta gaa gca cat          144
   Glu Gly Ala Ala Ala Ala Asp Ser Asp Gln Leu Leu Val Glu Ala His
           35                  40                  45
   ggc tct aca cga acg ctg att ttg aat agg cca aag cag ctc aat gca          192
   Gly Ser Thr Arg Thr Leu Ile Leu Asn Arg Pro Lys Gln Leu Asn Ala
       50                  55                  60
   ctc tcc tct gaa atg att aaa ggt ctc cta tgg tat ttc aca gct ttt          240
   Leu Ser Ser Glu Met Ile Lys Gly Leu Leu Trp Tyr Phe Thr Ala Phe
   65                  70                  75                  80
   gag aaa gat gat gga gtc aga ttg ttg att atg aag ggg aat aga aga          288
   Glu Lys Asp Asp Gly Val Arg Leu Leu Ile Met Lys Gly Asn Arg Arg
                   85                  90                  95
   gta ttt tgc tct gga ggt gat att gct caa tgt gcc cgg tgt tta tat          336
   Val Phe Cys Ser Gly Gly Asp Ile Ala Gln Cys Ala Arg Cys Leu Tyr
               100                 105                 110
   aat gga aac tgg aaa tgg gtc gat gat ttc ttc caa aat gaa tat ttg          384
   Asn Gly Asn Trp Lys Trp Val Asp Asp Phe Phe Gln Asn Glu Tyr Leu
           115                 120                 125
   cta aat tac atc att gcg act tac ata aaa cct cag gtt tcc att ctt          432
   Leu Asn Tyr Ile Ile Ala Thr Tyr Ile Lys Pro Gln Val Ser Ile Leu
       130                 135                 140
   agt gga att gtc atg ggt gga gga gct ggt gtt tca ata cat gga aga          480
   Ser Gly Ile Val Met Gly Gly Gly Ala Gly Val Ser Ile His Gly Arg
   145                 150                 155                 160
   ttt cga gtt gcc act gat aac acg att ttt gca atg cca gaa aca gct          528
   Phe Arg Val Ala Thr Asp Asn Thr Ile Phe Ala Met Pro Glu Thr Ala
                   165                 170                 175
   cta ggc ctc ttt cca gat gta ggg gcc tct tat ttt cta tct cgg cta          576
   Leu Gly Leu Phe Pro Asp Val Gly Ala Ser Tyr Phe Leu Ser Arg Leu
               180                 185                 190
   cca ggg ttc tat gga gaa tat gtt ggt cta act ggt gcc aga ttg gat          624
   Pro Gly Phe Tyr Gly Glu Tyr Val Gly Leu Thr Gly Ala Arg Leu Asp
           195                 200                 205
   ggt gcg gaa atg ctt gcc tgt ggt ttt ttt ttc tca ttt tgt ccc gtc          672
   Gly Ala Glu Met Leu Ala Cys Gly Phe Phe Phe Ser Phe Cys Pro Val
       210                 215                 220
   aaa gag gtt gcc att gct tga                                              693
   Lys Glu Val Ala Ile Ala
   225                 230
<210> 96
   <211> 230
   <212> PRT
   <213> Zea mays
<400> 96
   "000"
<210> 97
   <211> 603
   <212> DNA
   <213> Zea mays
<400> 97
   "000"
<210> 98
   <211> 200
   <212> PRT
   <213> Zea mays
<400> 98
   "000"
<210> 99
   <211> 849
   <212> DNA
   <213> Zea mays
<400> 99
   "000"
<210> 100
   <211> 282
   <212> PRT
   <213> Zea mays
<400> 100
   "000"
<210> 101
   <211> 741
   <212> DNA
   <213> Zea mays
<400> 101
   "000"
<210> 102
   <211> 246
   <212> PRT
   <213> Zea mays
<400> 102
   "000"
<210> 103
   <211> 1401
   <212> DNA
   <213> Zea mays
<400> 103
   "000"
<210> 104
   <211> 466
   <212> PRT
   <213> Zea mays
<400> 104
   "000"
<210> 105
   <211> 993
   <212> DNA
   <213> Zea mays
<400> 105
   "000"
<210> 106
   <211> 330
   <212> PRT
   <213> Zea mays
<400> 106
   "000"
<210> 107
   <211> 795
   <212> DNA
   <213> Zea mays
<400> 107
   "000"
<210> 108
   <211> 264
   <212> PRT
   <213> Zea mays
<400> 108
   "000"
<210> 109
   <211> 741
   <212> DNA
   <213> Zea mays
<400> 109
   "000"
<210> 110
   <211> 246
   <212> PRT
   <213> Zea mays
<400> 110
   "000"
<210> 111
   <211> 1173
   <212> DNA
   <213> Zea mays
<400> 111
   "000"
<210> 112
   <211> 390
   <212> PRT
   <213> Zea mays
<400> 112
   "000"

<210> 113
   <211> 444
   <212> DNA
   <213> Zea mays
<400> 113
   "000"
<210> 114
   <211> 147
   <212> PRT
   <213> Zea mays
<400> 114
   "000"
<210> 115
   <211> 876
   <212> DNA
   <213> Zea mays
<400> 115
   "000"
<210> 116
   <211> 291
   <212> PRT
   <213> Zea mays
<400> 116
   "000"
<210> 117
   <211> 1332
   <212> DNA
   <213> Zea mays
<400> 117
   "000"
<210> 118
   <211> 443
   <212> PRT
   <213> Zea mays
<400> 118
   "000"
<210> 119
   <211> 1002
   <212> DNA
   <213> Zea mays
<400> 119
   "000"
<210> 120
   <211> 333
   <212> PRT
   <213> Zea mays
<400> 120
   "000"
<210> 121
   <211> 1326
   <212> DNA
   <213> Zea mays
<400> 121
   "000"
<210> 122
   <211> 441
   <212> PRT
   <213> Zea mays
<400> 122
   "000"
<210> 123
   <211> 963
   <212> DNA
   <213> Zea mays
<400> 123
   "000"
<210> 124
   <211> 320
   <212> PRT
   <213> Zea mays
<400> 124
   "000"
<210> 125
   <211> 861
   <212> DNA
   <213> Zea mays
<400> 125
   "000"
<210> 126
   <211> 286
   <212> PRT
   <213> Zea mays
<400> 126
   "000"
<210> 127
   <211> 915
   <212> DNA
   <213> Zea mays
<400> 127
   "000"
<210> 128
   <211> 304
   <212> PRT
   <213> Zea mays
<400> 128
   "000"
<210> 129
   <211> 4590
   <212> DNA
   <213> Zea mays
<400> 129
   "000"
<210> 130
   <211> 1529
   <212> PRT
   <213> Zea mays
<400> 130
   "000"
<210> 131
   <211> 1701
   <212> DNA
   <213> Zea mays
<400> 131
   "000"
<210> 132
   <211> 566
   <212> PRT
   <213> Zea mays
<400> 132
   "000"
<210> 133
   <211> 852
   <212> DNA
   <213> Zea mays
<400> 133
   "000"
<210> 134
   <211> 283
   <212> PRT
   <213> Zea mays
<400> 134
   "000"
<210> 135
   <211> 342
   <212> DNA
   <213> Zea mays
<400> 135
   "000"
<210> 136
   <211> 113
   <212> PRT
   <213> Zea mays
<400> 136
   "000"
<210> 137
   <211> 867
   <212> DNA
   <213> Zea mays
<400> 137
   "000"
<210> 138
   <211> 288
   <212> PRT
   <213> Zea mays
<400> 138
   "000"
<210> 139
   <211> 414
   <212> DNA
   <213> Zea mays
<400> 139
   "000"
<210> 140
   <211> 137
   <212> PRT
   <213> Zea mays
<400> 140
   "000"
<210> 141
   <211> 423
   <212> DNA
   <213> Zea mays
<400> 141
   "000"
<210> 142
   <211> 140
   <212> PRT
   <213> Zea mays
<400> 142
   "000"
<210> 143
   <211> 426
   <212> DNA
   <213> Zea mays
<400> 143
   "000"
<210> 144
   <211> 141
   <212> PRT
   <213> Zea mays
<400> 144
   "000"
<210> 145
   <211> 1029
   <212> DNA
   <213> Zea mays
<400> 145
   "000"
<210> 146
   <211> 342
   <212> PRT
   <213> Zea mays
<400> 146
   "000"
<210> 147
   <211> 1125
   <212> DNA
   <213> Zea mays
<400> 147
   "000"
<210> 148
   <211> 374
   <212> PRT
   <213> Zea mays
<400> 148
   "000"
<210> 149
   <211> 966
   <212> DNA
   <213> Zea mays
<400> 149
   "000"
<210> 150
   <211> 321
   <212> PRT
   <213> Zea mays
<400> 150
   "000"
<210> 151
   <211> 1077
   <212> DNA
   <213> Zea mays
<400> 151
   "000"
<210> 152
   <211> 358
   <212> PRT
   <213> Zea mays
<400> 152
   "000"
<210> 153
   <211> 1095
   <212> DNA
   <213> Zea mays
<400> 153
   "000"
<210> 154
   <211> 364
   <212> PRT
   <213> Zea mays
<400> 154
   "000"
<210> 155
   <211> 837
   <212> DNA
   <213> Zea mays
<400> 155
   "000"
<210> 156
   <211> 278
   <212> PRT
   <213> Zea mays
<400> 156
   "000"
<210> 157
   <211> 570
   <212> DNA
   <213> Zea mays
<400> 157
   "000"
<210> 158
   <211> 189
   <212> PRT
   <213> Zea mays
<400> 158
   "000"
<210> 159
   <211> 765
   <212> DNA
   <213> Zea mays
<400> 159
   "000"
<210> 160
   <211> 254
   <212> PRT
   <213> Zea mays
<400> 160
   "000"
<210> 161
   <211> 1164
   <212> DNA
   <213> Zea mays
<400> 161
   "000"
<210> 162
   <211> 387
   <212> PRT
   <213> Zea mays
<400> 162
   "000"
<210> 163
   <211> 687
   <212> DNA
   <213> Zea mays
<400> 163
   "000"
<210> 164
   <211> 228
   <212> PRT
   <213> Zea mays
<400> 164
   "000"
<210> 165
   <211> 1134
   <212> DNA
   <213> Zea mays
<400> 165
   "000"
<210> 166
   <211> 377
   <212> PRT
   <213> Zea mays
<400> 166
   "000"
<210> 167
   <211> 840
   <212> DNA
   <213> Zea mays
<400> 167
   "000"
<210> 168
   <211> 279
   <212> PRT
   <213> Zea mays
<400> 168
   "000"
<210> 169
   <211> 1242
   <212> DNA
   <213> Zea mays
<400> 169
   "000"
<210> 170
   <211> 413
   <212> PRT
   <213> Zea mays
<400> 170
   "000"
<210> 171
   <211> 3300
   <212> DNA
   <213> Zea mays
<400> 171
   "000"
<210> 172
   <211> 1099
   <212> PRT
   <213> Zea mays
<400> 172
   "000"
<210> 173
   <211> 3303
   <212> DNA
   <213> Zea mays
<400> 173
   "000"
<210> 174
   <211> 1100
   <212> PRT
   <213> Zea mays
<400> 174
   "000"
<210> 175
   <211> 885
   <212> DNA
   <213> Zea mays
<400> 175
   "000"
<210> 176
   <211> 294
   <212> PRT
   <213> Zea mays
<400> 176
   "000"
<210> 177
   <211> 642
   <212> DNA
   <213> Zea mays
<400> 177
   "000"
<210> 178
   <211> 213
   <212> PRT
   <213> Zea mays
<400> 178
   "000"
<210> 179
   <211> 450
   <212> DNA
   <213> Zea mays
<400> 179
   "000"
<210> 180
   <211> 149
   <212> PRT
   <213> Zea mays
<400> 180
   "000"
<210> 181
   <211> 822
   <212> DNA
   <213> Zea mays
<400> 181
   "000"
<210> 182
   <211> 273
   <212> PRT
   <213> Zea mays
<400> 182
   "000"
<210> 183
   <211> 843
   <212> DNA
   <213> Zea mays
<400> 183
   "000"
<210> 184
   <211> 280
   <212> PRT
   <213> Zea mays
<400> 184
   "000"
<210> 185
   <211> 519
   <212> DNA
   <213> Zea mays
<400> 185
   "000"
<210> 186
   <211> 172
   <212> PRT
   <213> Zea mays
<400> 186
   "000"
<210> 187
   <211> 570
   <212> DNA
   <213> Zea mays
<400> 187
   "000"
<210> 188
   <211> 189
   <212> PRT
   <213> Zea mays
<400> 188
   "000"
<210> 189
   <211> 1620
   <212> DNA
   <213> Zea mays
<400> 189
   "000"
<210> 190
   <211> 539
   <212> PRT
   <213> Zea mays
<400> 190
   "000"
<210> 191
   <211> 795
   <212> DNA
   <213> Zea mays
<400> 191
   "000"
<210> 192
   <211> 264
   <212> PRT
   <213> Zea mays
<400> 192
   "000"
<210> 193
   <211> 1080
   <212> DNA
   <213> Zea mays
<400> 193
   "000"
<210> 194
   <211> 359
   <212> PRT
   <213> Zea mays
<400> 194
   "000"
<210> 195
   <211> 1077
   <212> DNA
   <213> Zea mays
<400> 195
   "000"
<210> 196
   <211> 358
   <212> PRT
   <213> Zea mays
<400> 196
   "000"
<210> 197
   <211> 1485
   <212> DNA
   <213> Zea mays
<400> 197
   "000"
<210> 198
   <211> 494
   <212> PRT
   <213> Zea mays
<400> 198
   "000"
<210> 199
   <211> 1041
   <212> DNA
   <213> Zea mays
<400> 199
   "000"
<210> 200
   <211> 346
   <212> PRT
   <213> Zea mays
<400> 200
   "000"
<210> 201
   <211> 612
   <212> DNA
   <213> Zea mays
<400> 201
   "000"
<210> 202
   <211> 203
   <212> PRT
   <213> Zea mays
<400> 202
   "000"
<210> 203
   <211> 810
   <212> DNA
   <213> Zea mays
<400> 203
   "000"
<210> 204
   <211> 269
   <212> PRT
   <213> Zea mays
<400> 204
   "000"
<210> 205
   <211> 1701
   <212> DNA
   <213> Zea mays
<400> 205
   "000"
<210> 206
   <211> 566
   <212> PRT
   <213> Zea mays
<400> 206
   "000"
<210> 207
   <211> 1452
   <212> DNA
   <213> Zea mays
<400> 207
   "000"
<210> 208
   <211> 483
   <212> PRT
   <213> Zea mays
<400> 208
   "000"
<210> 209
   <211> 1173
   <212> DNA
   <213> Zea mays
<400> 209
   "000"
<210> 210
   <211> 390
   <212> PRT
   <213> Zea mays
<400> 210
   "000"
<210> 211
   <211> 777
   <212> DNA
   <213> Zea mays
<400> 211
   "000"
<210> 212
   <211> 258
   <212> PRT
   <213> Zea mays
<400> 212
   "000"
<210> 213
   <211> 768
   <212> DNA
   <213> Zea mays
<400> 213
   "000"
<210> 214
   <211> 255
   <212> PRT
   <213> Zea mays
<400> 214
   "000"
<210> 215
   <211> 1572
   <212> DNA
   <213> Zea mays
<400> 215
   "000"
<210> 216
   <211> 523
   <212> PRT
   <213> Zea mays
<400> 216
   "000"
<210> 217
   <211> 1218
   <212> DNA
   <213> Zea mays
<400> 217
   "000"
<210> 218
   <211> 405
   <212> PRT
   <213> Zea mays
<400> 218
   "000"
<210> 219
   <211> 882
   <212> DNA
   <213> Zea mays
<400> 219
   "000"
<210> 220
   <211> 293
   <212> PRT
   <213> Zea mays
<400> 220
   "000"
<210> 221
   <211> 1350
   <212> DNA
   <213> Zea mays
<400> 221
   "000"
<210> 222
   <211> 449
   <212> PRT
   <213> Zea mays
<400> 222
   "000"
<210> 223
   <211> 861
   <212> DNA
   <213> Zea mays
<400> 223
   "000"
<210> 224
   <211> 286
   <212> PRT
   <213> Zea mays
<400> 224
   "000"
<210> 225
   <211> 912
   <212> DNA
   <213> Zea mays
<400> 225
   "000"
<210> 226
   <211> 303
   <212> PRT
   <213> Zea mays
<400> 226
   "000"
<210> 227
   <211> 744
   <212> DNA
   <213> Zea mays
<400> 227
   "000"
<210> 228
   <211> 247
   <212> PRT
   <213> Zea mays
<400> 228
   "000"
<210> 229
   <211> 774
   <212> DNA
   <213> Zea mays
<400> 229
   "000"
<210> 230
   <211> 257
   <212> PRT
   <213> Zea mays
<400> 230
   "000"
<210> 231
   <211> 1362
   <212> DNA
   <213> Zea mays
<400> 231
   "000"
<210> 232
   <211> 453
   <212> PRT
   <213> Zea mays
<400> 232
   "000"
<210> 233
   <211> 891
   <212> DNA
   <213> Zea mays
<400> 233
   "000"
<210> 234
   <211> 296
   <212> PRT
   <213> Zea mays
<400> 234
   "000"
<210> 235
   <211> 783
   <212> DNA
   <213> Zea mays
<400> 235
   "000"
<210> 236
   <211> 260
   <212> PRT
   <213> Zea mays
<400> 236
   "000"
<210> 237
   <211> 684
   <212> DNA
   <213> Zea mays
<400> 237
   "000"
<210> 238
   <211> 227
   <212> PRT
   <213> Zea mays
<400> 238
   "000"
<210> 239
   <211> 756
   <212> DNA
   <213> Zea mays
<400> 239
   "000"
<210> 240
   <211> 251
   <212> PRT
   <213> Zea mays
<400> 240
   "000"
<210> 241
   <211> 606
   <212> DNA
   <213> Zea mays
<400> 241
   "000"
<210> 242
   <211> 201
   <212> PRT
   <213> Zea mays
<400> 242
   "000"
<210> 243
   <211> 1203
   <212> DNA
   <213> Zea mays
<400> 243
   "000"
<210> 244
   <211> 400
   <212> PRT
   <213> Zea mays
<400> 244
   "000"
<210> 245
   <211> 1278
   <212> DNA
   <213> Zea mays
<400> 245
   "000"
<210> 246
   <211> 425
   <212> PRT
   <213> Zea mays
<400> 246
   "000"
<210> 247
   <211> 714
   <212> DNA
   <213> Zea mays
<400> 247
   "000"
<210> 248
   <211> 237
   <212> PRT
   <213> Zea mays
<400> 248
   "000"
<210> 249
   <211> 849
   <212> DNA
   <213> Zea mays
<400> 249
   "000"
<210> 250
   <211> 282
   <212> PRT
   <213> Zea mays
<400> 250
   "000"
<210> 251
   <211> 1557
   <212> DNA
   <213> Zea mays
<400> 251
   "000"
<210> 252
   <211> 518
   <212> PRT
   <213> Zea mays
<400> 252
   "000"
<210> 253
   <211> 981
   <212> DNA
   <213> Zea mays
<400> 253
   "000"
<210> 254
   <211> 326
   <212> PRT
   <213> Zea mays
<400> 254
   "000"
<210> 255
   <211> 546
   <212> DNA
   <213> Zea mays
<400> 255
   "000"
<210> 256
   <211> 181
   <212> PRT
   <213> Zea mays
<400> 256
   "000"
<210> 257
   <211> 438
   <212> DNA
   <213> Zea mays
<400> 257
   "000"
<210> 258
   <211> 145
   <212> PRT
   <213> Zea mays
<400> 258
   "000"
<210> 259
   <211> 4590
   <212> DNA
   <213> Zea mays
<400> 259
   "000"
<210> 260
   <211> 1529
   <212> PRT
   <213> Zea mays
<400> 260
   "000"
<210> 261
   <211> 1692
   <212> DNA
   <213> Zea mays
<400> 261
   "000"
<210> 262
   <211> 563
   <212> PRT
   <213> Zea mays
<400> 262
   "000"
<210> 263
   <211> 633
   <212> DNA
   <213> Zea mays
<400> 263
   "000"
<210> 264
   <211> 210
   <212> PRT
   <213> Zea mays
<400> 264
   "000"
<210> 265
   <211> 1263
   <212> DNA
   <213> Zea mays
<400> 265
   "000"
<210> 266
   <211> 420
   <212> PRT
   <213> Zea mays
<400> 266
   "000"
<210> 267
   <211> 1002
   <212> DNA
   <213> Zea mays
<400> 267
   "000"
<210> 268
   <211> 333
   <212> PRT
   <213> Zea mays
<400> 268
   "000"
<210> 269
   <211> 744
   <212> DNA
   <213> Zea mays
<400> 269
   "000"
<210> 270
   <211> 247
   <212> PRT
   <213> Zea mays
<400> 270
   "000"
<210> 271
   <211> 618
   <212> DNA
   <213> Zea mays
<400> 271
   "000"
<210> 272
   <211> 205
   <212> PRT
   <213> Zea mays
<400> 272
   "000"

<210> 273
   <211> 579
   <212> DNA
   <213> Zea mays
<400> 273
   "000"
<210> 274
   <211> 192
   <212> PRT
   <213> Zea mays
<400> 274
   "000"
<210> 275
   <211> 639
   <212> DNA
   <213> Zea mays
<400> 275
   "000"
<210> 276
   <211> 212
   <212> PRT
   <213> Zea mays
<400> 276
   "000"
<210> 277
   <211> 846
   <212> DNA
   <213> Zea mays
<400> 277
   "000"
<210> 278
   <211> 281
   <212> PRT
   <213> Zea mays
<400> 278
   "000"
<210> 279
   <211> 879
   <212> DNA
   <213> Zea mays
<400> 279
   "000"
<210> 280
   <211> 292
   <212> PRT
   <213> Zea mays
<400> 280
   "000"
<210> 281
   <211> 567
   <212> DNA
   <213> Zea mays
<400> 281
   "000"
<210> 282
   <211> 188
   <212> PRT
   <213> Zea mays
<400> 282
   "000"
<210> 283
   <211> 492
   <212> DNA
   <213> Zea mays
<400> 283
   "000"
<210> 284
   <211> 163
   <212> PRT
   <213> Zea mays
<400> 284
   "000"
<210> 285
   <211> 2016
   <212> DNA
   <213> Zea mays
<400> 285
   "000"
<210> 286
   <211> 671
   <212> PRT
   <213> Zea mays
<400> 286
   "000"
<210> 287
   <211> 1173
   <212> DNA
   <213> Zea mays
<400> 287
   "000"
<210> 288
   <211> 390
   <212> PRT
   <213> Zea mays
<400> 288
   "000"
<210> 289
   <211> 1323
   <212> DNA
   <213> Zea mays
<400> 289
   "000"
<210> 290
   <211> 440
   <212> PRT
   <213> Zea mays
<400> 290
   "000"
<210> 291
   <211> 918
   <212> DNA
   <213> Zea mays
<400> 291
   "000"
<210> 292
   <211> 305
   <212> PRT
   <213> Zea mays
<400> 292
   "000"
<210> 293
   <211> 477
   <212> DNA
   <213> Zea mays
<400> 293
   "000"
<210> 294
   <211> 158
   <212> PRT
   <213> Zea mays
<400> 294
   "000"
<210> 295
   <211> 696
   <212> DNA
   <213> Zea mays
<400> 295
   "000"
<210> 296
   <211> 231
   <212> PRT
   <213> Zea mays
<400> 296
   "000"
<210> 297
   <211> 414
   <212> DNA
   <213> Zea mays
<400> 297
   "000"
<210> 298
   <211> 137
   <212> PRT
   <213> Zea mays
<400> 298
   "000"
<210> 299
   <211> 1701
   <212> DNA
   <213> Zea mays
<400> 299
   "000"
<210> 300
   <211> 566
   <212> PRT
   <213> Zea mays
<400> 300
   "000"
<210> 301
   <211> 396
   <212> DNA
   <213> Zea mays
<400> 301
   "000"
<210> 302
   <211> 131
   <212> PRT
   <213> Zea mays
<400> 302
   "000"
<210> 303
   <211> 522
   <212> DNA
   <213> Zea mays
<400> 303
   "000"
<210> 304
   <211> 173
   <212> PRT
   <213> Zea mays
<400> 304
   "000"
<210> 305
   <211> 360
   <212> DNA
   <213> Zea mays
<400> 305
   "000"
<210> 306
   <211> 119
   <212> PRT
   <213> Zea mays
<400> 306
   "000"
<210> 307
   <211> 666
   <212> DNA
   <213> Zea mays
<400> 307
   "000"
<210> 308
   <211> 221
   <212> PRT
   <213> Zea mays
<400> 308
   "000"
<210> 309
   <211> 1110
   <212> DNA
   <213> Zea mays
<400> 309
   "000"
<210> 310
   <211> 369
   <212> PRT
   <213> Zea mays
<400> 310
   "000"
<210> 311
   <211> 900
   <212> DNA
   <213> Zea mays
<400> 311
   "000"
<210> 312
   <211> 299
   <212> PRT
   <213> Zea mays
<400> 312
   "000"
<210> 313
   <211> 1341
   <212> DNA
   <213> Zea mays
<400> 313
   "000"
<210> 314
   <211> 446
   <212> PRT
   <213> Zea mays
<400> 314
   "000"
<210> 315
   <211> 306
   <212> DNA
   <213> Zea mays
<400> 315
   "000"
<210> 316
   <211> 101
   <212> PRT
   <213> Zea mays
<400> 316
   "000"
<210> 317
   <211> 828
   <212> DNA
   <213> Zea mays
<400> 317
   "000"
<210> 318
   <211> 275
   <212> PRT
   <213> Zea mays
<400> 318
   "000"
<210> 319
   <211> 1164
   <212> DNA
   <213> Zea mays
<400> 319
   "000"
<210> 320
   <211> 387
   <212> PRT
   <213> Zea mays
<400> 320
   "000"
<210> 321
   <211> 1068
   <212> DNA
   <213> Zea mays
<400> 321
   "000"
<210> 322
   <211> 355
   <212> PRT
   <213> Zea mays
<400> 322
   "000"
<210> 323
   <211> 2190
   <212> DNA
   <213> Zea mays
<400> 323
   "000"
<210> 324
   <211> 729
   <212> PRT
   <213> Zea mays
<400> 324
   "000"
<210> 325
   <211> 1122
   <212> DNA
   <213> Zea mays
<400> 325
   "000"
<210> 326
   <211> 373
   <212> PRT
   <213> Zea mays
<400> 326
   "000"
<210> 327
   <211> 777
   <212> DNA
   <213> Zea mays
<400> 327
   "000"
<210> 328
   <211> 258
   <212> PRT
   <213> Zea mays
<400> 328
   "000"
<210> 329
   <211> 1701
   <212> DNA
   <213> Zea mays
<400> 329
   "000"
<210> 330
   <211> 566
   <212> PRT
   <213> Zea mays
<400> 330
   "000"
<210> 331
   <211> 867
   <212> DNA
   <213> Zea mays
<400> 331
   "000"
<210> 332
   <211> 288
   <212> PRT
   <213> Zea mays
<400> 332
   "000"
<210> 333
   <211> 627
   <212> DNA
   <213> Zea mays
<400> 333
   "000"
<210> 334
   <211> 208
   <212> PRT
   <213> Zea mays
<400> 334
   "000"
<210> 335
   <211> 816
   <212> DNA
   <213> Zea mays
<400> 335
   "000"
<210> 336
   <211> 271
   <212> PRT
   <213> Zea mays
<400> 336
   "000"
<210> 337
   <211> 921
   <212> DNA
   <213> Zea mays
<400> 337
   "000"
<210> 338
   <211> 306
   <212> PRT
   <213> Zea mays
<400> 338
   "000"
<210> 339
   <211> 1164
   <212> DNA
   <213> Zea mays
<400> 339
   "000"
<210> 340
   <211> 387
   <212> PRT
   <213> Zea mays
<400> 340
   "000"
<210> 341
   <211> 777
   <212> DNA
   <213> Zea mays
<400> 341
   "000"
<210> 342
   <211> 258
   <212> PRT
   <213> Zea mays
<400> 342
   "000"
<210> 343
   <211> 585
   <212> DNA
   <213> Zea mays
<400> 343
   "000"
<210> 344
   <211> 194
   <212> PRT
   <213> Zea mays
<400> 344
   "000"
<210> 345
   <211> 816
   <212> DNA
   <213> Zea mays
<400> 345
   "000"
<210> 346
   <211> 271
   <212> PRT
   <213> Zea mays
<400> 346
   "000"
<210> 347
   <211> 1314
   <212> DNA
   <213> Zea mays
<400> 347
   "000"
<210> 348
   <211> 437
   <212> PRT
   <213> Zea mays
<400> 348
   "000"
<210> 349
   <211> 1056
   <212> DNA
   <213> Zea mays
<400> 349
   "000"
<210> 350
   <211> 351
   <212> PRT
   <213> Zea mays
<400> 350
   "000"
<210> 351
   <211> 720
   <212> DNA
   <213> Zea mays
<400> 351
   "000"
<210> 352
   <211> 239
   <212> PRT
   <213> Zea mays
<400> 352
   "000"
<210> 353
   <211> 1350
   <212> DNA
   <213> Zea mays
<400> 353
   "000"
<210> 354
   <211> 449
   <212> PRT
   <213> Zea mays
<400> 354
   "000"
<210> 355
   <211> 756
   <212> DNA
   <213> Zea mays
<400> 355
   "000"
<210> 356
   <211> 251
   <212> PRT
   <213> Zea mays
<400> 356
   "000"
<210> 357
   <211> 780
   <212> DNA
   <213> Zea mays
<400> 357
   "000"
<210> 358
   <211> 259
   <212> PRT
   <213> Zea mays
<400> 358
   "000"
<210> 359
   <211> 747
   <212> DNA
   <213> Zea mays
<400> 359
   "000"
<210> 360
   <211> 248
   <212> PRT
   <213> Zea mays
<400> 360
   "000"
<210> 361
   <211> 780
   <212> DNA
   <213> Zea mays
<400> 361
   "000"
<210> 362
   <211> 259
   <212> PRT
   <213> Zea mays
<400> 362
   "000"
<210> 363
   <211> 1008
   <212> DNA
   <213> Zea mays
<400> 363
   "000"
<210> 364
   <211> 335
   <212> PRT
   <213> Zea mays
<400> 364
   "000"
<210> 365
   <211> 441
   <212> DNA
   <213> Zea mays
<400> 365
   "000"
<210> 366
   <211> 146
   <212> PRT
   <213> Zea mays
<400> 366
   "000"
<210> 367
   <211> 858
   <212> DNA
   <213> Zea mays
<400> 367
   "000"
<210> 368
   <211> 285
   <212> PRT
   <213> Zea mays
<400> 368
   "000"
<210> 369
   <211> 846
   <212> DNA
   <213> Zea mays
<400> 369
   "000"
<210> 370
   <211> 281
   <212> PRT
   <213> Zea mays
<400> 370
   "000"
<210> 371
   <211> 849
   <212> DNA
   <213> Zea mays
<400> 371
   "000"
<210> 372
   <211> 282
   <212> PRT
   <213> Zea mays
<400> 372
   "000"
<210> 373
   <211> 1122
   <212> DNA
   <213> Zea mays
<400> 373
   "000"
<210> 374
   <211> 373
   <212> PRT
   <213> Zea mays
<400> 374
   "000"
<210> 375
   <211> 462
   <212> DNA
   <213> Zea mays
<400> 375
   "000"
<210> 376
   <211> 153
   <212> PRT
   <213> Zea mays
<400> 376
   "000"
<210> 377
   <211> 690
   <212> DNA
   <213> Zea mays
<400> 377
   "000"
<210> 378
   <211> 229
   <212> PRT
   <213> Zea mays
<400> 378
   "000"
<210> 379
   <211> 618
   <212> DNA
   <213> Zea mays
<400> 379
   "000"
<210> 380
   <211> 205
   <212> PRT
   <213> Zea mays
<400> 380
   "000"
<210> 381
   <211> 1692
   <212> DNA
   <213> Zea mays
<400> 381
   "000"
<210> 382
   <211> 563
   <212> PRT
   <213> Zea mays
<400> 382
   "000"
<210> 383
   <211> 675
   <212> DNA
   <213> Zea mays
<400> 383
   "000"
<210> 384
   <211> 224
   <212> PRT
   <213> Zea mays
<400> 384
   "000"
<210> 385
   <211> 798
   <212> DNA
   <213> Zea mays
<400> 385
   "000"
<210> 386
   <211> 265
   <212> PRT
   <213> Zea mays
<400> 386
   "000"
<210> 387
   <211> 714
   <212> DNA
   <213> Zea mays
<400> 387
   "000"
<210> 388
   <211> 237
   <212> PRT
   <213> Zea mays
<400> 388
   "000"
<210> 389
   <211> 1350
   <212> DNA
   <213> Zea mays
<400> 389
   "000"
<210> 390
   <211> 449
   <212> PRT
   <213> Zea mays
<400> 390
   "000"
<210> 391
   <211> 741
   <212> DNA
   <213> Zea mays
<400> 391
   "000"
<210> 392
   <211> 246
   <212> PRT
   <213> Zea mays
<400> 392
   "000"
<210> 393
   <211> 456
   <212> DNA
   <213> Zea mays
<400> 393
   "000"
<210> 394
   <211> 151
   <212> PRT
   <213> Zea mays
<400> 394
   "000"
<210> 395
   <211> 630
   <212> DNA
   <213> Zea mays
<400> 395
   "000"
<210> 396
   <211> 209
   <212> PRT
   <213> Zea mays
<400> 396
   "000"
<210> 397
   <211> 795
   <212> DNA
   <213> Zea mays
<400> 397
   "000"
<210> 398
   <211> 264
   <212> PRT
   <213> Zea mays
<400> 398
   "000"
<210> 399
   <211> 723
   <212> DNA
   <213> Zea mays
<400> 399
   "000"
<210> 400
   <211> 240
   <212> PRT
   <213> Zea mays
<400> 400
   "000"
<210> 401
   <211> 738
   <212> DNA
   <213> Zea mays
<400> 401
   "000"
<210> 402
   <211> 245
   <212> PRT
   <213> Zea mays
<400> 402
   "000"
<210> 403
   <211> 726
   <212> DNA
   <213> Zea mays
<400> 403
   "000"
<210> 404
   <211> 241
   <212> PRT
   <213> Zea mays
<400> 404
   "000"
<210> 405
   <211> 330
   <212> DNA
   <213> Zea mays
<400> 405
   "000"
<210> 406
   <211> 109
   <212> PRT
   <213> Zea mays
<400> 406
   "000"
<210> 407
   <211> 1026
   <212> DNA
   <213> Zea mays
<400> 407
   "000"
<210> 408
   <211> 341
   <212> PRT
   <213> Zea mays
<400> 408
   "000"
<210> 409
   <211> 816
   <212> DNA
   <213> Zea mays
<400> 409
   "000"
<210> 410
   <211> 271
   <212> PRT
   <213> Zea mays
<400> 410
   "000"
<210> 411
   <211> 786
   <212> DNA
   <213> Zea mays
<400> 411
   "000"
<210> 412
   <211> 261
   <212> PRT
   <213> Zea mays
<400> 412
   "000"
<210> 413
   <211> 831
   <212> DNA
   <213> Zea mays
<400> 413
   "000"
<210> 414
   <211> 276
   <212> PRT
   <213> Zea mays
<400> 414
   "000"
<210> 415
   <211> 672
   <212> DNA
   <213> Zea mays
<400> 415
   "000"
<210> 416
   <211> 223
   <212> PRT
   <213> Zea mays
<400> 416
   "000"
<210> 417
   <211> 996
   <212> DNA
   <213> Zea mays
<400> 417
   "000"
<210> 418
   <211> 331
   <212> PRT
   <213> Zea mays
<400> 418
   "000"
<210> 419
   <211> 696
   <212> DNA
   <213> Zea mays
<400> 419
   "000"
<210> 420
   <211> 231
   <212> PRT
   <213> Zea mays
<400> 420
   "000"
<210> 421
   <211> 837
   <212> DNA
   <213> Zea mays
<400> 421
   "000"
<210> 422
   <211> 278
   <212> PRT
   <213> Zea mays
<400> 422
   "000"
<210> 423
   <211> 570
   <212> DNA
   <213> Zea mays
<400> 423
   "000"
<210> 424
   <211> 189
   <212> PRT
   <213> Zea mays
<400> 424
   "000"
<210> 425
   <211> 1659
   <212> DNA
   <213> Zea mays
<400> 425
   "000"
<210> 426
   <211> 552
   <212> PRT
   <213> Zea mays
<400> 426
   "000"
<210> 427
   <211> 750
   <212> DNA
   <213> Zea mays
<400> 427
   "000"
<210> 428
   <211> 249
   <212> PRT
   <213> Zea mays
<400> 428
   "000"
<210> 429
   <211> 450
   <212> DNA
   <213> Zea mays
<400> 429
   "000"
<210> 430
   <211> 149
   <212> PRT
   <213> Zea mays
<400> 430
   "000"
<210> 431
   <211> 678
   <212> DNA
   <213> Zea mays
<400> 431
   "000"
<210> 432
   <211> 225
   <212> PRT
   <213> Zea mays
<400> 432
   "000"

<210> 433
   <211> 768
   <212> DNA
   <213> Zea mays
<400> 433
   "000"
<210> 434
   <211> 255
   <212> PRT
   <213> Zea mays
<400> 434
   "000"
<210> 435
   <211> 1230
   <212> DNA
   <213> Zea mays
<400> 435
   "000"
<210> 436
   <211> 409
   <212> PRT
   <213> Zea mays
<400> 436
   "000"
<210> 437
   <211> 747
   <212> DNA
   <213> Zea mays
<400> 437
   "000"
<210> 438
   <211> 248
   <212> PRT
   <213> Zea mays
<400> 438
   "000"
<210> 439
   <211> 843
   <212> DNA
   <213> Zea mays
<400> 439
   "000"
<210> 440
   <211> 280
   <212> PRT
   <213> Zea mays
<400> 440
   "000"
<210> 441
   <211> 552
   <212> DNA
   <213> Zea mays
<400> 441
   "000"
<210> 442
   <211> 183
   <212> PRT
   <213> Zea mays
<400> 442
   "000"
<210> 443
   <211> 840
   <212> DNA
   <213> Zea mays
<400> 443
   "000"
<210> 444
   <211> 279
   <212> PRT
   <213> Zea mays
<400> 444
   "000"
<210> 445
   <211> 849
   <212> DNA
   <213> Zea mays
<400> 445
   "000"
<210> 446
   <211> 282
   <212> PRT
   <213> Zea mays
<400> 446
   "000"
<210> 447
   <211> 993
   <212> DNA
   <213> Zea mays
<400> 447
   "000"
<210> 448
   <211> 330
   <212> PRT
   <213> Zea mays
<400> 448
   "000"
<210> 449
   <211> 1575
   <212> DNA
   <213> Zea mays
<400> 449
   "000"
<210> 450
   <211> 524
   <212> PRT
   <213> Zea mays
<400> 450
   "000"
<210> 451
   <211> 723
   <212> DNA
   <213> Zea mays
<400> 451
   "000"
<210> 452
   <211> 240
   <212> PRT
   <213> Zea mays
<400> 452
   "000"
<210> 453
   <211> 1590
   <212> DNA
   <213> Zea mays
<400> 453
   "000"
<210> 454
   <211> 529
   <212> PRT
   <213> Zea mays
<400> 454
   "000"
<210> 455
   <211> 915
   <212> DNA
   <213> Zea mays
<400> 455
   "000"
<210> 456
   <211> 304
   <212> PRT
   <213> Zea mays
<400> 456
   "000"
<210> 457
   <211> 915
   <212> DNA
   <213> Zea mays
<400> 457
   "000"
<210> 458
   <211> 304
   <212> PRT
   <213> Zea mays
<400> 458
   "000"
<210> 459
   <211> 804
   <212> DNA
   <213> Zea mays
<400> 459
   "000"
<210> 460
   <211> 267
   <212> PRT
   <213> Zea mays
<400> 460
   "000"
<210> 461
   <211> 450
   <212> DNA
   <213> Zea mays
<400> 461
   "000"
<210> 462
   <211> 149
   <212> PRT
   <213> Zea mays
<400> 462
   "000"
<210> 463
   <211> 795
   <212> DNA
   <213> Zea mays
<400> 463
   "000"
<210> 464
   <211> 264
   <212> PRT
   <213> Zea mays
<400> 464
   "000"
<210> 465
   <211> 651
   <212> DNA
   <213> Zea mays
<400> 465
   "000"
<210> 466
   <211> 216
   <212> PRT
   <213> Zea mays
<400> 466
   "000"
<210> 467
   <211> 903
   <212> DNA
   <213> Zea mays
<400> 467
   "000"
<210> 468
   <211> 300
   <212> PRT
   <213> Zea mays
<400> 468
   "000"
<210> 469
   <211> 627
   <212> DNA
   <213> Zea mays
<400> 469
   "000"
<210> 470
   <211> 208
   <212> PRT
   <213> Zea mays
<400> 470
   "000"
<210> 471
   <211> 726
   <212> DNA
   <213> Zea mays
<400> 471
   "000"
<210> 472
   <211> 241
   <212> PRT
   <213> Zea mays
<400> 472
   "000"
<210> 473
   <211> 819
   <212> DNA
   <213> Zea mays
<400> 473
   "000"
<210> 474
   <211> 272
   <212> PRT
   <213> Zea mays
<400> 474
   "000"
<210> 475
   <211> 627
   <212> DNA
   <213> Zea mays
<400> 475
   "000"
<210> 476
   <211> 208
   <212> PRT
   <213> Zea mays
<400> 476
   "000"
<210> 477
   <211> 1701
   <212> DNA
   <213> Zea mays
<400> 477
   "000"
<210> 478
   <211> 566
   <212> PRT
   <213> Zea mays
<400> 478
   "000"
<210> 479
   <211> 1635
   <212> DNA
   <213> Zea mays
<400> 479
   "000"
<210> 480
   <211> 544
   <212> PRT
   <213> Zea mays
<400> 480
   "000"
<210> 481
   <211> 744
   <212> DNA
   <213> Zea mays
<400> 481
   "000"
<210> 482
   <211> 247
   <212> PRT
   <213> Zea mays
<400> 482
   "000"
<210> 483
   <211> 807
   <212> DNA
   <213> Zea mays
<400> 483
   "000"
<210> 484
   <211> 268
   <212> PRT
   <213> Zea mays
<400> 484
   "000"
<210> 485
   <211> 855
   <212> DNA
   <213> Zea mays
<400> 485
   "000"
<210> 486
   <211> 284
   <212> PRT
   <213> Zea mays
<400> 486
   "000"
<210> 487
   <211> 921
   <212> DNA
   <213> Zea mays
<400> 487
   "000"
<210> 488
   <211> 306
   <212> PRT
   <213> Zea mays
<400> 488
   "000"
<210> 489
   <211> 1266
   <212> DNA
   <213> Zea mays
<400> 489
   "000"
<210> 490
   <211> 421
   <212> PRT
   <213> Zea mays
<400> 490
   "000"
<210> 491
   <211> 828
   <212> DNA
   <213> Zea mays
<400> 491
   "000"
<210> 492
   <211> 275
   <212> PRT
   <213> Zea mays
<400> 492
   "000"
<210> 493
   <211> 834
   <212> DNA
   <213> Zea mays
<400> 493
   "000"
<210> 494
   <211> 277
   <212> PRT
   <213> Zea mays
<400> 494
   "000"
<210> 495
   <211> 720
   <212> DNA
   <213> Zea mays
<400> 495
   "000"
<210> 496
   <211> 239
   <212> PRT
   <213> Zea mays
<400> 496
   "000"
<210> 497
   <211> 1545
   <212> DNA
   <213> Zea mays
<400> 497
   "000"
<210> 498
   <211> 514
   <212> PRT
   <213> Zea mays
<400> 498
   "000"
<210> 499
   <211> 813
   <212> DNA
   <213> Zea mays
<400> 499
   "000"
<210> 500
   <211> 270
   <212> PRT
   <213> Zea mays
<400> 500
   "000"
<210> 501
   <211> 1308
   <212> DNA
   <213> Zea mays
<400> 501
   "000"
<210> 502
   <211> 435
   <212> PRT
   <213> Zea mays
<400> 502
   "000"
<210> 503
   <211> 717
   <212> DNA
   <213> Zea mays
<400> 503
   "000"
<210> 504
   <211> 238
   <212> PRT
   <213> Zea mays
<400> 504
   "000"
<210> 505
   <211> 1047
   <212> DNA
   <213> Zea mays
<400> 505
   "000"
<210> 506
   <211> 348
   <212> PRT
   <213> Zea mays
<400> 506
   "000"
<210> 507
   <211> 1320
   <212> DNA
   <213> Zea mays
<400> 507
   "000"
<210> 508
   <211> 439
   <212> PRT
   <213> Zea mays
<400> 508
   "000"
<210> 509
   <211> 678
   <212> DNA
   <213> Zea mays
<400> 509
   "000"
<210> 510
   <211> 225
   <212> PRT
   <213> Zea mays
<400> 510
   "000"
<210> 511
   <211> 1692
   <212> DNA
   <213> Zea mays
<400> 511
   "000"
<210> 512
   <211> 563
   <212> PRT
   <213> Zea mays
<400> 512
   "000"
<210> 513
   <211> 1359
   <212> DNA
   <213> Zea mays
<400> 513
   "000"
<210> 514
   <211> 452
   <212> PRT
   <213> Zea mays
<400> 514
   "000"
<210> 515
   <211> 810
   <212> DNA
   <213> Zea mays
<400> 515
   "000"
<210> 516
   <211> 269
   <212> PRT
   <213> Zea mays
<400> 516
   "000"
<210> 517
   <211> 1323
   <212> DNA
   <213> Zea mays
<400> 517
   "000"
<210> 518
   <211> 440
   <212> PRT
   <213> Zea mays
<400> 518
   "000"
<210> 519
   <211> 768
   <212> DNA
   <213> Zea mays
<400> 519
   "000"
<210> 520
   <211> 255
   <212> PRT
   <213> Zea mays
<400> 520
   "000"
<210> 521
   <211> 891
   <212> DNA
   <213> Zea mays
<400> 521
   "000"
<210> 522
   <211> 296
   <212> PRT
   <213> Zea mays
<400> 522
   "000"
<210> 523
   <211> 903
   <212> DNA
   <213> Zea mays
<400> 523
   "000"
<210> 524
   <211> 300
   <212> PRT
   <213> Zea mays
<400> 524
   "000"
<210> 525
   <211> 867
   <212> DNA
   <213> Zea mays
<400> 525
   "000"
<210> 526
   <211> 288
   <212> PRT
   <213> Zea mays
<400> 526
   "000"
<210> 527
   <211> 654
   <212> DNA
   <213> Zea mays
<400> 527
   "000"
<210> 528
   <211> 217
   <212> PRT
   <213> Zea mays
<400> 528
   "000"
<210> 529
   <211> 522
   <212> DNA
   <213> Zea mays
<400> 529
   "000"
<210> 530
   <211> 173
   <212> PRT
   <213> Zea mays
<400> 530
   "000"
<210> 531
   <211> 1047
   <212> DNA
   <213> Zea mays
<400> 531
   "000"
<210> 532
   <211> 348
   <212> PRT
   <213> Zea mays
<400> 532
   "000"
<210> 533
   <211> 843
   <212> DNA
   <213> Zea mays
<400> 533
   "000"
<210> 534
   <211> 280
   <212> PRT
   <213> Zea mays
<400> 534
   "000"
<210> 535
   <211> 1533
   <212> DNA
   <213> Zea mays
<400> 535
   "000"
<210> 536
   <211> 510
   <212> PRT
   <213> Zea mays
<400> 536
   "000"
<210> 537
   <211> 1029
   <212> DNA
   <213> Zea mays
<400> 537
   "000"
<210> 538
   <211> 342
   <212> PRT
   <213> Zea mays
<400> 538
   "000"
<210> 539
   <211> 828
   <212> DNA
   <213> Zea mays
<400> 539
   "000"
<210> 540
   <211> 275
   <212> PRT
   <213> Zea mays
<400> 540
   "000"
<210> 541
   <211> 1701
   <212> DNA
   <213> Zea mays
<400> 541
   "000"
<210> 542
   <211> 566
   <212> PRT
   <213> Zea mays
<400> 542
   "000"
<210> 543
   <211> 1644
   <212> DNA
   <213> Zea mays
<400> 543
   "000"
<210> 544
   <211> 547
   <212> PRT
   <213> Zea mays
<400> 544
   "000"
<210> 545
   <211> 618
   <212> DNA
   <213> Zea mays
<400> 545
   "000"
<210> 546
   <211> 205
   <212> PRT
   <213> Zea mays
<400> 546
   "000"
<210> 547
   <211> 1047
   <212> DNA
   <213> Zea mays
<400> 547
   "000"
<210> 548
   <211> 348
   <212> PRT
   <213> Zea mays
<400> 548
   "000"
<210> 549
   <211> 795
   <212> DNA
   <213> Zea mays
<400> 549
   "000"
<210> 550
   <211> 264
   <212> PRT
   <213> Zea mays
<400> 550
   "000"
<210> 551
   <211> 498
   <212> DNA
   <213> Glycine max
<400> 551
   "000"
<210> 552
   <211> 165
   <212> PRT
   <213> Glycine max
<400> 552
   "000"
<210> 553
   <211> 876
   <212> DNA
   <213> Brassica napus
<400> 553
   "000"
<210> 554
   <211> 291
   <212> PRT
   <213> Brassica napus
<400> 554
   "000"
<210> 555
   <211> 1626
   <212> DNA
   <213> Brassica napus
<400> 555
   "000"
<210> 556
   <211> 541
   <212> PRT
   <213> Brassica napus
<400> 556
   "000"
<210> 557
   <211> 1227
   <212> DNA
   <213> Brassica napus
<400> 557
   "000"
<210> 558
   <211> 408
   <212> PRT
   <213> Brassica napus
<400> 558
   "000"
<210> 559
   <211> 1002
   <212> DNA
   <213> Glycine max
<400> 559
   "000"
<210> 560
   <211> 333
   <212> PRT
   <213> Glycine max
<400> 560
   "000"
<210> 561
   <211> 1137
   <212> DNA
   <213> Oryza sativa
<400> 561
   "000"
<210> 562
   <211> 378
   <212> PRT
   <213> Oryza sativa
<400> 562
   "000"
<210> 563
   <211> 1056
   <212> DNA
   <213> Brassica napus
<400> 563
   "000"
<210> 564
   <211> 351
   <212> PRT
   <213> Brassica napus
<400> 564
   "000"
<210> 565
   <211> 1107
   <212> DNA
   <213> Zea mays
<400> 565
   "000"
<210> 566
   <211> 368
   <212> PRT
   <213> Zea mays
<400> 566
   "000"
<210> 567
   <211> 867
   <212> DNA
   <213> Oryza sativa
<400> 567
   "000"
<210> 568
   <211> 288
   <212> PRT
   <213> Oryza sativa
<400> 568
   "000"
<210> 569
   <211> 963
   <212> DNA
   <213> Glycine max
<400> 569
   "000"
<210> 570
   <211> 320
   <212> PRT
   <213> Glycine max
<400> 570
   "000"
<210> 571
   <211> 1746
   <212> DNA
   <213> Brassica napus
<400> 571
   "000"
<210> 572
   <211> 581
   <212> PRT
   <213> Brassica napus
<400> 572
   "000"
<210> 573
   <211> 1554
   <212> DNA
   <213> Oryza sativa
<400> 573
   "000"
<210> 574
   <211> 517
   <212> PRT
   <213> Oryza sativa
<400> 574
   "000"
<210> 575
   <211> 489
   <212> DNA
   <213> Glycine max
<400> 575
   "000"
<210> 576
   <211> 162
   <212> PRT
   <213> Glycine max
<400> 576
   "000"
<210> 577
   <211> 780
   <212> DNA
   <213> Brassica napus
<400> 577
   "000"
<210> 578
   <211> 259
   <212> PRT
   <213> Brassica napus
<400> 578
   "000"
<210> 579
   <211> 1299
   <212> DNA
   <213> Brassica napus
<400> 579
   "000"
<210> 580
   <211> 432
   <212> PRT
   <213> Brassica napus
<400> 580
   "000"
<210> 581
   <211> 723
   <212> DNA
   <213> Oryza sativa
<400> 581
   "000"
<210> 582
   <211> 240
   <212> PRT
   <213> Oryza sativa
<400> 582
   "000"
<210> 583
   <211> 1224
   <212> DNA
   <213> Brassica napus
<400> 583
   "000"
<210> 584
   <211> 407
   <212> PRT
   <213> Brassica napus
<400> 584
   "000"
<210> 585
   <211> 801
   <212> DNA
   <213> Brassica napus
<400> 585
   "000"
<210> 586
   <211> 266
   <212> PRT
   <213> Brassica napus
<400> 586
   "000"
<210> 587
   <211> 1206
   <212> DNA
   <213> Glycine max
<400> 587
   "000"
<210> 588
   <211> 401
   <212> PRT
   <213> Glycine max
<400> 588
   "000"
<210> 589
   <211> 1137
   <212> DNA
   <213> Oryza sativa
<400> 589
   "000"
<210> 590
   <211> 378
   <212> PRT
   <213> Oryza sativa
<400> 590
   "000"
<210> 591
   <211> 1017
   <212> DNA
   <213> Zea mays
<400> 591
   "000"
<210> 592
   <211> 338
   <212> PRT
   <213> Zea mays
<400> 592
   "000"

<210> 593
   <211> 720
   <212> DNA
   <213> Brassica napus
<400> 593
   "000"
<210> 594
   <211> 239
   <212> PRT
   <213> Brassica napus
<400> 594
   "000"
<210> 595
   <211> 879
   <212> DNA
   <213> Brassica napus
<400> 595
   "000"
<210> 596
   <211> 292
   <212> PRT
   <213> Brassica napus
<400> 596
   "000"
<210> 597
   <211> 1398
   <212> DNA
   <213> Brassica napus
<400> 597
   "000"
<210> 598
   <211> 465
   <212> PRT
   <213> Brassica napus
<400> 598
   "000"
<210> 599
   <211> 966
   <212> DNA
   <213> Brassica napus
<400> 599
   "000"
<210> 600
   <211> 321
   <212> PRT
   <213> Brassica napus
<400> 600
   "000"
<210> 601
   <211> 1620
   <212> DNA
   <213> Brassica napus
<400> 601
   "000"
<210> 602
   <211> 539
   <212> PRT
   <213> Brassica napus
<400> 602
   "000"
<210> 603
   <211> 357
   <212> DNA
   <213> Brassica napus
<400> 603
   "000"
<210> 604
   <211> 118
   <212> PRT
   <213> Brassica napus
<400> 604
   "000"
<210> 605
   <211> 876
   <212> DNA
   <213> Glycine max
<400> 605
   "000"
<210> 606
   <211> 291
   <212> PRT
   <213> Glycine max
<400> 606
   "000"
<210> 607
   <211> 465
   <212> DNA
   <213> Brassica napus
<400> 607
   "000"
<210> 608
   <211> 154
   <212> PRT
   <213> Brassica napus
<400> 608
   "000"
<210> 609
   <211> 963
   <212> DNA
   <213> Zea mays
<400> 609
   "000"
<210> 610
   <211> 320
   <212> PRT
   <213> Zea mays
<400> 610
   "000"
<210> 611
   <211> 1089
   <212> DNA
   <213> Oryza sativa
<400> 611
   "000"
<210> 612
   <211> 362
   <212> PRT
   <213> Oryza sativa
<400> 612
   "000"
<210> 613
   <211> 642
   <212> DNA
   <213> Brassica napus
<400> 613
   "000"
<210> 614
   <211> 213
   <212> PRT
   <213> Brassica napus
<400> 614
   "000"
<210> 615
   <211> 1224
   <212> DNA
   <213> Brassica napus
<400> 615
   "000"
<210> 616
   <211> 407
   <212> PRT
   <213> Brassica napus
<400> 616
   "000"
<210> 617
   <211> 1524
   <212> DNA
   <213> Oryza sativa
<400> 617
   "000"
<210> 618
   <211> 507
   <212> PRT
   <213> Oryza sativa
<400> 618
   "000"
<210> 619
   <211> 996
   <212> DNA
   <213> Brassica napus
<400> 619
   "000"
<210> 620
   <211> 331
   <212> PRT
   <213> Brassica napus
<400> 620
   "000"
<210> 621
   <211> 939
   <212> DNA
   <213> Glycine max
<400> 621
   "000"
<210> 622
   <211> 312
   <212> PRT
   <213> Glycine max
<400> 622
   "000"
<210> 623
   <211> 765
   <212> DNA
   <213> Brassica napus
<400> 623
   "000"
<210> 624
   <211> 254
   <212> PRT
   <213> Brassica napus
<400> 624
   "000"
<210> 625
   <211> 1158
   <212> DNA
   <213> Brassica napus
<400> 625
   "000"
<210> 626
   <211> 385
   <212> PRT
   <213> Brassica napus
<400> 626
   "000"
<210> 627
   <211> 1092
   <212> DNA
   <213> Brassica napus
<400> 627
   "000"
<210> 628
   <211> 363
   <212> PRT
   <213> Brassica napus
<400> 628
   "000"
<210> 629
   <211> 615
   <212> DNA
   <213> Brassica napus
<400> 629
   "000"
<210> 630
   <211> 204
   <212> PRT
   <213> Brassica napus
<400> 630
   "000"
<210> 631
   <211> 633
   <212> DNA
   <213> Brassica napus
<400> 631
   "000"
<210> 632
   <211> 210
   <212> PRT
   <213> Brassica napus
<400> 632
   "000"
<210> 633
   <211> 810
   <212> DNA
   <213> Oryza sativa
<400> 633
   "000"
<210> 634
   <211> 269
   <212> PRT
   <213> Oryza sativa
<400> 634
   "000"
<210> 635
   <211> 783
   <212> DNA
   <213> Oryza sativa
<400> 635
   "000"
<210> 636
   <211> 260
   <212> PRT
   <213> Oryza sativa
<400> 636
   "000"
<210> 637
   <211> 1065
   <212> DNA
   <213> Brassica napus
<400> 637
   "000"
<210> 638
   <211> 354
   <212> PRT
   <213> Brassica napus
<400> 638
   "000"
<210> 639
   <211> 984
   <212> DNA
   <213> Brassica napus
<400> 639
   "000"
<210> 640
   <211> 327
   <212> PRT
   <213> Brassica napus
<400> 640
   "000"
<210> 641
   <211> 1239
   <212> DNA
   <213> Glycine max
<400> 641
   "000"
<210> 642
   <211> 412
   <212> PRT
   <213> Glycine max
<400> 642
   "000"
<210> 643
   <211> 1341
   <212> DNA
   <213> Brassica napus
<400> 643
   "000"
<210> 644
   <211> 446
   <212> PRT
   <213> Brassica napus
<400> 644
   "000"
<210> 645
   <211> 1182
   <212> DNA
   <213> Brassica napus
<400> 645
   "000"
<210> 646
   <211> 393
   <212> PRT
   <213> Brassica napus
<400> 646
   "000"
<210> 647
   <211> 861
   <212> DNA
   <213> Brassica napus
<400> 647
   "000"
<210> 648
   <211> 286
   <212> PRT
   <213> Brassica napus
<400> 648
   "000"
<210> 649
   <211> 1131
   <212> DNA
   <213> Brassica napus
<400> 649
   "000"
<210> 650
   <211> 376
   <212> PRT
   <213> Brassica napus
<400> 650
   "000"
<210> 651
   <211> 1197
   <212> DNA
   <213> Brassica napus
<400> 651
   "000"
<210> 652
   <211> 398
   <212> PRT
   <213> Brassica napus
<400> 652
   "000"
<210> 653
   <211> 951
   <212> DNA
   <213> Glycine max
<400> 653
   "000"
<210> 654
   <211> 316
   <212> PRT
   <213> Glycine max
<400> 654
   "000"
<210> 655
   <211> 513
   <212> DNA
   <213> Brassica napus
<400> 655
   "000"
<210> 656
   <211> 170
   <212> PRT
   <213> Brassica napus
<400> 656
   "000"
<210> 657
   <211> 660
   <212> DNA
   <213> Brassica napus
<400> 657
   "000"
<210> 658
   <211> 219
   <212> PRT
   <213> Brassica napus
<400> 658
   "000"
<210> 659
   <211> 963
   <212> DNA
   <213> Glycine max
<400> 659
   "000"
<210> 660
   <211> 320
   <212> PRT
   <213> Glycine max
<400> 660
   "000"
<210> 661
   <211> 1512
   <212> DNA
   <213> Glycine max
<400> 661
   "000"
<210> 662
   <211> 503
   <212> PRT
   <213> Glycine max
<400> 662
   "000"
<210> 663
   <211> 798
   <212> DNA
   <213> Brassica napus
<400> 663
   "000"
<210> 664
   <211> 265
   <212> PRT
   <213> Brassica napus
<400> 664
   "000"
<210> 665
   <211> 654
   <212> DNA
   <213> Oryza sativa
<400> 665
   "000"
<210> 666
   <211> 217
   <212> PRT
   <213> Oryza sativa
<400> 666
   "000"
<210> 667
   <211> 1413
   <212> DNA
   <213> Brassica napus
<400> 667
   "000"
<210> 668
   <211> 470
   <212> PRT
   <213> Brassica napus
<400> 668
   "000"
<210> 669
   <211> 1314
   <212> DNA
   <213> Brassica napus
<400> 669
   "000"
<210> 670
   <211> 437
   <212> PRT
   <213> Brassica napus
<400> 670
   "000"
<210> 671
   <211> 921
   <212> DNA
   <213> Oryza sativa
<400> 671
   "000"
<210> 672
   <211> 306
   <212> PRT
   <213> Oryza sativa
<400> 672
   "000"
<210> 673
   <211> 1713
   <212> DNA
   <213> Brassica napus
<400> 673
   "000"
<210> 674
   <211> 570
   <212> PRT
   <213> Brassica napus
<400> 674
   "000"
<210> 675
   <211> 375
   <212> DNA
   <213> Glycine max
<400> 675
   "000"
<210> 676
   <211> 124
   <212> PRT
   <213> Glycine max
<400> 676
   "000"
<210> 677
   <211> 1893
   <212> DNA
   <213> Glycine max
<400> 677
   "000"
<210> 678
   <211> 630
   <212> PRT
   <213> Glycine max
<400> 678
   "000"
<210> 679
   <211> 1089
   <212> DNA
   <213> Glycine max
<400> 679
   "000"
<210> 680
   <211> 362
   <212> PRT
   <213> Glycine max
<400> 680
   "000"
<210> 681
   <211> 2535
   <212> DNA
   <213> Glycine max
<400> 681
   "000"
<210> 682
   <211> 844
   <212> PRT
   <213> Glycine max
<400> 682
   "000"
<210> 683
   <211> 744
   <212> DNA
   <213> Glycine max
<400> 683
   "000"
<210> 684
   <211> 247
   <212> PRT
   <213> Glycine max
<400> 684
   "000"
<210> 685
   <211> 423
   <212> DNA
   <213> Glycine max
<400> 685
   "000"
<210> 686
   <211> 140
   <212> PRT
   <213> Glycine max
<400> 686
   "000"
<210> 687
   <211> 1272
   <212> DNA
   <213> Brassica napus
<400> 687
   "000"
<210> 688
   <211> 423
   <212> PRT
   <213> Brassica napus
<400> 688
   "000"
<210> 689
   <211> 879
   <212> DNA
   <213> Brassica napus
<400> 689
   "000"
<210> 690
   <211> 292
   <212> PRT
   <213> Brassica napus
<400> 690
   "000"
<210> 691
   <211> 1290
   <212> DNA
   <213> Brassica napus
<400> 691
   "000"
<210> 692
   <211> 429
   <212> PRT
   <213> Brassica napus
<400> 692
   "000"
<210> 693
   <211> 978
   <212> DNA
   <213> Brassica napus
<400> 693
   "000"
<210> 694
   <211> 325
   <212> PRT
   <213> Brassica napus
<400> 694
   "000"
<210> 695
   <211> 1278
   <212> DNA
   <213> Brassica napus
<400> 695
   "000"
<210> 696
   <211> 425
   <212> PRT
   <213> Brassica napus
<400> 696
   "000"
<210> 697
   <211> 786
   <212> DNA
   <213> Oryza sativa
<400> 697
   "000"
<210> 698
   <211> 261
   <212> PRT
   <213> Oryza sativa
<400> 698
   "000"
<210> 699
   <211> 606
   <212> DNA
   <213> Glycine max
<400> 699
   "000"
<210> 700
   <211> 201
   <212> PRT
   <213> Glycine max
<400> 700
   "000"
<210> 701
   <211> 984
   <212> DNA
   <213> Oryza sativa
<400> 701
   "000"
<210> 702
   <211> 327
   <212> PRT
   <213> Oryza sativa
<400> 702
   "000"
<210> 703
   <211> 1026
   <212> DNA
   <213> Brassica napus
<400> 703
   "000"
<210> 704
   <211> 341
   <212> PRT
   <213> Brassica napus
<400> 704
   "000"
<210> 705
   <211> 927
   <212> DNA
   <213> Glycine max
<400> 705
   "000"
<210> 706
   <211> 308
   <212> PRT
   <213> Glycine max
<400> 706
   "000"
<210> 707
   <211> 813
   <212> DNA
   <213> Brassica napus
<400> 707
   "000"
<210> 708
   <211> 270
   <212> PRT
   <213> Brassica napus
<400> 708
   "000"
<210> 709
   <211> 1395
   <212> DNA
   <213> Zea mays
<400> 709
   "000"
<210> 710
   <211> 464
   <212> PRT
   <213> Zea mays
<400> 710
   "000"
<210> 711
   <211> 1158
   <212> DNA
   <213> Brassica napus
<400> 711
   "000"
<210> 712
   <211> 385
   <212> PRT
   <213> Brassica napus
<400> 712
   "000"
<210> 713
   <211> 1368
   <212> DNA
   <213> Brassica napus
<400> 713
   "000"
<210> 714
   <211> 455
   <212> PRT
   <213> Brassica napus
<400> 714
   "000"
<210> 715
   <211> 1089
   <212> DNA
   <213> Brassica napus
<400> 715
   "000"
<210> 716
   <211> 362
   <212> PRT
   <213> Brassica napus
<400> 716
   "000"
<210> 717
   <211> 726
   <212> DNA
   <213> Glycine max
<400> 717
   "000"
<210> 718
   <211> 241
   <212> PRT
   <213> Glycine max
<400> 718
   "000"
<210> 719
   <211> 1212
   <212> DNA
   <213> Brassica napus
<400> 719
   "000"
<210> 720
   <211> 403
   <212> PRT
   <213> Brassica napus
<400> 720
   "000"
<210> 721
   <211> 1569
   <212> DNA
   <213> Brassica napus
<400> 721
   "000"
<210> 722
   <211> 522
   <212> PRT
   <213> Brassica napus
<400> 722
   "000"
<210> 723
   <211> 702
   <212> DNA
   <213> Brassica napus
<400> 723
   "000"
<210> 724
   <211> 233
   <212> PRT
   <213> Brassica napus
<400> 724
   "000"
<210> 725
   <211> 1518
   <212> DNA
   <213> Brassica napus
<400> 725
   "000"
<210> 726
   <211> 505
   <212> PRT
   <213> Brassica napus
<400> 726
   "000"
<210> 727
   <211> 1515
   <212> DNA
   <213> Brassica napus
<400> 727
   "000"
<210> 728
   <211> 504
   <212> PRT
   <213> Brassica napus
<400> 728
   "000"
<210> 729
   <211> 1242
   <212> DNA
   <213> Brassica napus
<400> 729
   "000"
<210> 730
   <211> 413
   <212> PRT
   <213> Brassica napus
<400> 730
   "000"
<210> 731
   <211> 1089
   <212> DNA
   <213> Brassica napus
<400> 731
   "000"
<210> 732
   <211> 362
   <212> PRT
   <213> Brassica napus
<400> 732
   "000"
<210> 733
   <211> 1857
   <212> DNA
   <213> Glycine max
<400> 733
   "000"
<210> 734
   <211> 618
   <212> PRT
   <213> Glycine max
<400> 734
   "000"
<210> 735
   <211> 1635
   <212> DNA
   <213> Brassica napus
<400> 735
   "000"
<210> 736
   <211> 544
   <212> PRT
   <213> Brassica napus
<400> 736
   "000"
<210> 737
   <211> 1419
   <212> DNA
   <213> Glycine max
<400> 737
   "000"
<210> 738
   <211> 472
   <212> PRT
   <213> Glycine max
<400> 738
   "000"
<210> 739
   <211> 1008
   <212> DNA
   <213> Oryza sativa
<400> 739
   "000"
<210> 740
   <211> 335
   <212> PRT
   <213> Oryza sativa
<400> 740
   "000"
<210> 741
   <211> 1146
   <212> DNA
   <213> Zea mays
<400> 741
   "000"
<210> 742
   <211> 381
   <212> PRT
   <213> Zea mays
<400> 742
   "000"
<210> 743
   <211> 2199
   <212> DNA
   <213> Glycine max
<400> 743
   "000"
<210> 744
   <211> 732
   <212> PRT
   <213> Glycine max
<400> 744
   "000"
<210> 745
   <211> 1110
   <212> DNA
   <213> Brassica napus
<400> 745
   "000"
<210> 746
   <211> 369
   <212> PRT
   <213> Brassica napus
<400> 746
   "000"
<210> 747
   <211> 2532
   <212> DNA
   <213> Glycine max
<400> 747
   "000"
<210> 748
   <211> 843
   <212> PRT
   <213> Glycine max
<400> 748
   "000"
<210> 749
   <211> 525
   <212> DNA
   <213> Glycine max
<400> 749
   "000"
<210> 750
   <211> 174
   <212> PRT
   <213> Glycine max
<400> 750
   "000"
<210> 751
   <211> 963
   <212> DNA
   <213> Glycine max
<400> 751
   "000"
<210> 752
   <211> 320
   <212> PRT
   <213> Glycine max
<400> 752
   "000"

<210> 753
   <211> 687
   <212> DNA
   <213> Oryza sativa
<400> 753
   "000"
<210> 754
   <211> 228
   <212> PRT
   <213> Oryza sativa
<400> 754
   "000"
<210> 755
   <211> 771
   <212> DNA
   <213> Brassica napus
<400> 755
   "000"
<210> 756
   <211> 256
   <212> PRT
   <213> Brassica napus
<400> 756
   "000"
<210> 757
   <211> 1482
   <212> DNA
   <213> Brassica napus
<400> 757
   "000"
<210> 758
   <211> 493
   <212> PRT
   <213> Brassica napus
<400> 758
   "000"
<210> 759
   <211> 726
   <212> DNA
   <213> Brassica napus
<400> 759
   "000"
<210> 760
   <211> 241
   <212> PRT
   <213> Brassica napus
<400> 760
   "000"
<210> 761
   <211> 1023
   <212> DNA
   <213> Glycine max
<400> 761
   "000"
<210> 762
   <211> 340
   <212> PRT
   <213> Glycine max
<400> 762
   "000"
<210> 763
   <211> 495
   <212> DNA
   <213> Oryza sativa
<400> 763
   "000"
<210> 764
   <211> 164
   <212> PRT
   <213> Oryza sativa
<400> 764
   "000"
<210> 765
   <211> 666
   <212> DNA
   <213> Brassica napus
<400> 765
   "000"
<210> 766
   <211> 221
   <212> PRT
   <213> Brassica napus
<400> 766
   "000"
<210> 767
   <211> 1410
   <212> DNA
   <213> Brassica napus
<400> 767
   "000"
<210> 768
   <211> 469
   <212> PRT
   <213> Brassica napus
<400> 768
   "000"
<210> 769
   <211> 1542
   <212> DNA
   <213> Glycine max
<400> 769
   "000"
<210> 770
   <211> 513
   <212> PRT
   <213> Glycine max
<400> 770
   "000"
<210> 771
   <211> 1050
   <212> DNA
   <213> Brassica napus
<400> 771
   "000"
<210> 772
   <211> 349
   <212> PRT
   <213> Brassica napus
<400> 772
   "000"
<210> 773
   <211> 1422
   <212> DNA
   <213> Glycine max
<400> 773
   "000"
<210> 774
   <211> 473
   <212> PRT
   <213> Glycine max
<400> 774
   "000"
<210> 775
   <211> 759
   <212> DNA
   <213> Oryza sativa
<400> 775
   "000"
<210> 776
   <211> 252
   <212> PRT
   <213> Oryza sativa
<400> 776
   "000"
<210> 777
   <211> 909
   <212> DNA
   <213> Glycine max
<400> 777
   "000"
<210> 778
   <211> 302
   <212> PRT
   <213> Glycine max
<400> 778
   "000"
<210> 779
   <211> 1047
   <212> DNA
   <213> Oryza sativa
<400> 779
   "000"
<210> 780
   <211> 348
   <212> PRT
   <213> Oryza sativa
<400> 780
   "000"
<210> 781
   <211> 1479
   <212> DNA
   <213> Glycine max
<400> 781
   "000"
<210> 782
   <211> 492
   <212> PRT
   <213> Glycine max
<400> 782
   "000"
<210> 783
   <211> 1449
   <212> DNA
   <213> Brassica napus
<400> 783
   "000"
<210> 784
   <211> 482
   <212> PRT
   <213> Brassica napus
<400> 784
   "000"
<210> 785
   <211> 1416
   <212> DNA
   <213> Brassica napus
<400> 785
   "000"
<210> 786
   <211> 471
   <212> PRT
   <213> Brassica napus
<400> 786
   "000"
<210> 787
   <211> 732
   <212> DNA
   <213> Glycine max
<400> 787
   "000"
<210> 788
   <211> 243
   <212> PRT
   <213> Glycine max
<400> 788
   "000"
<210> 789
   <211> 1524
   <212> DNA
   <213> Brassica napus
<400> 789
   "000"
<210> 790
   <211> 507
   <212> PRT
   <213> Brassica napus
<400> 790
   "000"
<210> 791
   <211> 1734
   <212> DNA
   <213> Glycine max
<400> 791
   "000"
<210> 792
   <211> 577
   <212> PRT
   <213> Glycine max
<400> 792
   "000"
<210> 793
   <211> 1899
   <212> DNA
   <213> Glycine max
<400> 793
   "000"
<210> 794
   <211> 632
   <212> PRT
   <213> Glycine max
<400> 794
   "000"
<210> 795
   <211> 1392
   <212> DNA
   <213> Brassica napus
<400> 795
   "000"
<210> 796
   <211> 463
   <212> PRT
   <213> Brassica napus
<400> 796
   "000"
<210> 797
   <211> 1161
   <212> DNA
   <213> Brassica napus
<400> 797
   "000"
<210> 798
   <211> 386
   <212> PRT
   <213> Brassica napus
<400> 798
   "000"
<210> 799
   <211> 1221
   <212> DNA
   <213> Brassica napus
<400> 799
   "000"
<210> 800
   <211> 406
   <212> PRT
   <213> Brassica napus
<400> 800
   "000"
<210> 801
   <211> 1008
   <212> DNA
   <213> Oryza sativa
<400> 801
   "000"
<210> 802
   <211> 335
   <212> PRT
   <213> Oryza sativa
<400> 802
   "000"
<210> 803
   <211> 1758
   <212> DNA
   <213> Brassica napus
<400> 803
   "000"
<210> 804
   <211> 585
   <212> PRT
   <213> Brassica napus
<400> 804
   "000"
<210> 805
   <211> 1140
   <212> DNA
   <213> Brassica napus
<400> 805
   "000"
<210> 806
   <211> 379
   <212> PRT
   <213> Brassica napus
<400> 806
   "000"
<210> 807
   <211> 462
   <212> DNA
   <213> Brassica napus
<400> 807
   "000"
<210> 808
   <211> 153
   <212> PRT
   <213> Brassica napus
<400> 808
   "000"
<210> 809
   <211> 699
   <212> DNA
   <213> Brassica napus
<400> 809
   "000"
<210> 810
   <211> 232
   <212> PRT
   <213> Brassica napus
<400> 810
   "000"
<210> 811
   <211> 522
   <212> DNA
   <213> Oryza sativa
<400> 811
   "000"
<210> 812
   <211> 173
   <212> PRT
   <213> Oryza sativa
<400> 812
   "000"
<210> 813
   <211> 771
   <212> DNA
   <213> Glycine max
<400> 813
   "000"
<210> 814
   <211> 256
   <212> PRT
   <213> Glycine max
<400> 814
   "000"
<210> 815
   <211> 1089
   <212> DNA
   <213> Glycine max
<400> 815
   "000"
<210> 816
   <211> 362
   <212> PRT
   <213> Glycine max
<400> 816
   "000"
<210> 817
   <211> 1113
   <212> DNA
   <213> Brassica napus
<400> 817
   "000"
<210> 818
   <211> 370
   <212> PRT
   <213> Brassica napus
<400> 818
   "000"
<210> 819
   <211> 831
   <212> DNA
   <213> Glycine max
<400> 819
   "000"
<210> 820
   <211> 276
   <212> PRT
   <213> Glycine max
<400> 820
   "000"
<210> 821
   <211> 516
   <212> DNA
   <213> Brassica napus
<400> 821
   "000"
<210> 822
   <211> 171
   <212> PRT
   <213> Brassica napus
<400> 822
   "000"
<210> 823
   <211> 1524
   <212> DNA
   <213> Brassica napus
<400> 823
   "000"
<210> 824
   <211> 507
   <212> PRT
   <213> Brassica napus
<400> 824
   "000"
<210> 825
   <211> 1017
   <212> DNA
   <213> Brassica napus
<400> 825
   "000"
<210> 826
   <211> 338
   <212> PRT
   <213> Brassica napus
<400> 826
   "000"
<210> 827
   <211> 1092
   <212> DNA
   <213> Brassica napus
<400> 827
   "000"
<210> 828
   <211> 363
   <212> PRT
   <213> Brassica napus
<400> 828
   "000"
<210> 829
   <211> 954
   <212> DNA
   <213> Glycine max
<400> 829
   "000"
<210> 830
   <211> 317
   <212> PRT
   <213> Glycine max
<400> 830
   "000"
<210> 831
   <211> 1191
   <212> DNA
   <213> Glycine max
<400> 831
   "000"
<210> 832
   <211> 396
   <212> PRT
   <213> Glycine max
<400> 832
   "000"
<210> 833
   <211> 1035
   <212> DNA
   <213> Brassica napus
<400> 833
   "000"
<210> 834
   <211> 344
   <212> PRT
   <213> Brassica napus
<400> 834
   "000"
<210> 835
   <211> 1281
   <212> DNA
   <213> Brassica napus
<400> 835
   "000"
<210> 836
   <211> 426
   <212> PRT
   <213> Brassica napus
<400> 836
   "000"
<210> 837
   <211> 1041
   <212> DNA
   <213> Brassica napus
<400> 837
   "000"
<210> 838
   <211> 346
   <212> PRT
   <213> Brassica napus
<400> 838
   "000"
<210> 839
   <211> 1542
   <212> DNA
   <213> Glycine max
<400> 839
   "000"
<210> 840
   <211> 513
   <212> PRT
   <213> Glycine max
<400> 840
   "000"
<210> 841
   <211> 1089
   <212> DNA
   <213> Oryza sativa
<400> 841
   "000"
<210> 842
   <211> 362
   <212> PRT
   <213> Oryza sativa
<400> 842
   "000"
<210> 843
   <211> 960
   <212> DNA
   <213> Brassica napus
<400> 843
   "000"
<210> 844
   <211> 319
   <212> PRT
   <213> Brassica napus
<400> 844
   "000"
<210> 845
   <211> 1077
   <212> DNA
   <213> Brassica napus
<400> 845
   "000"
<210> 846
   <211> 358
   <212> PRT
   <213> Brassica napus
<400> 846
   "000"
<210> 847
   <211> 1134
   <212> DNA
   <213> Glycine max
<400> 847
   "000"
<210> 848
   <211> 377
   <212> PRT
   <213> Glycine max
<400> 848
   "000"
<210> 849
   <211> 1398
   <212> DNA
   <213> Brassica napus
<400> 849
   "000"
<210> 850
   <211> 465
   <212> PRT
   <213> Brassica napus
<400> 850
   "000"
<210> 851
   <211> 750
   <212> DNA
   <213> Brassica napus
<400> 851
   "000"
<210> 852
   <211> 249
   <212> PRT
   <213> Brassica napus
<400> 852
   "000"
<210> 853
   <211> 513
   <212> DNA
   <213> Brassica napus
<400> 853
   "000"
<210> 854
   <211> 170
   <212> PRT
   <213> Brassica napus
<400> 854
   "000"
<210> 855
   <211> 654
   <212> DNA
   <213> Oryza sativa
<400> 855
   "000"
<210> 856
   <211> 217
   <212> PRT
   <213> Oryza sativa
<400> 856
   "000"
<210> 857
   <211> 1506
   <212> DNA
   <213> Glycine max
<400> 857
   "000"
<210> 858
   <211> 501
   <212> PRT
   <213> Glycine max
<400> 858
   "000"
<210> 859
   <211> 615
   <212> DNA
   <213> Brassica napus
<400> 859
   "000"
<210> 860
   <211> 204
   <212> PRT
   <213> Brassica napus
<400> 860
   "000"
<210> 861
   <211> 1728
   <212> DNA
   <213> Glycine max
<400> 861
   "000"
<210> 862
   <211> 575
   <212> PRT
   <213> Glycine max
<400> 862
   "000"
<210> 863
   <211> 1044
   <212> DNA
   <213> Glycine max
<400> 863
   "000"
<210> 864
   <211> 347
   <212> PRT
   <213> Glycine max
<400> 864
   "000"
<210> 865
   <211> 834
   <212> DNA
   <213> Glycine max
<400> 865
   "000"
<210> 866
   <211> 277
   <212> PRT
   <213> Glycine max
<400> 866
   "000"
<210> 867
   <211> 1227
   <212> DNA
   <213> Brassica napus
<400> 867
   "000"
<210> 868
   <211> 408
   <212> PRT
   <213> Brassica napus
<400> 868
   "000"
<210> 869
   <211> 30
   <212> DNA
   <213> Artificial
<220>
   <223> primer
<400> 869
   atagaattcc caccgggcac attgagcaat 30
<210> 870
   <211> 29
   <212> DNA
   <213> Artificial
<220>
   <223> primer
<400> 870
   atagtcgacc accgggcaca ttgagcaat 29
<210> 871
   <211> 29
   <212> DNA
   <213> Artificial
<220>
   <223> primer
<400> 871 atagtcgaca tgcttcgact gatcgacga 29
<210> 872
   <211> 29
   <212> DNA
   <213> Artificial
<220>
   <223> primer
<400> 872
   atagaattcc accgggcaca ttgagcaat 29
<210> 873
   <211> 29
   <212> DNA
   <213> Artificial
<220>
   <223> primer
<400> 873
   atacccggga tgcttcgact gatcgacga 29
<210> 874
   <211> 29
   <212> DNA
   <213> Artificial
<220>
   <223> primer
<400> 874
   atatctagac accgggcaca ttgagcaat 29
<210> 875
   <211> 29
   <212> DNA
   <213> Artificial
<220>
   <223> primer
<400> 875
   atagtcgaca tgcttcgact gatcgacga 29
<210> 876
   <211> 29
   <212> DNA
   <213> Artificial
<220>
   <223> primer
<400> 876
   atagaattca tgcttcgact gatcgacga 29